# EUROPEAN PATENT SPECIFICATION

(11) **EP 1 817 330 B1**
(45) Date of publication and mention of the grant of the patent: **10.12.2008**
(21) Application number: 05812763.0
(22) Date of filing: 02.12.2005
(51) Int. Cl.: C07K 14/415, A61K 38/16

(54) **PROTEIN ALLERGEN DERIVATIVES**
PROTEINALLERGENDERIVATE
DERIVES D'ALLERGENES PROTEINIQUES

(30) Priority: 02.12.2004 AT 20282004
(43) Date of publication of application: 15.08.2007
(73) Proprietor: BIOMAY AG, 1090 Wien (AT)
(72) Inventor: WESTRITSCHNIG, Kerstin, A-1180 Vienna (AT); FOCKE, Margarete, A-1140 Vienna (AT); VALENT, Peter, A-1180 Vienna (AT); KELLER, Walter, A-8111 Graz (AT); VALENTA, Rudolf, A-2604 Theresienfeld (AT)
(74) Representative: Sonn & Partner Patentanwälte
(86) International application number: PCT/AT2005/000486
(87) International publication number: WO 2006/058359

(56) References cited:
- EP-A- 1 403 280
- WESTRITSCHNIG KERSTIN ET AL: "Can we genetically engineer safer and more effective immunotherapy reagents?" CURRENT OPINION IN ALLERGY AND CLINICAL IMMUNOLOGY. DEC 2003, vol. 3, no. 6, December 2003 (2003-12), pages 495-500, XP009065969 ISSN: 1528-4050 cited in the application
- VRTALA S ET AL: "Strategies for converting allergens into hypoallergenic vaccine candidates" METHODS : A COMPANION TO METHODS IN ENZYMOLOGY, ACADEMIC PRESS INC., NEW YORK, NY, US, vol. 32, no. 3, March 2004 (2004-03), pages 313-320, XP004488982 ISSN: 1046-2023
- NIEDERBERGER V ET AL: "Vaccination with genetically engineered allergens prevents progression of allergic disease" PROCEEDINGS OF THE NATIONAL ACADEMY OF SCIENCES OF THE UNITED STATES OF AMERICA, vol. 101, no. Suppl. 2, 5 October 2004 (2004-10-05), pages 14677-14682, XP002379301 ISSN: 0027-8424
- VALENTA R ET AL: "IDENTIFICATION OF PROFILIN AS A NOVEL POLLEN ALLERGEN IGE AUTOREACTIVITY IN SENSITIZED INDIVIDUALS" SCIENCE (WASHINGTON D C), vol. 253, no. 5019, 1991, pages 557-560, XP001246905 ISSN: 0036-8075
- CARLSSON L ET AL: "ACTIN POLYMERIZABILITY IS INFLUENCED BY PROFILIN A LOW MOLECULAR WEIGHT PROTEIN IN NONMUSCLE CELLS" JOURNAL OF MOLECULAR BIOLOGY, vol. 115, no. 3, 1977, pages 465-484, XP009065988 ISSN: 0022-2836

## Description

The present invention relates to a method for reducing allergenic activity of wild-type protein allergens, novel allergen derivatives and allergy vaccination strategies.

Allergy is the inherited or acquired specific alternation of the reaction capability against foreign (i.e. non-self) substances which are normally harmless ("allergens"). Allergy is connected with inflammatory reactions in the affected organ systems (skin, conjunctiva, nose, pharynx, bronchial mucosa, gastrointestinal tract), immediate disease symptoms, such as allergic rhinitis, conjunctivitis, dermatitis, anaphylactic shock and asthma, and chronic disease manifestations, such as late stage reactions in asthma and atopic dermatitis.

Type I allergy represents a genetically determined hypersensitivity disease which affects about 20 % of the industrialised world population. The pathophysiological hallmark of Type I allergy is the production of immunoglobulin E (IgE) antibodies against otherwise harmless antigens (allergens).

Currently, the only causative form of allergy treatment is an allergen-specific immunotherapy wherein increasing allergen doses are administered to the patient in order to induce allergen-specific unresponsiveness. While several studies have shown clinical effectiveness of allergen-specific immunotherapy, the underlying mechanisms are not fully understood.

The major disadvantage of allergen-specific immunotherapy is the dependency on the use of natural allergen extracts which are difficult, if not impossible to standardise, at least to an industrial production level. Such natural allergen extracts consist of different allergenic and non allergenic compounds and due to this fact it is possible that certain allergens are not present in the administered extract or - even worse - that patients can develop new IgE-specificities to components in the course of the treatment. Another disadvantage of extract-based therapy results from the fact that the administration of biologically active allergen preparations can induce anaphylactic side effects.

The application of molecular biology techniques in the field of allergen characterisation has allowed to isolate the cDNAs coding for all relevant environmental allergens and allowed the production of recombinant allergens. Using such recombinant allergens has made it possible to determine the individual patient's reactivity profile either by in vitro diagnostic methods (i.e. detection of allergen-specific IgE antibodies in serum) or by in vivo testing. Based on this technology, the possibility to develop novel component-based vaccination strategies against allergy, especially against Type I allergy, which are tailored to the patient's sensitisation profile appeared to be possible. However, due to the similarity of the recombinant allergens to their natural counterparts, also recombinant allergens exhibit significant allergenic activity. Since the recombinant allergens closely mimick the allergenic activity of the wild-type allergens, all the drawbacks connected with this allergenic activity in immunotherapy applying natural allergens are also present for recombinant allergens. In order to improve immunotherapy the allergenic activity of the recombinant allergens has to be reduced so that the dose of the administered allergens can be increased with only a low risk of anaphylactic side effects.

It has been suggested to influence exclusively the activity of allergen-specific T cells by administration of peptides containing T cell epitopes only. T cell epitopes represent small peptides which result from the proteolytic digestion of intact allergens by antigen representing cells. Such T cell epitopes can be produced as synthetic peptides. Tests conducted so far with T cell epitopes, however, only showed poor results and low efficacy. Several explanations for the low efficacy of T cell peptide-based immunotherapy have been considered: first, it may be difficult to administer the optimal dose to achieve T cell tolerance instead of activation. Second, small T cell epitope peptides will have a short half-life in the body. Third, there is considerable evidence that IgE production in atopic individuals represents a memory immune response which does not require de novo class switching and thus cannot be controlled by T cell-derived cytokines. Therapy forms which are based exclusively on the administration of T cell epitopes may therefore modulate the activity of allergen-specific T cells but may have little influence on the production of allergen-specific IgE antibodies by already switched memory B cells.

It has further been suggested to produce hypoallergenic allergen derivates or fragments by recombinant DNA technology or peptide synthesis. Such derivatives or fragments bear T cell epitopes and can induce IgG antibodies that compete with IgE recognition of the native allergen. It was demonstrated more than 20 years ago that proteolytic digestion of allergens yielded small allergen fragments which in part retained their IgE binding capacity but failed to elicit immediate type reactions. While proteolysis of allergens is difficult to control and standardise, molecular biology has opened up new avenues for the production of IgE binding haptens. Such IgE binding haptens have been suggested to be useful for active immunisation with reduced risks of anaphylactic effects and for passive therapy to saturate effector cell-bound IgE prior to allergen contact and thus block allergen-induced mediator release.

Another suggestion was to produce hypoallergenic allergen versions by genetic engineering based on the observation that allergens can naturally occur as isoforms with differ in only a few amino acid residues and/or in conformations with low IgE binding capacity. For example, oligomerisation of the major birch pollen allergen, Bet v 1, by genetic engineering yielded a recombinant trimer with greatly reduced allergenic activity. Alternatively, introduction of point mutations has been suggested to either lead to conformational changes in the allergen structure and thus disrupt discontinuous IgE epitopes or directly affect the IgE binding capacity (Valenta et al., Biol.Chem.380 (1999), 815-824).

It has also been shown that fragmentation of the allergen into few parts (e.g. into two parts) leads to an almost complete loss of IgE binding capacity and allergenic activity of the allergen due to a loss of their native-like folds (Vrtala et al. (J.Clin.Invest.99 (1997), 1673-1681) for Bet v 1, Twardosz et al. (BBRC 239 (1997), 197-204) for Bet v 4, Hayek et al. (J. Immunol.161 (1998), 7031-7039) for Aln g 4, Zeiler et al. (J.Allergy Clin. Immunol.100 (1997), 721-727) for bovine dander allergen, Elfman (Int.Arch.Allergy Immunol.117 (1998), 167-173) for Lep d2), Westritschnig (J.Immunol.172 (2004), 5684-5692) for Phlp 7),...). Fragmentation of proteins containing primarily discontinuous/conformational IgE epitopes leads to a substantial reduction of the allergen's IgE binding capacity. Based on this knowledge, it was investigated in the prior art whether such hypoallergenic allergen fragments can induce protective immune responses in vivo (Westritschnig et al. (Curr. Opinion in Allergy and Clin. Immunol. 3 (2003), 495-500)).

In Vrtala S. et al. (Methods (2004) 32:313-320) strategies for converting allergens into hypoallergenic molecules are described. These strategies involve the production of allergen fragments, allergen mutants and chemically modified allergen derivatives.

EP 1 403 280 relates to vaccines comprising a hypoallergenic molecule derived from timothy grass pollen allergen Phl p 7.

In the article of Niederberger V. et al. (PNAS (2004) 101:14677-14682) the use of genetically engineered allergens, in particular of recombinant Bet v 1 fragments for vaccination, is described.

In Valenta R. et al. (Science (1991) 253:557-560) the identification of a birch pollen allergen having high sequence homology to profilins is disclosed. The authors of this article discovered that IgE antibodies from allergic individuals are able to bind the birch profilin as well as the human profilin.

It is an object of the present invention to provide means and methods for improved allergy immunotherapy based on the above mentioned knowledge. Such methods and means should be effective, connected with a low risk for anaphylactic shock, easily applicable and adapted to the needs of an individual patient and easily transformable into industrial scales.

Therefore the present invention provides a method for producing derivatives of wild-type protein allergens with reduced allergenic activity, which is characterized in by the following steps:
- providing a wild-type protein allergen with an allergenic activity,
- splicing said wild-type protein allergen into two parts said two parts having a reduced allergenic activity or lacking allergenic activity and
- rejoining said two fragments in inverse orientation.

The present method is based on the fact that fragmentation of proteins containing primarily discontinuous/conformational IgE epitopes leads to a substantial reduction of the allergen's IgE binding capacity. However, fragments of certain allergens were too less immunogenic to induce a protective antibody response (Westritschnig et al., (2004)).

With the present method, new and defined protein allergen derivatives are provided which combine the advantages of the T cell and B cell epitope-based approaches. At the same time, the disadvantages of vaccination with fragments only or sophisticated arrangements of fragments (such as IgE binding haptens and shuffling with three or more fragments) are not present for the allergen derivatives of the present invention.

In fact it could be shown with the present invention that the optimal results can be obtained with the structure which - with respect to completeness of structure elements - most closely resembles the wild-type allergen (i.e. with all amino acids of the wild-type allergen), however, without its allergenic activity (or with a sufficiently reduced allergenic activity). Of course, if only a few amino acid residues are lost (deleted) or added (inserted) in the course of generation of the allergen derivatives or if the parts are combined by a linker instead of a direct combination, the advantages according to the present invention are still present. This reduction or abolishment of allergenic activity is achieved by the known and general principle of dividing the allergen into defined fragments. In addition to this general principle, the present invention rejoins the two parts of the allergen obtained in inverse orientation which leads to allergen derivatives which contain essentially all relevant structural information of the allergen (because the amino acid sequence is contained in full or almost in full in the allergen derivates according to the present invention) but with only low (or no) remaining allergenic activity compared to the wild-type allergen.

These "head-to tail" derivatives according to the present invention enable a suitable, individual and efficient immunotherapy for allergy patients which is easily up-scaleable with routine steps. The derivates according to the present invention induce protective IgG antibodies which can block patient's IgE binding to wild-type allergens and inhibit allergen-induced basophil degranulation.

The present method is specifically suitable for recombinant DNA technology. Once the derivative is constructed by genetic engineering, it can easily be obtained in considerable amounts by transgene expression on an industrial scale in suitable hosts. The allergen derivatives according to the present invention can preferably be produced in a host with high expression capacity.

Preferred allergens to be modified by the present invention include all major protein allergens available e.g. under www.allergen.org/List.htm. Specifically preferred groups of allergens according to the present invention include profilins, especially Phl p 12, birch allergens, especially Bet v 4, dust mite allergens, especially Der p2, storage mite allergens, especially Lep d 2, timothy grass allergens, especially Phl p 7, and the allergens listed in table A.

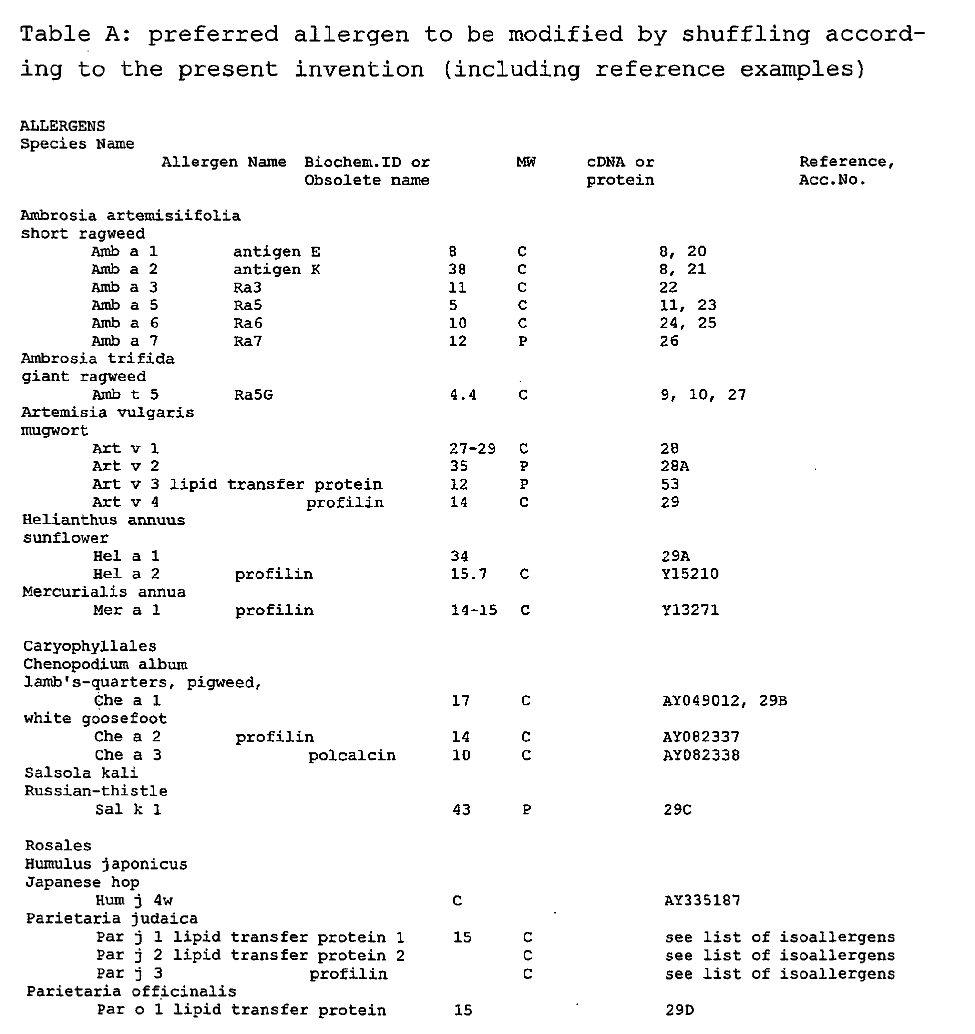

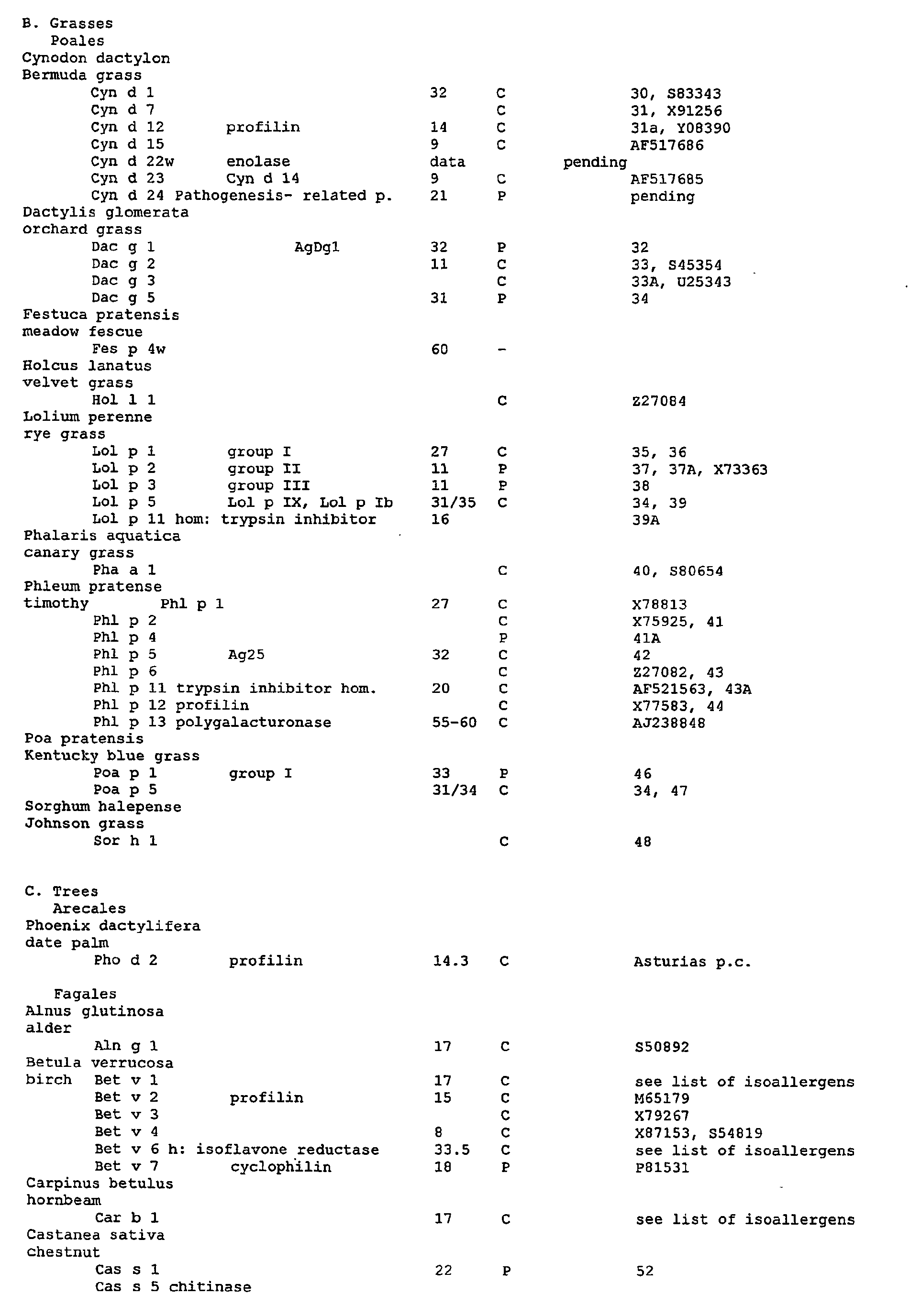

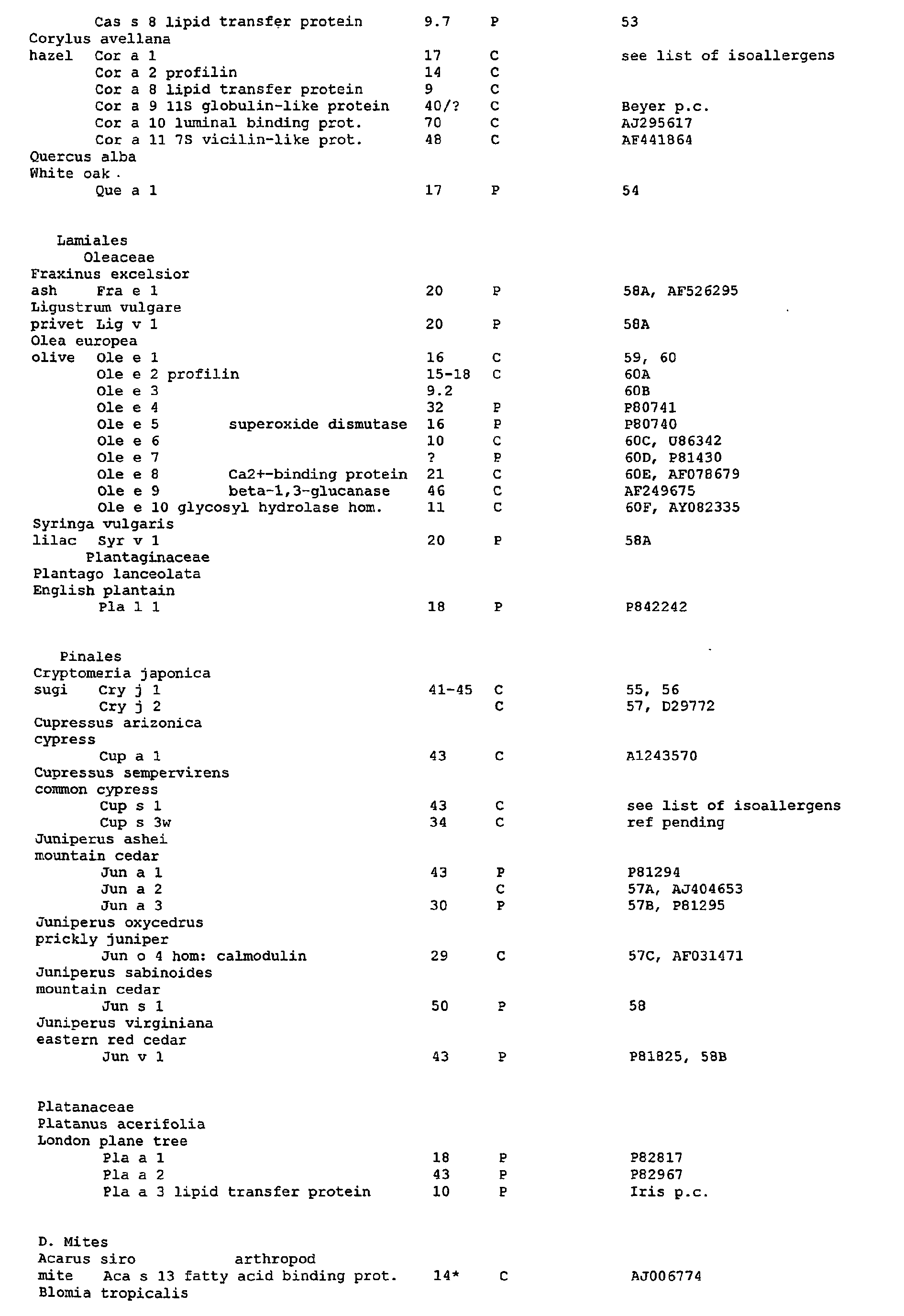

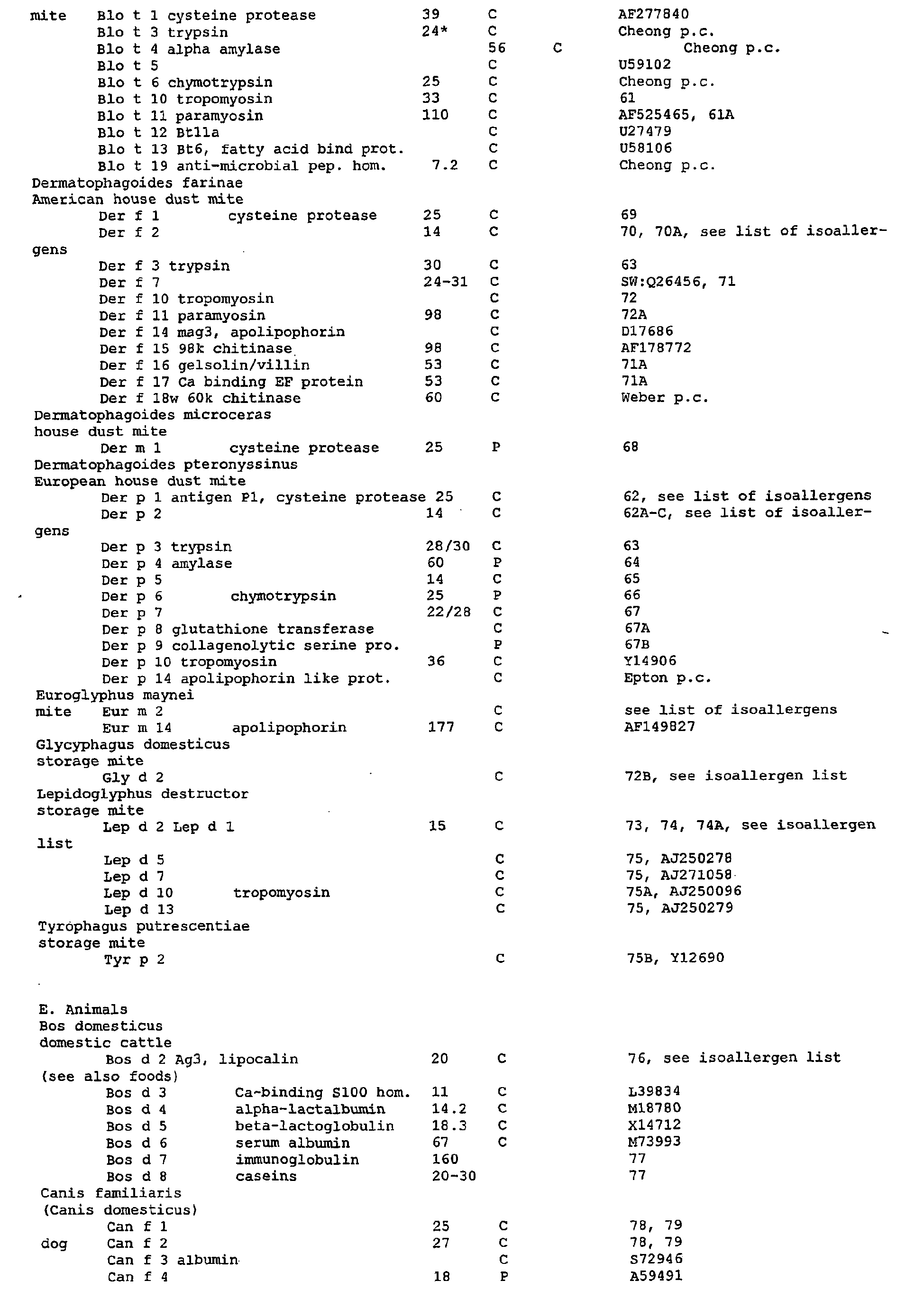

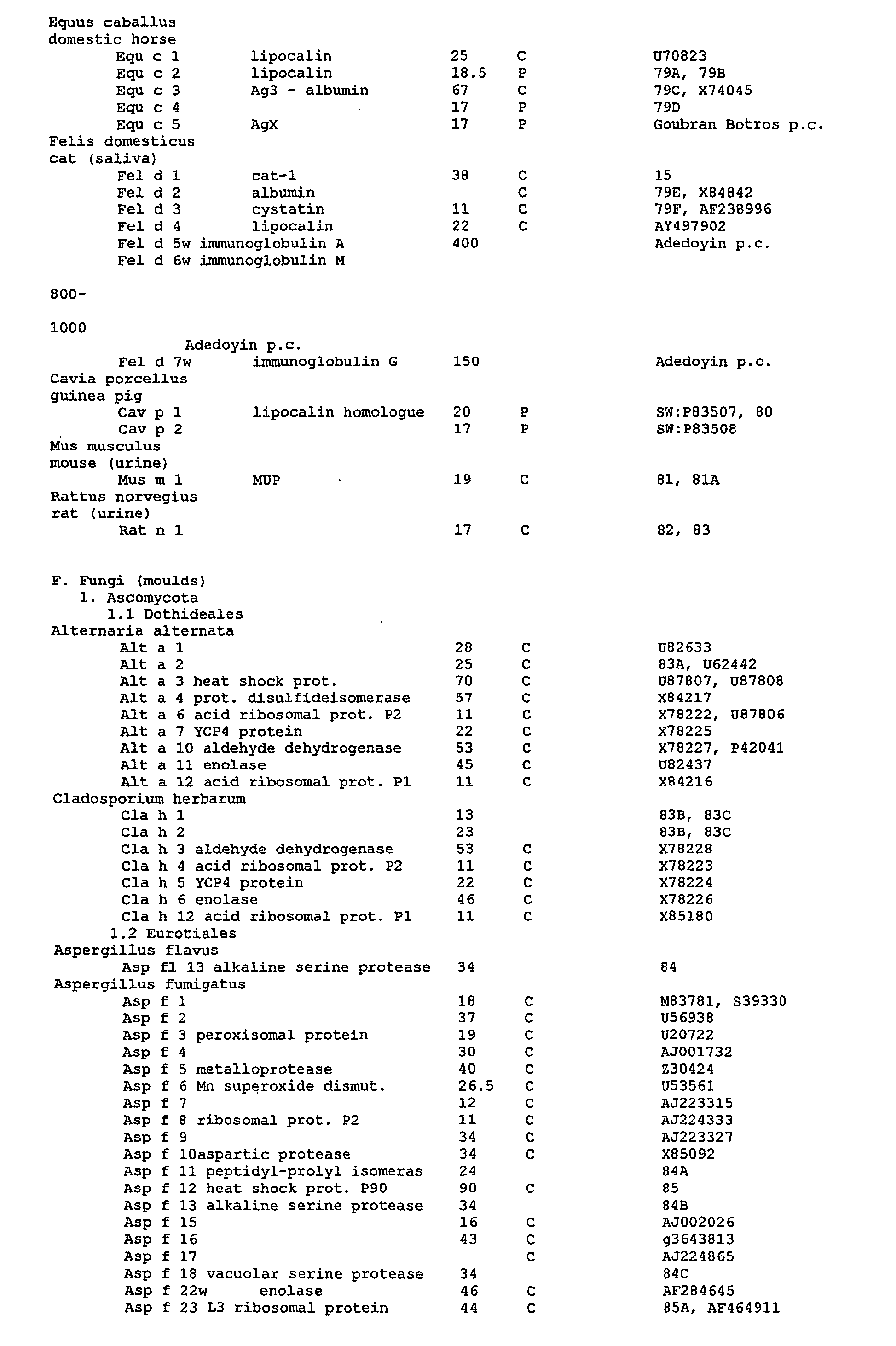

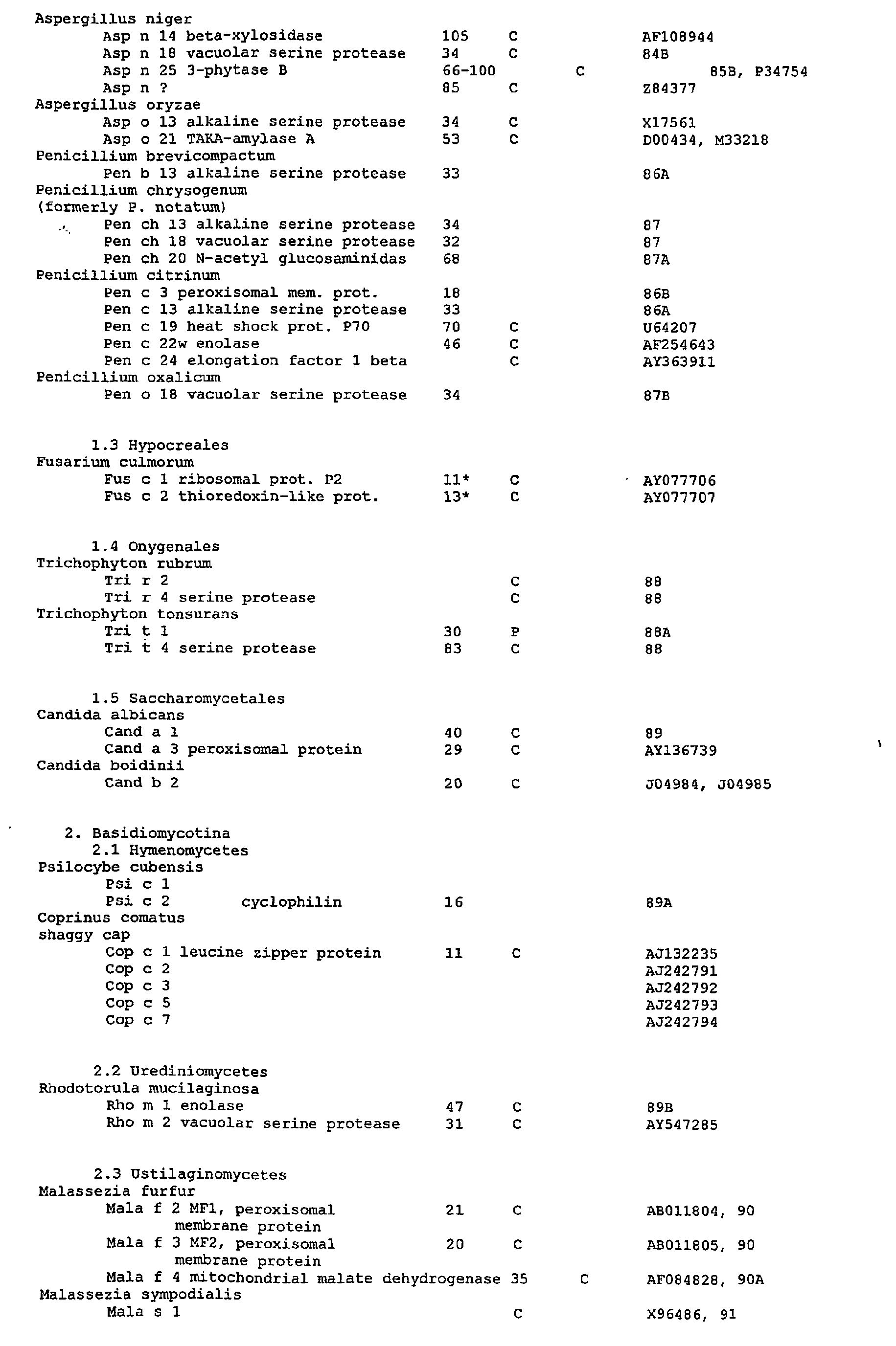

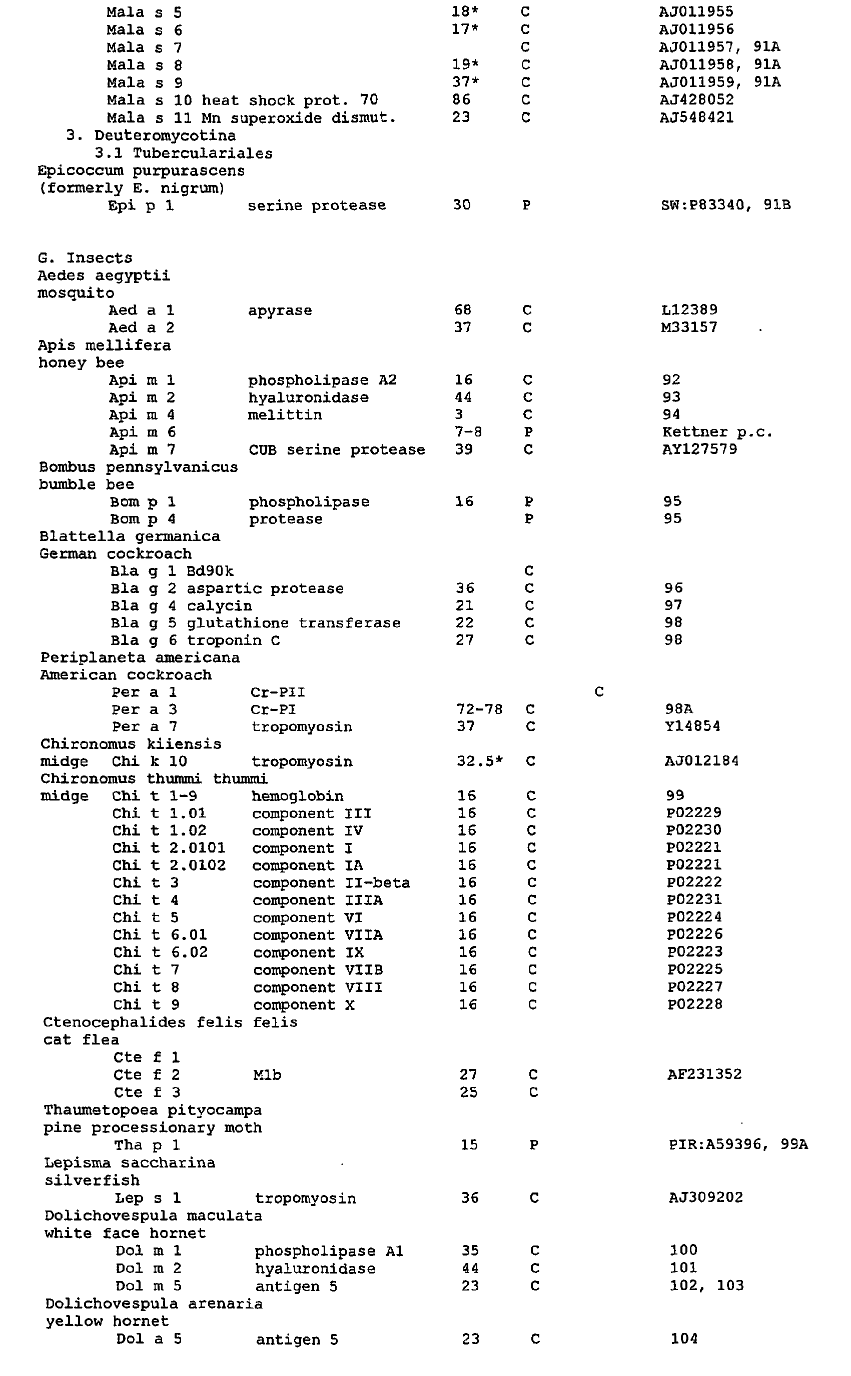

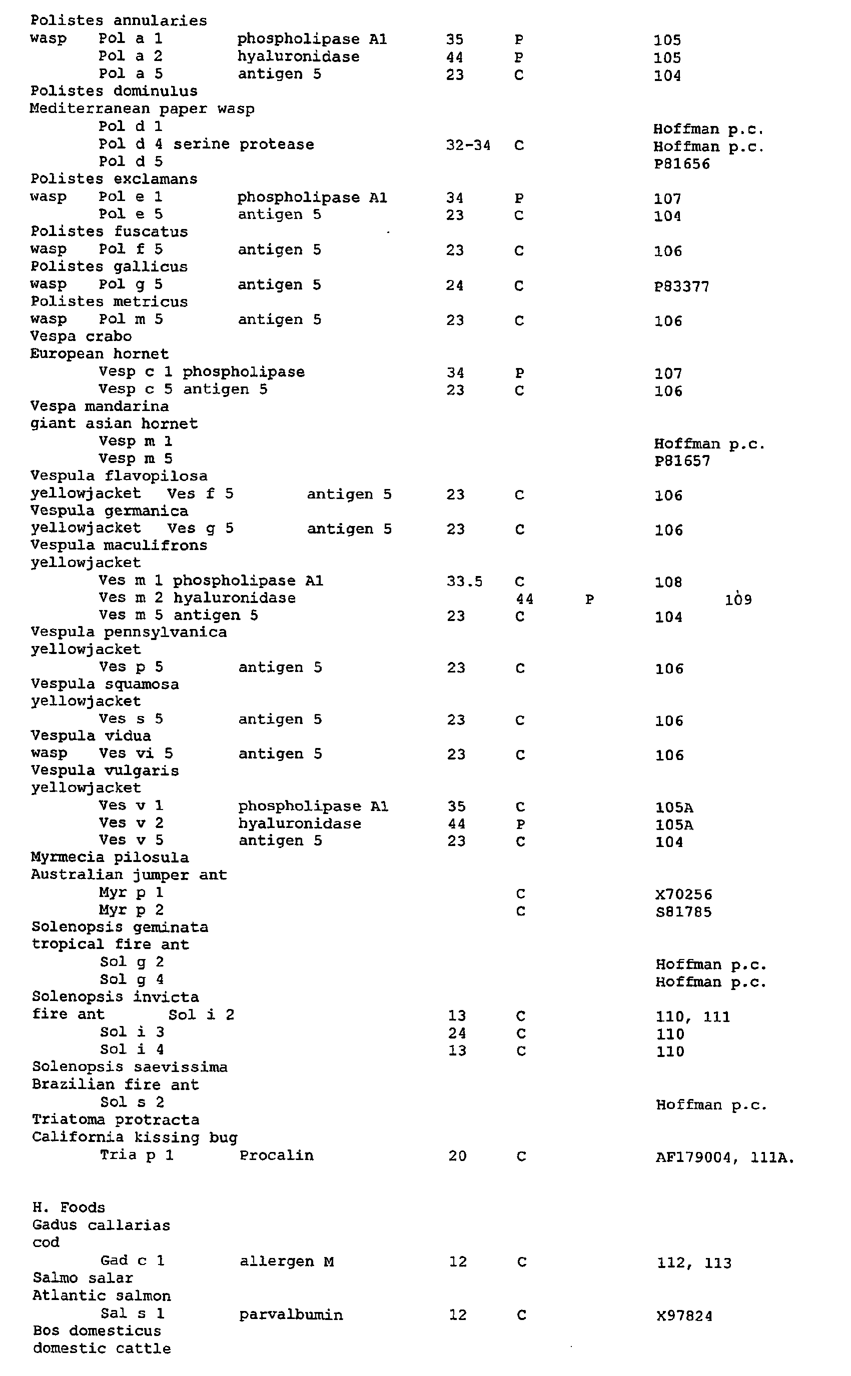

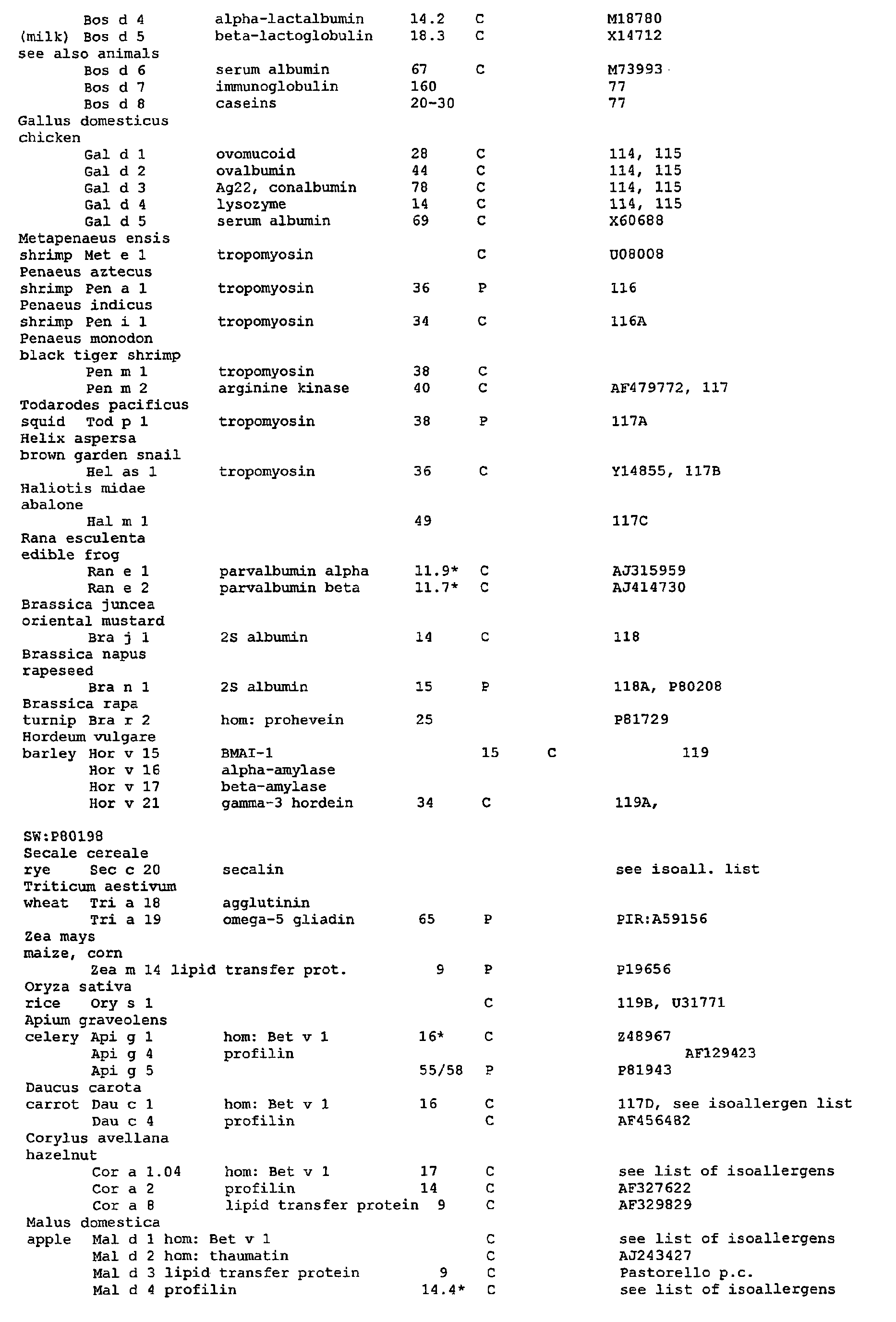

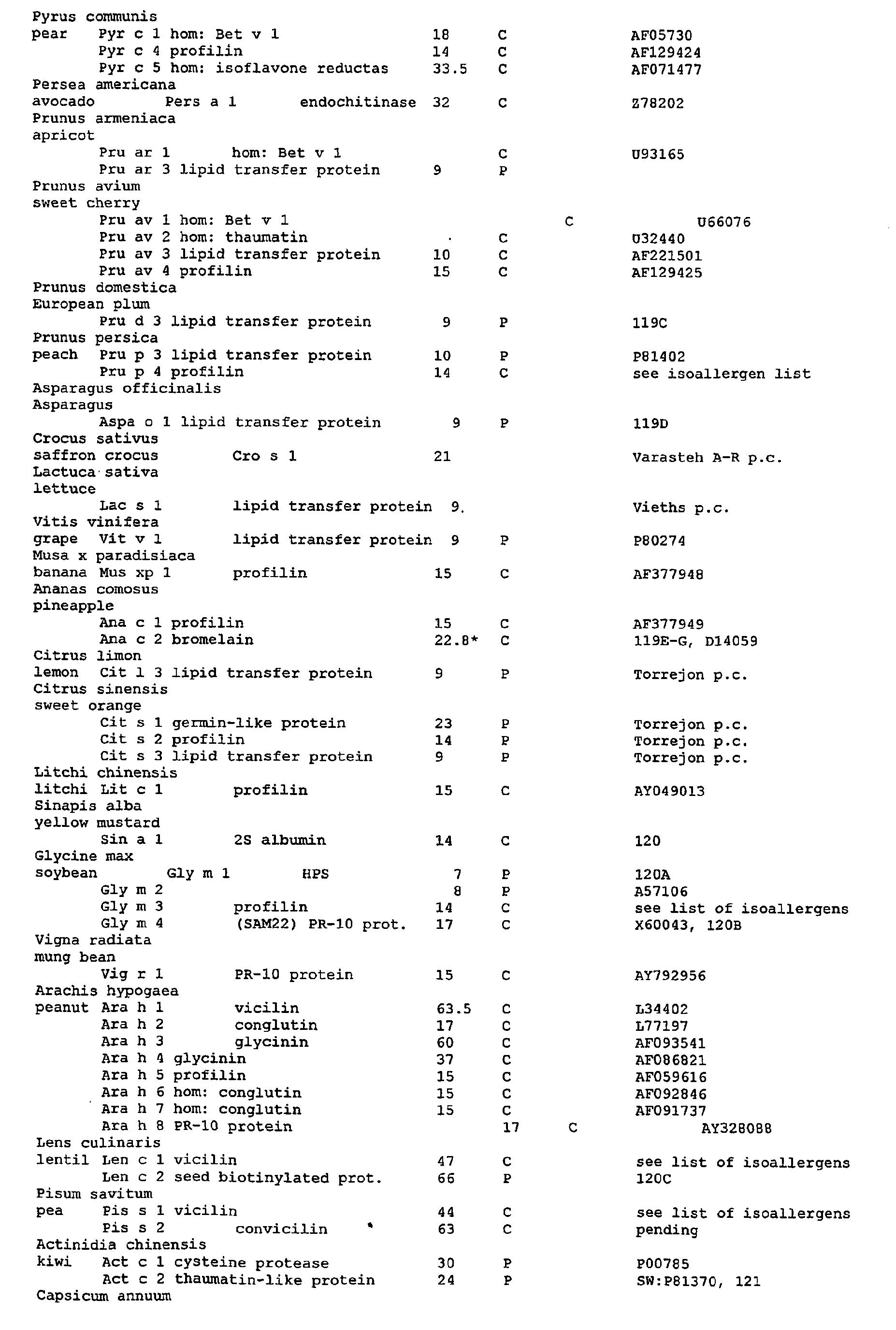

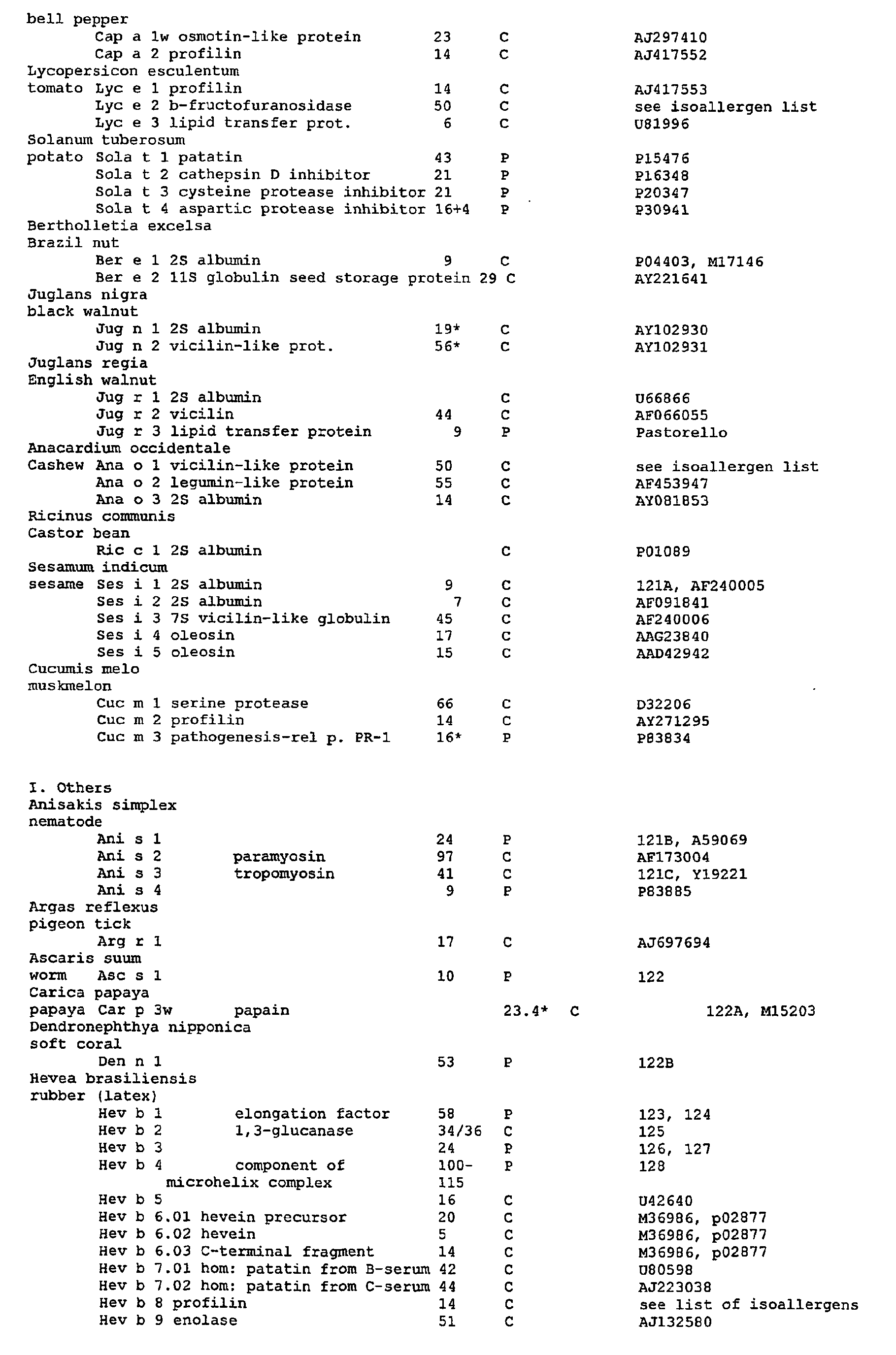

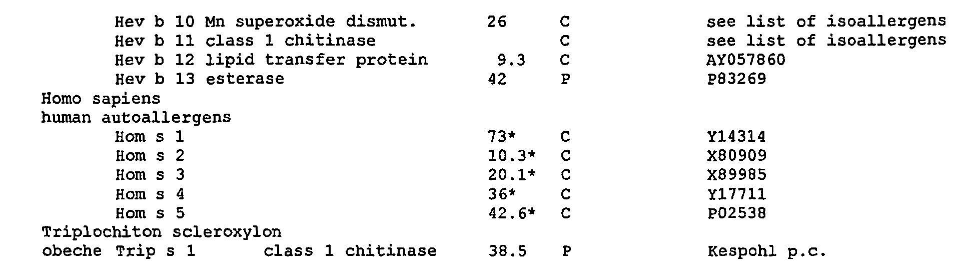

### References

1. Marsh, D.G., and L.R. Freidhoff. 1992. ALBE, an allergen database. IUIS, Baltimore, MD, Edition 1.0.
2. Marsh, D. G., L. Goodfriend, T. P. King, H. Lowenstein, and T. A. E. Platts-Mills. 1986. Allergen nomenclature. Bull WHO 64:767-770.
3. King, T.P., P.S. Norman, and J.T. Cornell. 1964. Isolation and characterization of allergen from ragweed pollen. II. Biochemistry 3:458-468.
4. Lowenstein, H. 1980. Timothy pollen allergens. Allergy 35:188-191.
5. Aukrust, L. 1980. Purification of allergens in Cladosporium herbarum. Allergy 35:206-207.
6. Demerec, M., E. A. Adelberg, A. J. Clark, and P. E. Hartman. 1966. A proposal for a uniform nomenclature in bacterial genetics. Genetics 54:61-75.
7. Bodmer, J. G., E. D. Albert, W. F. Bodmer, B. Dupont, H. A. Erlich, B. Mach, S. G. E. Marsh, W. R. Mayr, P. Parham, T. Sasuki, G. M. Th. Schreuder, J. L. Strominger, A. Svejgaard, and P. I. Terasaki. 1991. Nomenclature for factors of the HLA system, 1990. Immunogenetics 33:301-309.
8. Griffith, I.J., J. Pollock, D.G. Klapper, B.L. Rogers, and A.K. Nault. 1991. Sequence polymorphism of Amb a I and Amb a II, the major allergens in Ambrosia artemisiifolia (short ragweed). Int. Arch. Allergy Appl. Immunol. 96:296-304.
9. Roebber, M., D. G. Klapper, L. Goodfriend, W. B. Bias, S. H. Hsu, and D. G. Marsh. 1985. Immunochemical and genetic studies of Amb t V (Ra5G), an Ra5 homologue from giant ragweed pollen. J. Immunol. 134:3062-3069.
10. Metzler, W. J., K. Valentine, M. Roebber, M. Friedrichs, D. G. Marsh, and L. Mueller. 1992. Solution structures of ragweed allergen Amb t V. Biochemistry 31:5117-5127.
11. Metzler, W. J., K. Valentine, M. Roebber, D. G. Marsh, and L. Mueller. 1992. Proton resonance assignments and three-dimensional solution structure of the ragweed allergen Amb a V by nuclear magnetic resonance spectroscopy. Biochemistry 31:8697-8705.
12. Goodfriend, L., A.M. Choudhury, J. Del Carpio, and T.P. King. 1979. Cytochromes C: New ragweed pollen allergens. Fed. Proc. 38:1415.
13. Ekramoddoullah, A. K. M., F. T. Kisil, and A. H. Sehon. 1982. Allergenic cross reactivity of cytochrome c from Kentucky bluegrass and perennial ryegrass pollens. Mol. Immunol. 19:1527-1534.
14. Ansari, A. A., E. A. Killoran, and D. G. Marsh. 1987. An investigation of human response to perennial ryegrass (Lolium perenne) pollen cytochrome c (Lol p X). J. Allergy Clin. Immunol. 80:229-235.
15. Morgenstern, J.P., I.J. Griffith, A.W. Brauer, B.L. Rogers, J.F. Bond, M.D. Chapman, and M. Kuo. 1991. Amino acid sequence of Fel d I, the major allergen of the domestic cat: protein sequence analysis and cDNA cloning. Proc. Natl. Acad. Sci. USA 88:9690-9694.
16. Griffith, I.J., S. Craig, J. Pollock, X. Yu, J.P. Morgenstern, and B.L.Rogers. 1992. Expression and genomic structure of the genes encoding FdI, the major allergen from the domestic cat. Gene 113:263-268.
17. Weber, A., L. Marz, and F. Altmann. 1986. Characteristics of the asparagine-linked oligosaccharide from honey-bee venom phospholipase A2. Comp. Biochem. Physiol. 83B:321-324.
18. Weber, A., H. Schroder, K. Thalberg, and L. Marz. 1987. Specific interaction of IgE antibodies with a carbohydrate epitope of honey bee venom phospholipase A2. Allergy 42:464-470.
19. Stanworth, D. R., K. J. Dorrington, T. E. Hugli, K. Reid, and M. W. Turner. 1990. Nomenclature for synthetic peptides representative of immunoglobulin chain sequences. Bulletin WHO 68:109-111.
20. Rafnar, T., I. J. Griffith, M. C. Kuo, J. F. Bond, B. L. Rogers, and D.G. Klapper. 1991. Cloning of Amb a I (Antigen E), the major allergen family of short ragweed pollen. J. Biol. Chem. 266: 1229-1236.
21. Rogers, B.L., J.P. Morgenstern, I.J. Griffith, X.B. Yu, C.M. Counsell, A.W. Brauer, T.P. King, R.D. Garman, and M.C. Kuo. 1991. Complete sequence of the allergen Amb a II: recombinant expression and reactivity with T cells from ragweed allergic patients. J. Immunol. 147:2547-2552.
22. Klapper, D.G., L. Goodfriend, and J.D. Capra. 1980. Amino acid sequence of ragweed allergen Ra3. Biochemistry 19:5729-5734.
23. Ghosh, B., M.P. Perry, T. Rafnar, and D.G. Marsh. 1993. Cloning and expression of immunologically active recombinant Amb a V allergen of short ragweed (Ambrosia artemisiifolia) pollen. J. Immunol. 150:5391-5399.
24. Roebber, M., R. Hussain, D. G. Klapper, and D. G. Marsh. 1983. Isolation and properties of a new short ragweed pollen allergen, Ra6. J. Immunol. 131:706-711.
25. Lubahn, B., and D.G. Klapper. 1993. Cloning and characterization of ragweed allergen Amb a VI (abst). J. Allergy Clin. Immunol. 91:338.
26. Roebber, M., and D.G. Marsh. 1991. Isolation and characterization of allergen Amb a VII from short ragweed pollen. J. Allergy Clin. Immunol. 87:324.
27 Goodfriend L, Choudhury AM, Klapper DG, Coulter KM, Dorval G, DelCarpio J, Osterland CK. Ra5G, a homologue of Ra5 in giant ragweed pollen: isolation, HLA-DR-associated activity and amino acid sequence. Mol Immunol 22: 899-906, 1985.
28 Himly M, Jahn-Schmid B, Dedic A, Kelemen P, Wopfner N, Altmann F, van Ree R, Briza P, Richter K, Ebner C, Ferreira F. Art v 1, the major allergen of mugwort pollen, is a modular glycoprotein with a defensin-like and a hydroxyproline-rich domain. FASEB J 17: 106-108, 2003.
28A Nilsen, B. M., K. Sletten, M. O'Neill, B. Smestead Paulsen, and H. van Halbeek. 1991. Structural analysis of the glycoprotein allergen Art v II from pollen of mugwort (Artemesia vulgaris). J. Biol. Chem. 266:2660-2668.
29 Wopfner N, Willeroidee M, Hebenstreit D, van Ree R, Aalbers M, Briza P, Thalhamer J, Ebner C, Richter K, Ferreira F. Molecular and immunological characterization of profilin from mugwort pollen. Biol Chem 383: 1779-1789, 2002.
29A Jimenez A, Moreno C, Martinez J, Martinez A, Bartolome B, Guerra F, Palacios R 1994. Sensitization to sunflower pollen: only an occupational allergy? Int Arch Allergy Immunol 105:297-307. 29B Barderas R, Villalba M, Lombardero M, Rodriguez R. Identification and characterization of Che a 1 allergen from Chenopodium album pollen. Int Arch Allergy Immunol 127: 47-54, 2002.
29C Carnés J, Fernández-Caldas E, Casanovas M, Lahoz C, Colás C. Immunochemical characterization of Salsola kali pollen extracts. Allergy 56, Supplement 68: 274, 2001.
29D Giuliani A, Pini C, Bonini S, Mucci N, Ferroni L, Vicari G: Isolation and purification of a major allergen from Parietaria officinalis pollen. Allergy 42: 434-440, 1987.
30 Smith,P.M., Suphioglu,C., Griffith,I.J., Theriault,K., Knox,R.B. and Singh,M.B. 1996. Cloning and expression in yeast Pichia pastoris of a biologically active form of Cyn d 1, the major allergen of Bermuda grass pollen. J. Allergy Clin. Immunol. 98:331-343.
31 Suphioglu,C., Ferreira,F. and Knox,R.B. 1997. Molecular cloning and immunological characterisation of Cyn d 7, a novel calcium-binding allergen from Bermuda grass pollen. FEBS Lett. 402:167-172.
31a. Asturias JA, Arilla MC, Gomez-Bayon N, Martinez J, Martinez A, and Palacios R. 1997. Cloning and high level expression of Cynodon dactylon (Bermuda grass) pollen profilin (Cyn d 12) in Escherichia coli: purification and characterization of the allergen. Clin Exp Allergy 27:1307-1313.
32. Mecheri, S., G. Peltre, and B. David. 1985. Purification and characterization of a major allergen from Dactylis glomerata pollen: The Ag Dg 1. Int. Arch. Allergy Appl. Immunol. 78:283-289.
33. Roberts, A.M., L.J. Bevan, P.S. Flora, I. Jepson, and M.R. Walker. 1993. Nucleotide sequence of cDNA encoding the Group II allergen of Cocksfoot/Orchard grass (Dactylis glomerata), Dac g II. Allergy 48:615-623.
33a. Guerin-Marchand,C., Senechal,H., Bouin,A.P., Leduc-Brodard,V., Taudou,G., Weyer,A., Peltre,G. and David,B. 1996. Cloning, sequencing and immunological characterization of Dac g 3, a major allergen from Dactylis glomerata pollen. Mol. Immunol. 33:797-806.
34. Klysner, S., K. Welinder, H. Lowenstein, and F. Matthiesen. 1992. Group V allergens in grass pollen IV. Similarities in amino acid compositions and amino terminal sequences of the group V allergens from Lolium perenne, Poa pratensis and Dactylis glomerata. Clin. Exp. Allergy 22: 491-497.
35. Perez, M., G. Y. Ishioka, L. E. Walker, and R. W. Chesnut. 1990. cDNA cloning and immunological characterization of the rye grass allergen Lol p I. J. Biol. Chem. 265:16210-16215.
36. Griffith, I. J., P. M. Smith, J. Pollock, P. Theerakulpisut, A. Avjioglu, S. Davies, T. Hough, M. B. Singh, R. J. Simpson, L. D. Ward, and R. B. Knox. 1991. Cloning and sequencing of Lol p I, the major allergenic protein of rye-grass pollen. FEBS Letters 279:210-215.
37. Ansari, A. A., P. Shenbagamurthi, and D.G. Marsh. 1989. Complete amino acid sequence of a Lolium perenne (perennial rye grass) pollen allergen, Lol p II. J. Biol. Chem. 264:11181-11185.
37a. Sidoli,A., Tamborini,E., Giuntini,I., Levi,S., Volonte,G., Paini,C., De Lalla,C., Siccardi,A.G., Baralle,F.E., Galliani,S. and Arosio,P. 1993. Cloning, expression, and immunological characterization of recombinant Lolium perenne allergen Lol p II. J. Biol. Chem. 268:21819-21825.
38. Ansari, A. A., P. Shenbagamurthi, and D. G. Marsh. 1989. Complete primary structure of a Lolium perenne (perennial rye grass) pollen allergen, Lol p III: Comparison with known Lol p I and II sequences. Biochemistry 28:8665-8670.
39. Singh, M. B., T. Hough, P. Theerakulpisut, A. Avjioglu, S. Davies, P. M. Smith, P. Taylor, R. J. Simpson, L. D. Ward, J. McCluskey, R. Puy, and R.B. Knox. 1991. Isolation of cDNA encoding a newly identified major allergenic protein of rye-grass pollen: Intracellular targeting to the amyloplost. Proc. Natl. Acad. Sci. 88:1384-1388.
39a. van Ree R, Hoffman DR, van Dijk W, Brodard V, Mahieu K, Koeleman CA, Grande M, van Leeuwen WA, Aalberse RC. 1995. Lol p XI, a new major grass pollen allergen, is a member of a family of soybean trypsin inhibitor-related proteins. J Allergy Clin Immunol 95:970-978.
40. Suphioglu,C. and Singh,M.B. 1995. Cloning, sequencing and expression in Escherichia coli of Pha a 1 and four isoforms of Pha a 5, the major allergens of canary grass pollen. Clin. Exp. Allergy 25:853-865.
41 Dolecek,C., Vrtala,S., Laffer,S., Steinberger,P., Kraft,D., Scheiner,o. and Valenta,R. 1993. Molecular characterization of Phl p II, a major timothy grass (Phleum pratense) pollen allergen. FEBS Lett. 335:299-304.
41A Fischer S, Grote M, Fahlbusch B, Muller WD, Kraft D, Valenta R. 1996. Characterization of Phl p 4, a major timothy grass (Phleum pratense) pollen allergen. J Allergy Clin Immunol 98:189-198.
42 Matthiesen, F., and H. Lowenstein. 1991. Group V allergens in grass pollens. I. Purification and characterization of the group V allergen from Phleum pratense pollen, Phl p V. Clin. Exp. Allergy 21:297-307.
43 Petersen,A., Bufe,A., Schramm,G., Schlaak,M. and Becker,W.M. 1995. Characterization of the allergen group VI in timothy grass pollen (Phl p 6). II. cDNA cloning of Phl p 6 and structural comparison to grass group V. Int. Arch. Allergy Immunol. 108:55-59.
43A Marknell DeWitt A, Niederberger V, Lehtonen P, Spitzauer S, Sperr WR, Valent P, Valenta R, Lidholm J. Molecular and immunological characterization of a novel timothy grass (Phleum pratense) pollen allergen, Phl p 11. Clin Exp Allergy 32: 1329-1340, 2002.
44 Valenta,R., Ball,T., Vrtala,S., Duchene,M., Kraft,D. and Scheiner,O. 1994. cDNA cloning and expression of timothy grass (Phleum pratense) pollen profilin in Escherichia coli: comparison with birch pollen profilin. Biochem. Biophys. Res. Commun. 199:106-118.
46 Esch, R. E., and D. G. Klapper. 1989. Isolation and characterization of a major cross-reactive grass group I allergenic determinant. Mol. Immunol. 26:557-561.
47. Olsen, E., L. Zhang, R. D. Hill, F. T. Kisil, A. H. Sehon, and S. Mohapatra. 1991. Identification and characterization of the Poa p IX group of basic allergens of Kentucky bluegrass pollen. J. Immunol. 147:205-211.
48. Avjioglu, A., M. Singh, and R.B. Knox. 1993. Sequence analysis of Sor h I, the group I allergen of Johnson grass pollen and it comparison to rye-grass Lol p I (abst). J. Allergy Clin. Immunol. 91:340.
52. Kos T, Hoffmann-Sommergruber K, Ferreira F, Hirschwehr R, Ahorn H, Horak F, Jager S, Sperr W, Kraft D, Scheiner O. 1993. Purification, characterization and N-terminal amino acid sequence of a new major allergen from European chestnut pollen--Cas s 1. Biochem Biophys Res Commun 196:1086-92.
53. Diaz-Perales A, Lombardero M, Sánchez-Monge R, Garcia-Sellés FJ, Pernas M, Fernández-Rivas M, Barber D, Salcedo G. 2000. Lipid-transfer proteins as potential plant panallergens: cross-reactivity among proteins of Artemisia pollen, Castaneae nut and Rosaceae fruits, with different IgE-binding capacities. Clin Exp Allergy 30:1403-1410.
54. Ipsen, H., and O.C. Hansen. 1991. The NH2-terminal amino acid sequence of the immunochemically partial identical major allergens of alder (Alnus glutinosa) Aln g I, birch (Betula verrucosa) Bet v I, hornbeam (Carpinus betulus) Car b I and oak (Quercus alba) Que a I pollens. Mol. Immunol. 28: 1279-1288.
55. Taniai, M., S. Ando, M. Usui, M. Kurimoto, M. Sakaguchi, S. Inouye, and T. Matuhasi. 1988. N-terminal amino acid sequence of a major allergen of Japanese cedar pollen (Cry j I). FEBS Lett. 239:329-332.
56. Griffith, I.J., A. Lussier, R. Garman, R. Koury, H. Yeung, and J. Pollock. 1993. The cDNA cloning of Cry j I, the major allergen of Cryptomeria japonica (Japanese cedar) (abst). J. Allergy Clin. Immunol. 91:339.
57. Sakaguchi, M., S. Inouye, M. Taniai, S. Ando, M. Usui, and T. Matuhasi. Identification of the second major allergen of Japanese cedar pollen. Allergy 45: 309-312, 1990.
57A Yokoyama M, Miyahara M, Shimizu K, Kino K, Tsunoo H. Purification, identification, and cDNA cloning of Jun a 2, the second major allergen of mountain cedar pollen. Biochem Biophys Res Commun 275: 195-202, 2000.
57B Midoro-Horiuti T, Goldblum RM, Kurosky A, Wood TG, Brooks EG. Variable Expression of Pathogenesis-Related Protein Allergen in Mountain Cedar (Juniperus ashei) Pollen. J Immunol 164: 2188-2192, 2000.
57C Tinghino R., Barletta B., Palumbo S., Afferni C., Iacovacci P., Mari A., Di Felice G., Pini,C. Molecular characterization of a cross-reactive Juniperus oxycedrus pollen allergen, Jun o 2: a novel calcium-binding allergen J. Allergy Clin. Immunol. 101: 772-777, 1998.
58 Gross GN, Zimburean JM, Capra JD. Isolation and partial characterization of the allergen in mountain cedar pollen. Scand J Immunol 8: 437-441, 1978.
58A Obispo TM, Melero JA, Carpizo JA, Carreira J, Lombardero M. The main allergen of Olea europaea (Ole e I) is also present in other species of the oleaceae family. Clin Exp Allergy 23: 311-316, 1993.
58B Midoro-Horiuti T, Goldblum RM, Brooks EG. Identification of mutations in the genes for the pollen allergens of eastern red cedar (Juniperus virginiana). Clin Exp Allergy 31: 771-778, 2001. 59 Lombardero M., Barbas J.A., Moscoso del Prado J., Carreira J. cDNA sequence analysis of the main olive allergen, Ole e I. Clin. Exp. Allergy 24: 765-770, 1994.
60 Villalba, M., E. Batanero, C. Lopez-Otin, L.M. Sanchez, R.I. Monsalve, M.A. Gonzalez de la Pena, C. Lahoz, and R. Rodriguez. Amino acid sequence of Ole e I, the major allergen from olive tree pollen (Olea europaea). Eur. J. Biochem. 216: 863-869, 1993.
60A Asturias JA, Arilla MC, Gomez-Bayon N, Martinez J, Martinez A, Palacios R. Cloning and expression of the panallergen profilin and the major allergen (Ole e 1) from olive tree pollen. J Allergy Clin Immunol 100: 365-372, 1997.
60B Batanero E, Villalba M, Ledesma A Puente XS, Rodriguez R. Ole e 3, an olive-tree allergen, belongs to a widespread family of pollen proteins. Eur J Biochem 241: 772-778, 1996.
60C Batanero E, Ledesma A, Villalba M, Rodriguez R. Purification, amino acid sequence and immunological characterization of Ole e 6, a cysteine-enriched allergen from olive tree pollen. FEBS Lett. 410: 293-296, 1997.
60D Tejera ML, Villalba M, Batanero E, Rodriguez R. Identification, isolation, and characterization of Ole e 7, a new allergen of olive tree pollen. J Allergy Clin Immunol 104: 797-802, 1999. 60E Ledesma A, Villalba M, Rodriguez R. Cloning, expression and characterization of a novel four EF-hand Ca(2+)-binding protein from olive pollen with allergenic activity. FEBS Lett 466: 192-196, 2000.
60F Barral P, Batanero E, Palomares O, Quiralte J, Villalba M, Rodriguez R. A major allergen from pollen defines a novel family of plant proteins and shows intra- and interspecies [correction of interspecie] cross-reactivity. J Immunol 172: 3644-3651, 2004.
61 Yi FC, Cheong N, Shek PC, Wang DY, Chua KY, Lee BW. Identification of shared and unique immunoglobulin E epitopes of the highly conserved tropomyosins in Blomia tropicalis and Dermatophagoides pteronyssinus. Clin Exp Allergy 32: 1203-1210, 2002.
61A Ramos JD, Cheong N, Lee BW, Chua KY. cDNA cloning and expression of Blo t 11, the Blomia tropicalis allergen homologous to paramyosin. Int Arch Allergy Immunol 126: 286-293, 2001.
62 Chua, K. Y., G. A. Stewart, and W. R. Thomas. Sequence analysisof cDNA encoding for a major house dust mite allergen, Der p I. J. Exp. Med. 167: 175-182, 1988.
62A Chua, K. Y., C. R. Doyle, R. J. Simpson, K. J. Turner, G. A. Stewart, and W. R. Thomas. Isolation of cDNA coding for the major mite allergen Der p II by IgE plaque immunoassay. Int. Arch. Allergy Appl. Immunol. 91: 118-123, 1990.
62B Smith AM, Benjamin DC, Derewenda U, Smith WA , Thomas WR, Chapman MD. Sequence polymorphisms and antibody binding to the group 2 dust mite allergens. Int Arch Allergy Immunol 124: 61-63, 2001.
62C Smith AM, Benjamin DC, Hozic N, Derewenda U, Smith WA, Thomas WR, Gafvelin G, van Hage-Hamsten M, Chapman MD. The molecular basis of antigenic cross-reactivity between the group 2 mite allergens. J Allergy Clin Immunol 107: 977-984, 2001.
63 Smith WA, Thomas WR. Comparative analysis of the genes encoding group 3 allergens from Dermatophagoides pteronyssinus and Dermatophagoides farinae. Int Arch Allergy Immunol 109: 133-140, 1996.
64 Lake, F.R., L.D. Ward, R.J. Simpson, P.J. Thompson, and G.A. Stewart. House dust mite-derived amylase: Allergenicity and physicochemical characterisation. J. Allergy Clin. Immunol. 87: 1035-1042, 1991.
65 Tovey, E. R., M. C. Johnson, A. L. Roche, G. S. Cobon, and B. A. Baldo. Cloning and sequencing of a cDNA expressing a recombinant house dust mite protein that binds human IgE and corresponds to an important low molecular weight allergen. J. Exp. Med. 170: 1457-1462, 1989.
66 Yasueda, H., T. Shida, T. Ando, S. Sugiyama, and H. Yamakawa. 1991. Allergenic and proteolytic properties of fourth allergens from Dermatophagoides mites. In: "Dust Mite Allergens and Asthma. Report of the 2nd international workshop" A. Todt, Ed., UCB Institute of Allergy, Brussels, Belgium, pp. 63-64.
67 Shen, H.-D., K.-Y. Chua, K.-.L. Lin, K.-H. Hsieh, and W.R. Thomas. Molecular cloning of a house dust mite allergen with common antibody binding specificities with multiple components in mite extracts. Clin. Exp. Allergy 23: 934-940, 1993.
67A O'Neil GM, Donovan GR, Baldo BA. Cloning and charaterisation of a major allergen of the house dust mite Dermatophagoides pteronyssinus, homologous with glutathione S-transferase. Biochim Biophys Acta,1219: 521-528, 1994.
67B King C, Simpson RJ, Moritz RL, Reed GE, Thompson PJ, Stewart GA. The isolation and characterization of a novel collagenolytic serine protease allergen (Der p 9) from the dust mite Dermatophagoides pteronyssinus. J Allergy Clin Immunol 98: 739-747, 1996.
68 Lind P, Hansen OC, Horn N. The binding of mouse hybridoma and human IgE antibodies to the major fecal allergen, Der p I of D. pteronyssinus. J. Immunol. 140: 4256-4262, 1988.
69 Dilworth, R. J., K. Y. Chua, and W. R. Thomas. Sequence analysis of cDNA coding for a mojor house dust allergn Der f I. Clin. Exp. Allergy 21: 25-32, 1991.
70 Nishiyama, C., T. Yunki, T. Takai, Y. Okumura, and H. Okudaira. Determination of three disulfide bonds in a major house dust mite allergen, Der f II. Int. Arch. Allergy Immunol. 101: 159-166, 1993.
70A Trudinger, M., K. Y. Chua, and W. R. Thomas. cDNA encoding the major dust mite allergen Der f II. Clin. Exp. Allergy 21: 33-38, 1991.
71 Shen HD, Chua KY, Lin WL, Hsieh KH, Thomas WR. Molecular cloning and immunological characterization of the house dust mite allergen Der f 7. Clin Exp Allergy 25: 1000-1006, 1995.
71A Tategaki A, Kawamoto S, Aki T, Jyo T, Suzuki O, Shigeta S, Ono K. Newly described house dust mite allergens. ACI International suppl. 1: 74-76, 2000.
72 Aki T, Kodama T, Fujikawa A, Miura K, Shigeta S, Wada T, Jyo T, Murooka Y, Oka S, Ono K. Immunochemical characteristion of recombinant and native tropomyosins as a new allergen from the house dust mite Dermatophagoides farinae. J Allergy Clin Immunol 96: 74-83, 1995.
72A Tsai L, Sun Y, Chao P, Ng H, Hung M, Hsieh K, Liaw S, Chua K. Sequence analysis and expression of a cDNA clone encoding a 98-kDa allergen in Dermatophagoides farinae. Clin Exp Allergy 29: 1606-1613, 1999.
72B Gafvelin G, Johansson E, Lundin A, Smith AM, Chapman MD, Benjamin DC, Derewenda U, Van Hage-Hamsten M. Cross-reactivity studies of a new group 2 allergen from the dust mite Glycyphagus domesticus, Gly d 2, and group 2 allergens from Dermatophagoides pteronyssinus, Lepidoglyphus destructor, and Tyrophagus putrescentiae with recombinant allergens. J Allergy Clin Immunol 107: 511-518, 2001.
73 van Hage-Hamsten, M., T. Bergman, E. Johansson, B. Persson, H. Jornvall, B. Harfast, and S.G.O. Johansson. N-terminal amino acid sequence of major allergen of the mite lepidoglyphus destructor (abst). J. Allergy Clin. Immunol. 91:353, 1993.
74 Varela J, Ventas P, Carreira J, Barbas JA, Gimenez-Gallego G, Polo F. Primary structure of Lep d I, the main Lepidoglyphus destructor allergen. Eur J Biochem 225: 93-98, 1994.
74A Schmidt M, van der Ploeg I, Olsson S, van Hage Hamsten M. The complete cDNA encoding the Lepidoglyphus destructor major allergen Lep d 1. FEBS Lett 370: 11-14, 1995.
75 Eriksson TLJ, Rasool O, Huecas S, Whitley P, Crameri R, Appenzeller U, Gafvelin G, van Hage-Hamsten M. Cloning of three new allergens from the dust mite Lepidoglyphus destructor using phage surface display technology. Eur. J. Biochem. 268: 287-294, 2001.
75A Saarne T, Kaiser L, Rasool O, Huecas S, van Hage-Hamsten M, Gafvelin G: Cloning and characterisation of two IgE-binding proteins, homologous to tropomyosin and a-tubulin, from the mite Lepidoglyphus destructor. Int Arch Allergy Immunol 130: 258-265, 2003.
75B Eriksson TL, Johansson E, Whitley P, Schmidt M, Elsayed S, van Hage-Hamsten M. Cloning and characterisation of a group II allergen from the dust mite Tyrophagus putrescentiae. Eur. J. Biochem. 251 (1-2), 443-447, 1998.
76. Rautiainen J, Rytkonen M, Pelkonen J, Pentikainen J, Perola O, Virtanen T, Zeiler T, Mantyjarvi R. BDA20, a major bovine dander allergen characterized at the sequence level is Bos d 2. Submitted.
77. Gjesing B, Lowenstein H. Immunochemistry of food antigens. Ann Allergy 53:602, 1984.
78. de Groot, H., K.G.H. Goei, P. van Swieten, and R.C. Aalberse. Affinity purification of a major and a minor allergen from dog extract: Serologic activity of affiity-purified Can f I and Can f I-depleted extract. J. Allergy Clin. Immunol. 87:1056-1065, 1991.
79. Konieczny, A. Personal communication; Immunologic Pharmaceutical Corp.
79A. Bulone, V. Separation of horse dander allergen proteins by two-dimensional electrophoresis. Molecular characterisation and identification of Equ c 2.0101 and Equ c 2.0102 as lipocalin proteins. Eur J Biochem 253: 202-211, 1998.
79B. Swiss-Prot acc. P81216, P81217.
79C. Dandeu J. P., Rabillon J., Divanovic A., Carmi-Leroy A., David B. (1993). Hydrophobic interaction chromatography for isolation and purification of Equ c 1, the horse major allergen. J. Chromatogr. 621:23-31.
79D. Goubran Botros H., Rabillon J., Grégoire C., David B., Dandeu J.P. 1998. Thiophilic absorption chromatography: purification of Equ c 2 and Equ c 3, two horse allergens from horse sweat. J. Chromatogr. B 710:57-65.
79E. Hilger C, Kohnen M, Grigioni F, Lehners C, Hentges F. Allergic cross-reactions between cat and pig serum albumin. Allergy 52:179-187, 1997; and Hilger C, Grigioni F, Hentges F. Sequence of the gene encoding cat (Felis domesticus) serum albumin. Gene 169:295-296, 1996.
79F. Ichikawa K, Vailes LD, Pomes A, Chapman MD. Molecular cloning, expression and modeling of cat allergen, cystatin (Fel d 3), a cysteine protease inhibitor. Clin Exp Allergy, In Press 2001.
80 Fahlbusch B, Rudeschko O, Szilagyi U, Schlott B, Henzgen M, Schlenvoigt G, Schubert H. Purification and partial characterization of the major allergen, Cav p 1, from guinea pig Cavia porcellus. Allergy 57: 417-422, 2002.
81 McDonald, B., M. C. Kuo, J. L. Ohman, and L. J. Rosenwasser. 1988. A 29 amino acid peptide derived from rat alpha 2 euglobulin triggers murine allergen specific human T cells (abst). J. Allergy Clin. Immunol. 83:251.
81A Clarke, A. J., P. M. Cissold, R. A. Shawi, P. Beattie, and J. Bishop. 1984. Structure of mouse urinary protein genes: differential splicing configurations in the 3'-non-coding region. EMBO J 3:1045-1052.
82. Longbottom, J. L. 1983. Chracterization of allergens from the urines of experimental animals. McMillan Press, London, pp. 525-529.
83. Laperche, Y., K. R. Lynch, K. P. Dolans, and P. Feigelsen. 1983. Tissue-specific control of alpha 2u globulin gene expression: constitutive synthesis in submaxillary gland. Cell 32:453-460.
83A. Bush RK, Sanchez H, Geisler D. 1999. Molecular cloning of a major Alternaria alternata allergen, rAlt a 2. J Allergy Clin Immunol 104:665-671.
83B. Aukrust L, Borch SM. 1979. Partial purification and characterization of two Cladosporium herbarum allergens. Int Arch Allergy Appl Immunol 60:68-79.
83C. Sward-Nordmo M, Paulsen BS, Wold JK. 1988. The glycoprotein allergen Ag-54 (Cla h II) from Cladosporium herbarum. Structural studies of the carbohydrate moiety. Int Arch Allergy Appl Immunol 85:288-294.
84 Shen, et al. J. Allergy Clin. Immunol. 103:S157, 1999.
84A Crameri R. Epidemiology and molecular basis of the involvement of Aspergillus fumigatus in allergic diseases. Contrib. Microbiol. Vol. 2, Karger, Basel (in press).
84B Shen, et al. (manuscript submitted), 1999
84C Shen HD, Ling WL, Tan MF, Wang SR, Chou H, Han SIH. Vacuolar serine proteinase: A major allergen of Aspergillus fumigatus. 10th International Congress of Immunology, Abstract, 1998.
85 Kumar A, Reddy LV, Sochanik A, Kurup VP. 1993. Isolation and characterization of a recombinant heat shock protein of Aspergillus fumigatus. J. Allergy Clin. Immunol. 91:1024-1030.
85A Saxena S, Madan T, Muralidhar K, Sarma PU. 2003. cDNA cloning, expression and characterization of an allergenic L3 ribosomal protein of Aspergillus fumigatus. Clin Exp Immunol 134:86-91.
85B Baur X, Melching-Kollmuss S, Koops F, Strassburger K, Zober A. IgE-mediated allergy to phytase -- a new animal feed additive. Allergy 57: 943-945, 2002.
86A Shen HD, Lin WL, Tsai JJ, Liaw SF, Han SH. 1996. Allergenic components in three different species of Penicillium: crossreactivity among major allergens. Clin Exp Allergy 26:444-451.
86B. Shen, et al. Abstract; The XVIII Congress of the European Academy of Allergology and Clinical Immunology, Brussels, Belgium, 3-7 July 1999.
87 Shen HD, Lin WL, Tam MF, Wang SR, Tzean SS, Huang MH, Han SH. Characterization of allergens from Penicillium oxalicum and P. notatum by immunoblotting and N-terminal amino acid sequence analysis. Clin Exp Allergy 29: 642-651, 1999.
87A Shen HD, Liaw SF, Lin WL, Ro LH, Yang HL, Han SH. Molecular cloning of cDNA coding for the 68 kDa allergen of Penicillium notatum using MoAbs. Clin Exp Allergy 25: 350-356, 1995.
87B Shen HD, Wang CW, Lin WL, Lai HY, Tam MF, Chou H, Wang SR, Han SH. cDNA cloning and immunologic characterization of Pen o 18, the vacuolar serine protease major allergen of Penicillium oxalicum. J Lab Clin Med 137: 115-124, 2001.
88 Woodfolk JA, Wheatley LM, Piyasena RV, Benjamin DC, Platts-Mills TA.1998. Trichophyton antigens associated with IgE antibodies and delayed type hypersensitivity. Sequence homology to two families of serine proteinases. J Biol Chem 273:29489-96.
88A Deuell, B., L.K. Arruda, M.L. Hayden, M.D. Chapman and T.A.E. Platts-Mills. 1991. Trichophyton tonsurans Allergen I. J. Immunol. 147:96-101.
89 Shen, H.D., K.B. Choo, H.H. Lee, J.C. Hsieh, and S.H. Han. 1991. The 40 kd allergen of Candida albicans is an alcohol dehydrogenease: molecular cloning and immunological analysis using monoclonal antibodies. Clin. Exp. Allergy 21:675-681.
89A Horner WE, Reese G, Lehrer SB. 1995. Identification of the allergen Psi c 2 from the basidiomycete Psilocybe cubensis as a fungal cyclophilin. Int Arch Allergy Immunol 107:298-300.
89B Chang CY, Chou H, Tam MF, Tang RB, Lai HY, Shen HD. Characterization of Enolase Allergen from Rhodotorula mucilaginosa. J Biomed Sci 9: 645-655, 2002.
90 Yasueda H, Hashida-Okado T, Saito A, Uchida K, Kuroda M, Onishi Y, Takahashi K, Yamaguchi H, Takesako K, Akiyama K. Identification and cloning of two novel allergens from the lipophilic yeast, Malassezia furfur. Biochem Biophys Res Commun 248: 240-244, 1998. NB:strain TIMM2782 (Teikyo University Institute for Medical Mycology) equal to strain CBS1878 (Central Bureau von Schimmelkulturen).
90A Onishi Y, Kuroda M, Yasueda H, Saito A, Sono-Koyama E, Tunasawa S, Hashida-Okado T, Yagihara T, Uchida K, Yamaguchi H, Akiyama K, Kato I, Takesako K. Two-dimensional electrophoresis of Malassezia allergens for atopic dermatitis and isolation of Mal f 4 homologs with mitochondrial malate dehydrogenase. Eur J Biochem 261: 148-154, 1999. NB: strain TIMM2782 (Teikyo University Institute for Medical Mycology) equal to strain CBS1878 (Central Bureau von Schimmelkulturen).
91 Schmidt M, Zargari A, Holt P, Lindbom L, Hellman U, Whitley P, van der Ploeg I, Harfast B, Scheynius A. The complete cDNA sequence and expression of the first major allergenic protein of Malassezia furfur, Mal f 1. Eur J Biochem 246:181-185, 1997. NB: strain ATCC no. 42132 (American Type Culture Collection).
91A Rasool O, Zargari A, Almqvist J, Eshaghi H, Whitley P, Scheynius A. Cloning, characterization and expression of complete coding sequences of three IgE binding Malassezia furfur allergens, Mal f 7, Mal f 8 and Mal f 9. Eur J Biochem 267: 4355-4361, 2000. NB: strain ATCC no. 42132 (American Type Culture Collection).
91B NB: ; strain 4625 (Indian Agricultural Research Institute, PUSA; New Delhi , India ).
92 Kuchler, K., M. Gmachl, M. J. Sippl, and G. Kreil. 1989. Analysis of the cDNA for phospholipase A2 from honey bee venom glands: The deduced amino acid sequence reveals homology to the corresponding vertebrate enzymes. Eur. J. Biochem. 184:249-254.
93 Gmachl, M., and G. Kreil. 1993. Bee venom hyaluronidase is homologous to a membrane protein of mammalian sperm. Proc. Natl. Acad. Sci. USA 90:3569-3573.
93A Hoffman DR. 1977. Allergens in bee venom III. Identification of allergen B as an acid phosphatase. J Allergy Clin. Immunol. 59:364-366.
94 Habermann, E. 1972. Bee and wasp venoms. Science 177:314-322.
95 Hoffman DR, Jacobson RS. 1996. Allergens in Hymenoptera venom XXVII: Bumblebee venom allergy and allergens. J. Allergy Clin. Immunol. 97:812-821.
95A Hoffman DR, El-Choufani AE, Smith MM, de Groot H. 2001. Occupational allergy to bumblebee venom: Allergens of Bombus terrestris. J Allergy Clin Immunol In press.
95B Helm R, Cockrell G, Stanley JS, Brenner RJ, Burks W, Bannon GA. 1996. Isolation and characterization of a clone encoding a majore allergen (Bla g Bd90K) involved in IgE mediated cockroach hypersensitivity. J Allerg Clin Immunol 98:172-180.
95C Pomes A, Melen E, Vailes LD, Retief JD, Arruda LK, Chapman MD. 1998. Novel allergen structures with tandem amino acid repeats derived from German and American cockroach. J Biol Chem 273:30801-30807.
96 Arruda LK, Vailes LD, Mann BJ, Shannon J, Fox JW, Vedvick TS, Hayden ML, Chapman MD. Molecular cloning of a major cockroach (Blattella germanica) allergen, Bla g 2. Sequence homology to the aspartic proteases. J Biol Chem 270:19563-19568, 1995.
97 Arruda LK, Vailes LD, Hayden ML, Benjamin DC, Chapman MD. Cloning of cockroach allergen, Bla g 4, identifies ligand binding proteins (or calycins) as a cause of IgE antibody responses. J Biol Chem 270:31196-31201, 1995.
98 Arruda LK, Vailes LD, Benjamin DC, Chapman MD. Molecular cloning of German Cockroach (Blattella germanica) allergens. Int Arch Allergy Immunol 107:295-297, 1995.
98A Wu CH, Wang NM, Lee MF, Kao CYY, Luo SF. 1998. Cloning of the American cockroach Cr-PII allergens: Evidence for the existence of cross-reactive allergens between species. J Allergy Clin Immunol 101:832-840.
98B Melen E, Pomés A, Vailes LD, Arruda LK, Chapman MD. 1999. Molecular cloning of Per a 1 and definition of the cross-reactive Group 1 cockroach allergens. J Allergy Clin Immunol 103:859-64.
98C Wu CH, Lee MF, Liao SC, Luo SF. Sequencing analysis of cDNA clones encoding the American cockroach Cr-PI allergens. J Biol Chem 271:17937-17943, 1996.
98D Wu CH, Lee MF, Wang NM, Luo SF. Sequencing and immunochemical characterization of the American cockroach Per a 3 (Cr-PI) isoallergenic variants. Molecular Immunol 34:1-8, 1997.
98E Santos ABR, Chapman MD, Aalberse RC, Vailes LD, Ferriani VPL, Oliver C, Rizzo MC, Naspitz CK, Arruda LK. 1999. Cockroach allergens and asthma in Brazil: Identification of tropomyosin as a major allergen with potential cross-reactivity with mite and shrimp allergens. J Allergy Clin Immunol 104:329-337.
98F Asturias JA, Gómez-Bayón N, Arilla MC, Martinez A, Palacios R, Sánchez-Gascón, Martinez J. 1999. Molecular characterization of American cockroach tropomyosin (Periplaneta americana allergen 7), a cross-reactive allergen. J Immunol 162:4342-4348.
99 Mazur, G., X. Baur, and V. Liebers. 1990. Hypersensitivity to hemoglobins of the Diptera family Chironomidae: Structural and functional studies of their immunogenic/allergenic sites. Monog. Allergy 28:121-137.
99A Moneo I, Vega JM, Caballero ML, Vega J, Alday E. Isolation and characterization of Tha p 1, a major allergen from the pine processionary caterpillar Thaumetopoea pityocampa. Allergy 58: 34-37, 2003.
100 Soldatova, L., L. Kochoumian, and T.P. King. 1993. Sequence similarity of a hornet (D. maculata) venom allergen phospholipase A1 with mammalian lipases. FEBS Letters 320:145-149.
101 Lu, G., L. Kochoumian and T.P. King. Whiteface hornet venom allergen hyaluronidase: cloning and its sequence similarity with other proteins (abst.). 1994. J. Allergy Clin. Immunol. 93:224.
102 Fang, K. S. F., M. Vitale, P. Fehlner, and T. P. King. 1988. cDNA cloning and primary structure of a white-faced hornet venom allergen, antigen 5. Proc. Natl. Acad. Sci., USA 85:895-899.
103 King, T. P., D. C. Moran, D. F. Wang, L. Kochoumian, and B.T. Chait. 1990. Structural studies of a hornet venom allergen antigen 5, Dol m V and its sequence similarity with other proteins. Prot. Seq. Data Anal. 3:263-266.
104. Lu, G., M. Villalba, M.R. Coscia, D.R. Hoffman, and T.P. King. 1993. Sequence analysis and antigen cross reactivity of a venom allergen antigen 5 from hornets, wasps and yellowjackets. J. Immunol. 150: 2823-2830.
105. King, T. P. and Lu, G. 1997. Unpublished data.
105A. King TP, Lu G, Gonzalez M, Qian N and Soldatova L. 1996. Yellow jacket venom allergens, hyaluronidase and phospholipase: sequence similarity and antigenic cross-reactivity with their hornet and wasp homologs and possible implications for clinical allergy. J. Allergy Clin. Immunol. 98:588-600.
106. Hoffman, D.R. 1993. Allergens in hymenoptera venom XXV: The amino acid sequences of antigen 5 molecules and the structural basis of antigenic cross-reactivity. J. Allergy Clin. Immunol. 92:707-716.
107. Hoffman DR. 1992. Unpublished data.
108. Hoffman DR. The complete amino acid sequence of a yellowjacket venom phospholipase (abst). J. Allergy Clin. Immunol. 91:187, 1993.
109. Jacobson RS, Hoffman DR, Kemeny DM. The cross-reactivity between bee and vespid hyaluronidases has a structural basis (abst). J. Allergy Clin. Immunol. 89:292, 1992.
110. Hoffman DR. Allergens in Hymenoptera venom XXIV: The amino acid sequences of imported fire ant venom allergens Sol i II, Sol i III, and Sol i IV. J. Allergy Clin. Immunol 91: 71-78, 1993.
111. Schmidt M, Walker RB, Hoffman DR, McConnell TJ. Nucleotide sequence of cDNA encoding the fire ant venom protein Sol i II. FEBS Letters 319: 138-140, 1993.
111A. Paddock CD, McKerrow JH, Hansell E, Foreman KW, Hsieh I, Marshall N. Identification, cloning, and recombinant expression of procalin, a major triatomine allergen. J Immunol 167: 2694-2699, 2001.
112. Elsayed S, Bennich H. The primary structure of Allergen M from cod. Scand J Immunol 3: 683-686, 1974.
113. Elsayed S, Aas K, Sletten K, Johansson SGO. Tryptic cleavage of a homogeneous cod fish allergen and isolation of two active polypeptide fragments. Immunochemistry 9: 647-661, 1972.
114. Hoffman, D. R. 1983. Immunochemical identification of the allergens in egg white. J. Allergy Clin. Immunol. 71: 481-486.
115. Langeland, T. 1983. A clinical and immunological study of allergy to hen's egg white. IV. specific IgE antibodies to individual allergens in hen's egg white related to clinical and immunolgical parameters in egg-allergic patients. Allergy 38:493-500.
116 Daul CB, Slattery M, Morgan JE, Lehrer SB. 1993. Common crustacea allergens: identification of B cell epitopes with the shrimp specific monoclonal antibodies. In: "Molecular Biology and Immunology of Allergens" (D. Kraft and A. Sehon, eds.). CRC Press, Boca Raton. pp. 291-293.
116A Shanti KN, Martin BM, Nagpal S, Metcalfe DD, Subba Rao PV. Identification of tropomyosin as the major shrimp allergen and characterization of its IgE-binding epitopes. J. Immunol. 151: 5354-5363, 1993.
117 Yu CJ, Lin YF, Chiang BL, Chow LP. Proteomics and Immunological Analysis of a Novel Shrimp Allergen, Pen m 2. J Immunol 170: 445-453, 2003.
117A Miyazawa M, Fukamachi H, Inagaki Y, Reese G, Daul CB, Lehrer SB, Inouye S, Sakaguchi M. Identification of the first major allergen of a squid (Todarodes pacificus). J. Allergy Clin. Immunol. 98: 948-953, 1996.
117B Asturias JA, Eraso E, Arilla MC, Gomez-Bayon N, Inacio F, Martinez A. Cloning, isolation, and IgE-binding properties of Helix aspersa (brown garden snail) tropomyosin. Int Arch Allergy Immunol 128: 90-96, 2002.
117C Lopata AL, Zinn C, Potter PC. Characteristics of hypersensitivity reactions and identification of a unique 49 kd IgE-binding protein (Hal-m-1) in abalone (Haliotis midae). J. Allergy Clin. Immunol. 100: 642-648, 1997.
117D Hoffmann-Sommergruber K, O'Riordain G, Ahorn H, Ebner C, Laimer Da Camara Machado M, Pühringer H, Scheiner O, Breiteneder H. Molecular characterization of Dau c 1, the Bet v 1 homologous protein from carrot and its cross-reactivity with Bet v 1 and Api g 1. Clin. Exp. Allergy 29: 840-847, 1999.
118 Monsalve RI, Gonzalez de la Pena MA, Menendez-Arias L, Lopez-Otin C, Villalba M, Rodriguez R. Characterization of a new mustard allergen, Bra j IE. Detection of an allergenic epitope. Biochem. J. 293: 625-632 1993.
118A. Monsalve RI, Gonzalez de la Pena MA, Lopez-Otin C, Fiandor A, Fernandez C, Villalba M, Rodriguez R. 1997. Detection, isolation and complete amino acid sequence of an aeroallergenic protein from rapeseed flour. Clin Exp Allergy 27:833-841.
119. Mena, M., R. Sanchez-Monge, L. Gomez, G. Salcedo, and P. Carbonero. A major barley allergen associated with baker's asthma disease is a glycosylated monomeric inhibitor of insect alpha-amylase: cDNA cloning and chromosomal location of the gene. Plant Molec. Biol. 20: 451-458, 1992.
119A. Palosuo K, Varjonen E, Kekki OM, Klemola T, Kalkkinen N, Alenius H, Reunala T. Wheat omega-5 gliadin is a major allergen in children with immediate allergy to ingested wheat. J. Allergy Clin. Immunol. 108: 634-638, 2001.
119B. Xu H, Theerakulpisut P, Goulding N, Suphioglu C, Singh M. B. Bhalla P. L. Cloning expression and immunological characterization of Ory s 1, the major allergen of rice pollen. Gene 164: 255-259, 1995.
119C. Pastorello EA, Ortolani C, Farioli L, Pravettoni V, Ispano M, Borga A, Bengtsson A, Incorvaia C, Berti C, Zanussi C. Allergenic cross-reactivity among peach, apricot, plum, and cherry in patients with oral allergy syndrome: an in vivo and in vitro study. J. Allergy Clin. Immunol. 94: 699-707, 1994.
119D. Diaz-Perales A, Tabar AI, Sanchez-Monge R, Garcia BE, Gomez B, Barber D, Salcedo G. Characterization of asparagus allergens: a relevant role of lipid transfer proteins. J Allergy Clin Immunol 110: 790-796, 2002.
119E Galleguillos F, Rodriguez JC. Asthma caused by bromelin inhalation. Clin Allergy 8: 21-24, 1978.
119F Baur X. Studies on the specificity of human IgE-antibodies to the plant proteases papain and bromelain. Clin Allergy 9: 451-457, 1979.
119G Gailhofer G, Wilders-Truschnig M, Smolle J, Ludvan M. Asthma caused by bromelain: an occupational allergy. Clin Allergy 18: 445-450, 1988.
120. Menendez-Arias, L., I. Moneo, J. Dominguez, and R. Rodriguez. 1988. Primary structure of the major allergen of yellow mustard (Sinapis alba L.) seed, Sin a I. Eur. J. Biochem. 177:159-166.
120A Gonzalez R, Varela J, Carreira J, Polo F. Soybean hydrophobic protein and soybean hull allergy. Lancet 346:48-49, 1995.
120B Kleine-Tebbe J, Vogel L, Crowell DN, Haustein UF, Vieths S. Severe oral allergy syndrome and anaphylactic reactions caused by a Bet v 1- related PR-10 protein in soybean, SAM22. J Allergy Clin Immun ol 110: 797-804, 2002.
120C Sanchez-Monge R, Pascual CY, Diaz-Perales A, Fernandez-Crespo J, Martin-Esteban M, Salcedo G. Isolation and characterization of relevant allergens from boiled lentils. J. Allergy Clin. Immunol. 106: 955-961, 2000.
121 Gavrovic-Jankulovic M, cIrkovic T, Vuckovic O, Atanaskovic-Markovic M, Petersen A, Gojgic G, Burazer L, Jankov RM. Isolation and biochemical characterization of a thaumatin-like kiwi allergen. J Allergy Clin Immun ol 110: 805-810, 2002.
121A Pastorello EA, Varin E, Farioli L, Pravettoni V, Ortolani C, Trambaioli C, Fortunato D, Giuffrida MG, Rivolta F, Robino A, Calamari AM, Lacava L, Conti A. The major allergen of sesame seeds (Sesamum indicum) is a 2S albumin. J. Chromatogr. B Biomed. Sci. Appl. 756: 85-93, 2001.
121B Moneo I, Caballero ML, Gomez F, Ortega E, Alonso MJ. Isolation and characterization of a major allergen from the fish parasite Anisakis simplex. J. Allergy Clin. Immunol. 106: 177-182, 2000.
121C Asturias JA, Eraso E, Martinez A. 2000. Is tropomysoin an allergen in Anisakis? Allergy 55:898-890.
122 Christie, J. F., B. Dunbar, I. Davidson, and M. W. Kennedy. 1990. N-terminal amino acid sequence identity between a major allergen of Ascaris lumbricoides and Ascaris suum and MHC-restricted IgE responses to it. Immunology 69:596-602.
122A Baur X, Konig G, Bencze K, Fruhmann G. Clinical symptoms and results of skin test, RAST and bronchial provocation test in thirty-three papain workers: evidence for strong immunogenic potency and clinically relevant 'proteolytic effects of airborne papain'. Clin Allergy 12: 9-17, 1982.
122B Onizuka R, Kamiya H, Muramoto K, Goto R, Inoue K, Kumamoto K, Nakajima Y, Iida S, Ishigami F. Purification of the major allergen of red soft coral (Dendronephthya nipponica). Int Arch Allergy Immunol 125: 135-143, 2001.
123. Czuppon AB, Chen Z, Rennert S, Engelke T, Meyer HE, Heber M, Baur X. The rubber elongation factor of rubber trees (Hevea brasiliensis) is the major allergen in latex. J Allergy Clin Immunol 92:690-697, 1993.
124. Attanayaka DPSTG, Kekwick RGO, Franklin FCH. 1991. Molecular cloning and nucleotide sequencing of the rubber elongation factor gene from hevea brasiliensis. Plant Mol Biol 16:1079-1081.
125. Chye ML, Cheung KY. 1995. J 1,3-glucanase is highly expressed in Laticifers of Hevea brasiliensis. Plant Mol Biol 26:397-402.
126. Alenius H, Palosuo T, Kelly K, Kurup V, Reunala T, Makinen-Kiljunen S, Turjanmaa K Fink J. 1993. IgE reactivity to 14-kD and 27-kD natural rubber proteins in Latex-allergic children with Spina bifida and other congenital anomalies. Int Arch Allergy Immunol 102:61-66.
127. Yeang HY, Cheong KF, Sunderasan E, Hamzah S, Chew NP, Hamid S, Hamilton RG, Cardosa MJ. 1996. The 14.6 kD (REF, Hev b 1) and 24 kD (Hev b 3) rubber particle proteins are recognized by IgE from Spina Bifida patients with Latex allergy. J Allerg Clin Immunol in press.
128. Sunderasan E, Hamzah S, Hamid S, Ward MA, Yeang HY, Cardosa MJ. 1995. Latex B-serum J-1,3-glucanase (Hev b 2) and a component of the microhelix (Hev b 4) are major Latex allergens. J nat Rubb Res 10:82-99.

With the present splicing/head to tail modification significant reduction in allergenic activity can be obtained. Depending on the method, this activity can mostly be extinguished from the wild-type protein allergen. According to a preferred embodiment of the present invention, reduction in allergenic activity is measured by a reduction of inhibition of IgE binding capacity of at least 10 %, preferably at least 20 %, especially at least 30 %, compared to the wild-type allergen. A preferred method is shown in the example section below.

An alternative, but also preferred way for defining the reduction in allergenic activity uses measurement of IgE binding. Lack of binding of IgE antibodies of allergen sensitised patient's sera to a dot blot of said derivative is taken as an indication of most significant reduction. Also this method is shown in the example section below.

The derivatives obtained according to the present invention may be easily combined with a pharmaceutically acceptable excipient and finished to a pharmaceutical preparation.

Preferably, the derivatives are combined with a suitable vaccine adjuvant and finished to a pharmaceutically acceptable vaccine preparation.

According to a preferred embodiment, the derivatives according to the present invention are combined with further allergens to a combination vaccine. Such allergens are preferably wild-type allergens, especially a mixture of wild-type allergens, recombinant wild-type allergens, derivatives of wild-type protein allergens or mixtures thereof. Such mixtures may be made specifically for the needs (allergen profile) of a certain patient.

In a preferred embodiment, such a pharmaceutical preparation further contains an allergen extract.

According to another aspect of the present invention, an allergen derivative of a wild-type protein allergen is provided, said wild-type protein allergen having an amino acid sequence of 1 to Z, characterized in that said derivative adjacently contains - in N-terminus to C-terminus orientation - the two wild-type allergen fragments X to Z and 1 to X, said two wild-type allergen fragments having reduced allergenic activity or lacking allergenic activity.

Preferably, the allergen derivative according to the present invention is characterized in that X to Z and 1 to X are at least 30 amino acid residues long, preferably at least 50 amino acid residues, especially at least 60 amino acid residues.

It is even more preferred, if X to Z and 1 to X differ in length by 50 % or less, preferably by 30 % or less, especially by 20 % or less.

Specifically preferred allergen derivatives according to the present invention are selected from a type I allergen, preferably from an allergen of table A, more preferred of timothy grass (Phelum pratense) pollen, especially Ph1 p 12, birch (Betula verrucosa) pollen, especially Bet v 2 and Bet v 4, yellow jacket (Vespula vulgaris) venom, paper wasp (Polistes annularis) venom, Parietaria judaica pollen, ryegrass pollen, dustmite allergens, especially Der p 2, etc..

Preferably, the derivatives according to the present invention are provided as a allergen composition wherein not only one allergen is present, but two or more. The present derivatives may also be mixed with allergen extracts which are supplemented by the derivatives of the present invention to substitute for the lack of sufficient amounts of specific allergens in the natural extracts. Mixtures of allergens are specifically needed in patients which have allergenic reactions to not only one allergen. It is therefore preferred to provide the present derivatives as in combination with further (other) allergens to a combination vaccine.

The allergen derivatives according to the present invention may therefore be preferably combined with wild-type allergen to an allergen composition, especially a mixture of a wild-type allergens, recombinant wild-type allergens, derivatives of wild-type protein allergens or mixtures thereof (each of the same and/or different allergen and/or isoforms or mutants thereof; as long as an overall reduction of allergenic activity, compared to the wild-type protein or recombinant allergen is given in the preparation as a whole).

Preferably, the present preparation further contains an allergen extract.

The allergen or allergen composition according to the present invention preferably contains a pharmaceutically acceptable excipient.

Another aspect of the present invention relates to the use of an allergen derivative according to the present invention for the preparation of an allergen specific immunotherapy medicament.

Yet another aspect of the present invention relates to the use of an allergen derivative or an allergen composition according to the present invention for the preparation of a medicament for the passive immunisation.

Another aspect of the present invention relates to the use of an allergen derivative or an allergen composition according to the present invention for the preparation of a medicament for the prophylactic immunisation.

The allergen derivatives and compositions according to the present invention can be used for the prophylactic immunisation of individuals leading to an effective prevention of allergy. Since the allergen derivatives and compositions according to the present invention, like Der p 2 allergen derivatives, show a reduced allergic immune response compared to the wild-type allergen, they do not lead to undesired side effects. Advantageously such a medicament may be administered to children at the age of 1 to 3 years. Such a vaccination before said child will get in contact with allergens prevents the formation of allergen specific IgE antibodies in said child.

Preferably, the medicament further contains other suitable ingredients, such as adjuvants, diluents, preservatives, etc..

According to a preferred embodiment of the present invention the medicament comprises 10 ng to 1 g, preferably 100 ng to 10 mg, especially 0,5 µg to 200 µg of said recombinant allergen derivative per application dose. Preferred ways of administration include all standard administration regimes described and suggested for vaccination in general and allergy immunotherapy specifically (orally, transdermally, intraveneously, intranasally, via mucosa, etc). The present invention includes a method for treating and preventing allergy by administering an effective amount of the pharmaceutical preparations according to the present invention.

Another aspect of the present invention relates to a method for producing an allergen derivative according to the present invention which is characterized in by the following steps:
- providing a DNA molecule encoding an allergen derivative according to the present invention,
- transforming a host cell with said DNA molecule and
- expressing said derivative in said host cell and isolating said derivative.

Preferably, said host is a host with high expression capacity.

As used herein, a "host with high expression capacity" is a host which expresses a protein of interest in an amount of at least 10mg/l culture, preferably of at least 15mg/l, more preferably of at least 20mg/l. Of course, the expression capacity depends also on the selected host and expression system (e.g. vector). Preferred hosts according to the present invention are E.coli, Pichia pastoris, Baciullus subtilis, pant cells (e.g. derived form tabacco) etc..

Of course, the allergen derivatives according to the present invention can also be produced by any other suitable method, especially chemical synthesis or semi-chemical synthesis.

Another aspect of the present invention relates to the use of a profilin derivative obtainable from a first wild-type profilin molecule by a method according to the present invention or an allergen derivative of a first wild-type profilin molecule according to the present invention for the manufacture of a medicament for the prevention or the treatment of allergic diseases caused by a second wild-type profilin molecule.

It turned surprisingly out that antibodies induced by an directed to profilin derivatives of a first wild-type profilin molecule according to the present invention bind also to other wild-type profilin molecules. Therefore said derivatives can be employed for the treatment or prevention of a number of allergic diseases. Such profilin derivatives may be used as broad spectrum vaccines which allow to immunize individuals with only one or two immunogenic molecules. Profilin represents an allergen that is expressed in all eukaryotic cells and thus represents a pan-allergen that might induce inhalative allergies (e.g. rhinoconjunctivits, asthma) as well as oral allergy syndromes after oral ingestion (itching and swelling of lips and the tounge) in sensitized patients.

For instance, the reshuffled Phl p 12-derivative, MP12, induces IgG antibodies after immunization that recognize profilins from both pollens as well as form plant-derived food. MP 12-induced antibodies inhibit patients' serum IgE binding to profilins from pollens and also to plant food-derived profilin. Thus, the MP12 as well as other reshuffled profilin molecules are suitable for the treatment of pollen-food cross-sensitization attributable to profilin allergy.

According to a preferred embodiment said first and said second profilin molecules are selected from the group consisting of Phl p 12, Bet v 2, Art v 4, Ana c, Api g 4, Mus xp 1, Cor a 2, and Dau c 4.

Especially these allergens are suited to be used according to the present invention because of their structural similarities. However, it is obvious that also other allergens which share structural similarities among each other can be used accordingly.

Said first profilin molecule is preferably Phl p 12 and said second profilin molecule is preferably selected from the group consisting of Bet v 2, Art v 4, Ana c, Api g 4, Mus xp 1, Cor a 2, and Dau c 4.

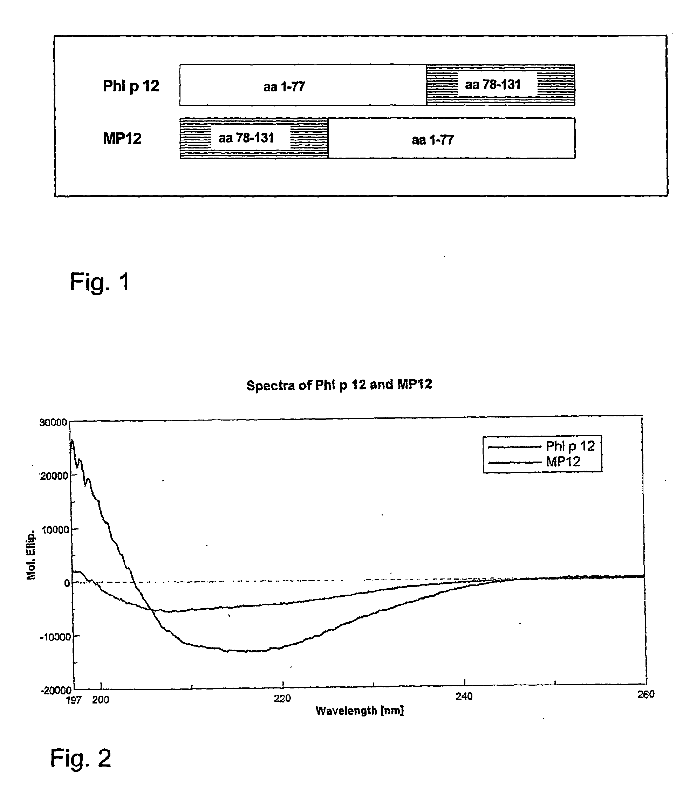

Experiments revealed that especially derivatives of Phl p 12 can be used as broad spectrum vaccines. A particular preferred derivative consists of a fusion protein, wherein amino acids 1 to 77 of the wild-type Phl p 12 are N-terminally fused to amino acids 78 to 131 (see Fig. 1).

Profilin derivatives of Bet v 2, Art v 4, Ana c, Api g 4, Mus xp 1, Cor a 2, and Dau c 4 as disclosed herein and obtainable by a method according to the present invention are preferably used for the treatment and/or prevention of pollen-food sensitization attributable to profilin allergy.
The present invention is further described by the following examples and the drawing figures, yet without being restricted thereto.
**Fig. 1** shows a schematic representation of the primary structure of MP12 (a reshuffled Phl p 12 allergen according to the present invention) compared to Phl p 12 wild-type;
**Fig. 2** shows CD spectra of Phl p 12 wild-type and MP12. The mean residue ellipticity [Θ] (y-axis) of Phl p 12 and the derivative MP12 is shown for a range of wavelengths (x-axis);

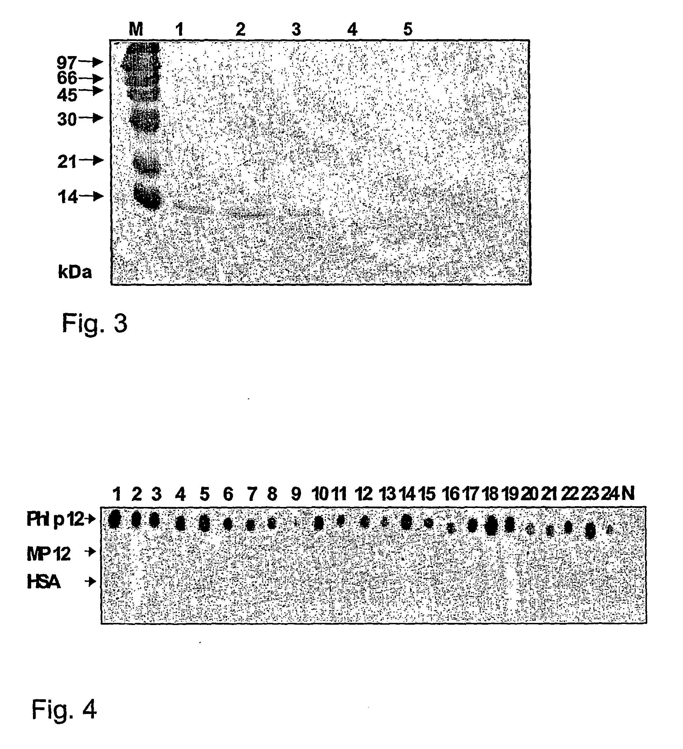

**Fig. 3** shows Coomassie staining of a 14% SDS PAGE loaded with fractions of recombinant MP12 that was exposed to a polyproline column. Lane M represents the molecular weight marker, lane 1 represents the flow-through fraction, lanes 2-4 wash fractions, lanes 5-6 elution fractions. Molecular weights (kDa) are indicated on the left margin;
**Fig. 4** shows IgE reactivity of nitrocellulose-dotted Phl p 12 and MP12. Dotted proteins, as well as human serum albumin (HSA) for negative control purposes, were exposed to sera from 24 Phl p 12-allergic patients (lanes 1-24). Lane N represents serum from a non-allergic control individual. Bound IgE antibodies were detected with anti-human IgE antibodies;

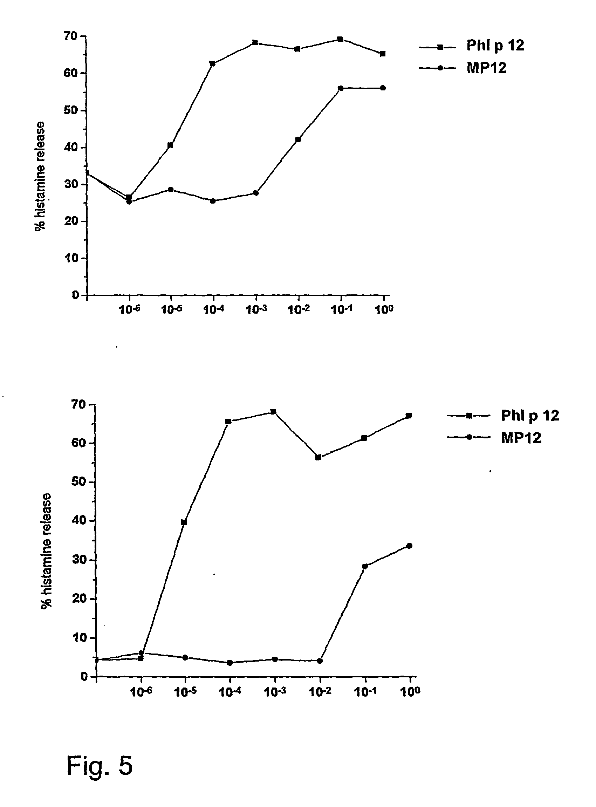

**Fig. 5** shows induction of basophil histamine release in two Phl p 12-allergic patients. Patients' granulocytes were incubated with various concentrations (x-axis) of Phl p 12 (squares) and MP12 (circles). The percentage of total histamine released into the supernatant is displayed on the y-axis;

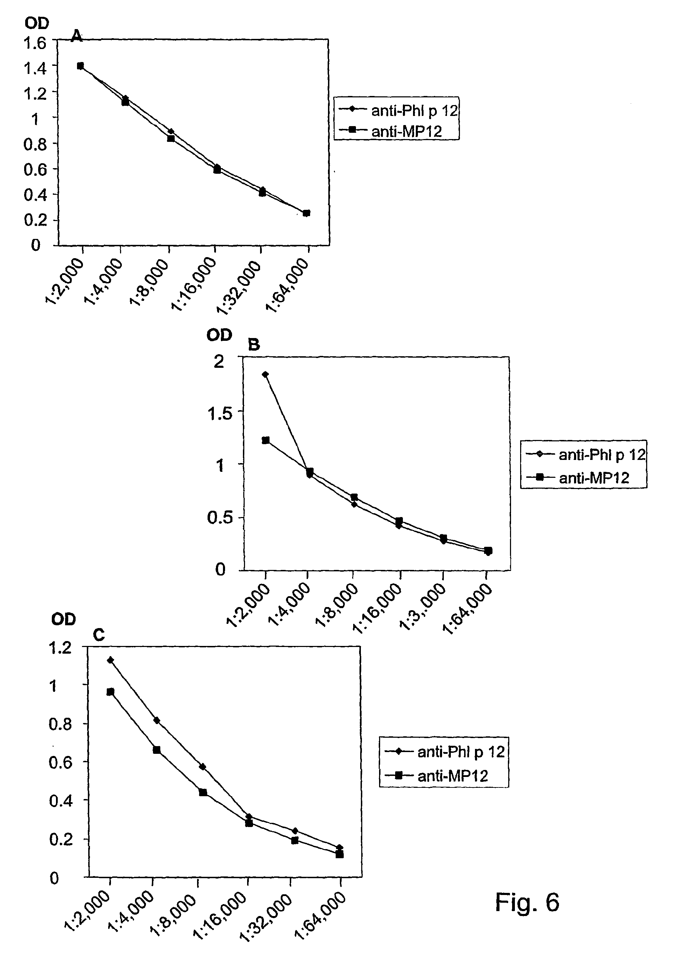

**Fig. 6** shows reactivity of rabbit antisera with profilins from timothy grass, birch and mugwort pollen. Rabbit antisera raised against Phl p 12 (diamonds) and MP12 (squares) were tested for reactivity to Phl p 12 (A), Bet v 2 (B), and mugwort profilin (C) by ELISA. Dilutions of sera are shown on the x-axis, the corresponding OD values on the y-axis. The corresponding preimmune sera did not display any reactivity;

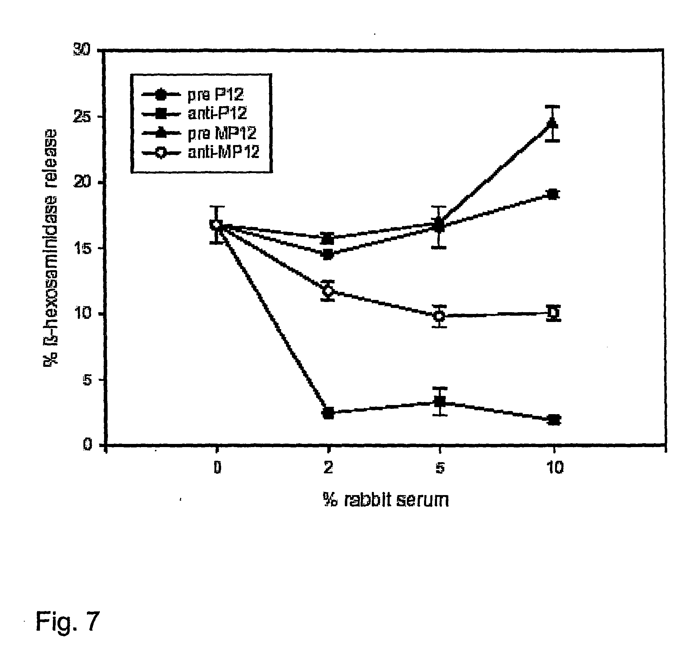

**Fig. 7** shows inhibition of rPhl p 12-induced basophil degranulation by anti-rPhl p 12 (P12) and anti-MP12-induced IgG. Rat basophils had been loaded with Phl p 12-specific mouse IgE;

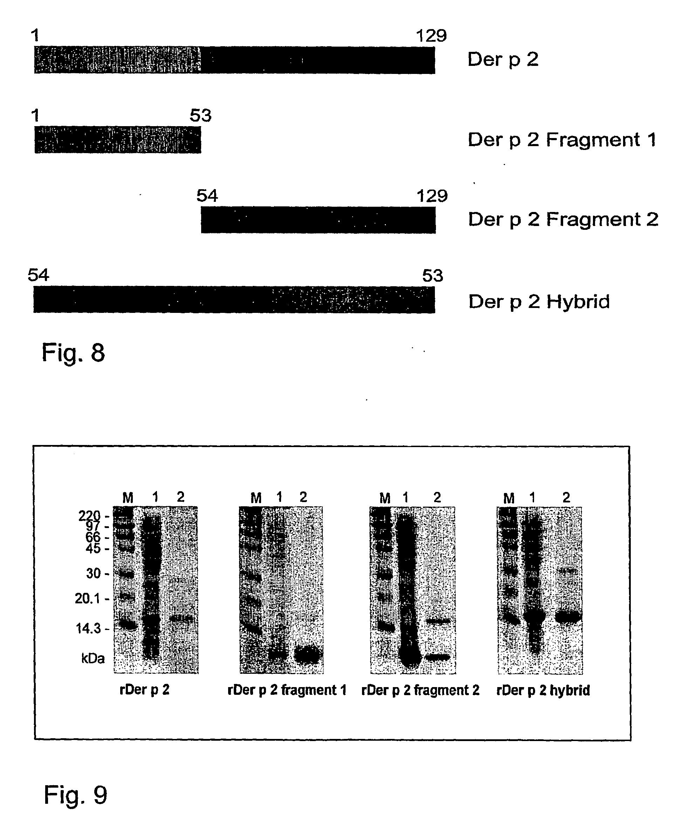

**Fig. 8** shows a schematic representation of the primary structure and generation of Der p 2 Hybrid (a reshuffled Der p 2 allergen according to the present invention) compared to Der p 2 wild-type;
**Fig. 9** shows Coomassie-stained SDS-PAGE containing protein extracts of BL21 (DE3) expressing rDer p 2 and rDer p 2 derivatives as his-tagged proteins (lanes 1), purified rDer p 2, rDer p 2 fragments and rDer p 2 hybrid (lanes 2), and a molecular marker (lanes M).

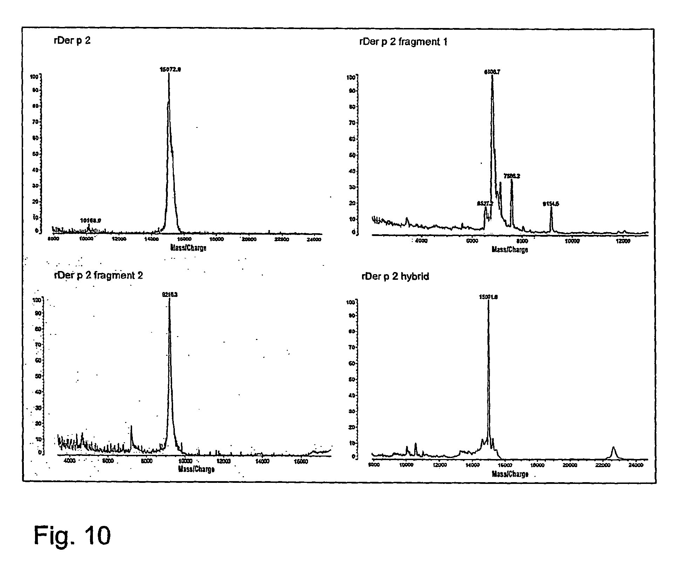

**Fig. 10** shows a mass spectroscopical analysis of purified rDer p 2 and rDer p 2 derivatives. The x-axes show the mass/charge ratios and the signal intensities are displayed on the y-axes as percentages of the most intensive signals.

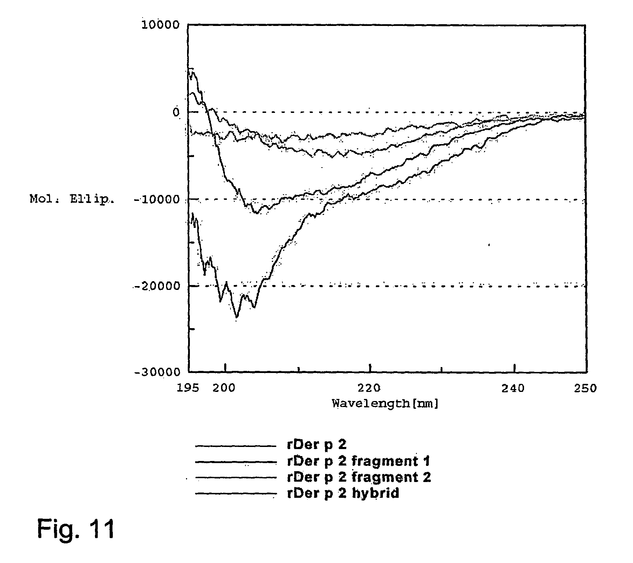

**Fig. 11** shows far ultraviolet CD spectra of purified recombinant Der p 2, rDer p 2 fragments and rDer p 2 hybrid. The spectra of the proteins are expressed as mean residue ellipticities (y-axis) at given wavelengths (x-axis).

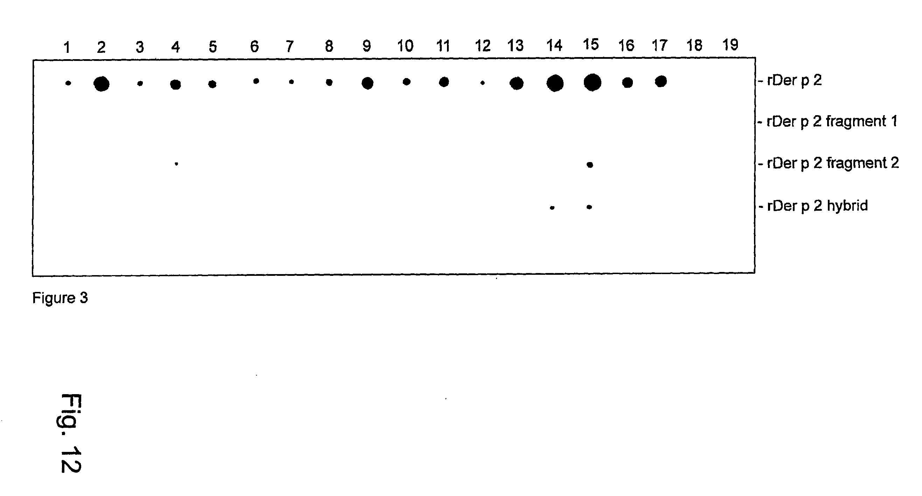

**Fig. 12** shows IgE-recognition of recombinant Der p 2 and recombinant Der p 2 derivatives. Sera from 17 mite allergic individuals (lanes 1-17), a non-allergic individual (lane 18) and buffer without serum (lane 19) were tested for IgE reactivity with dot-blotted recombinant Der p 2, rDer p 2 fragments, rDer p 2 hybrid and BSA. Bound IgE was detected with 125I-labeled anti-human IgE antibodies and visualized by autoradiography.

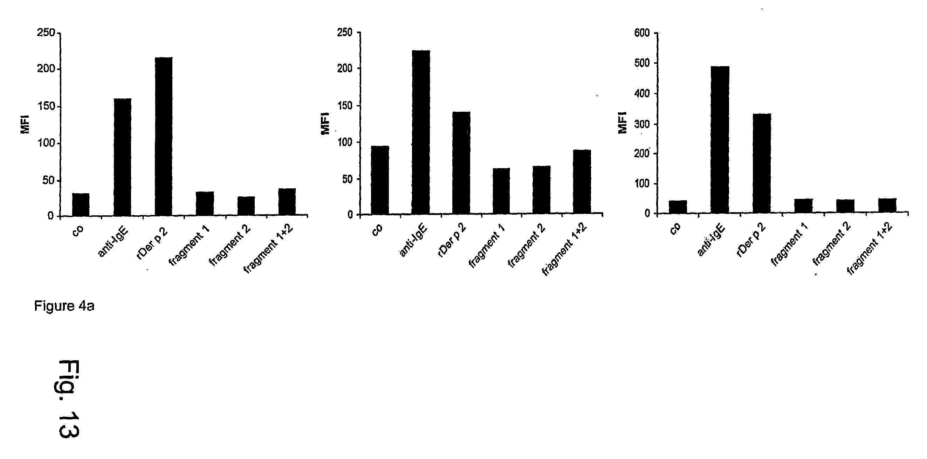

**Fig. 13** shows basophil activation by recombinant Der p 2 and rDer p 2 derivatives as measured by CD203c expression. Blood samples from 10 mite-allergic patients were exposed to 10µg/ml recombinant rDer p 2, each of the Der p 2 fragments, a mixture of the fragments, αIgE or buffer. The results of three representative patients are shown. CD203c expression was determined by FACS analysis and is displayed as mean fluorescence index (MFI).

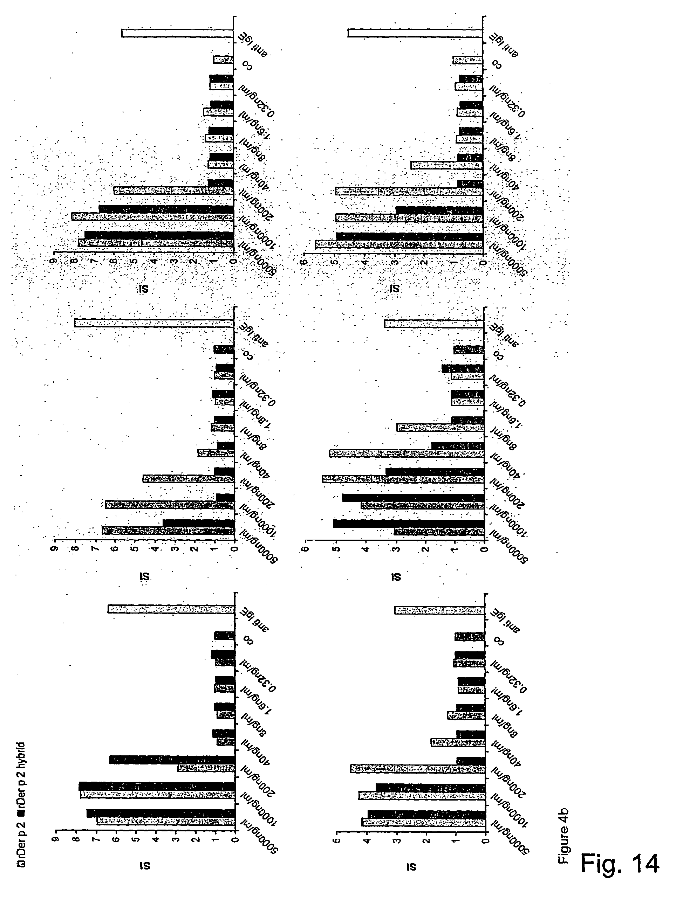

**Fig. 14** shows basophil activation by recombinant Der p 2 and rDer p 2 derivatives as measured by CD203c expression. Blood samples from the same 10 mite allergic patients were exposed to several concentrations of rDer p 2 and rDer p 2 hybrid, aIgE or buffer (x-axes). The results of six representative patients are shown. CD203c expression was determined by FACS analysis and is displayed as stimulation index (SI).

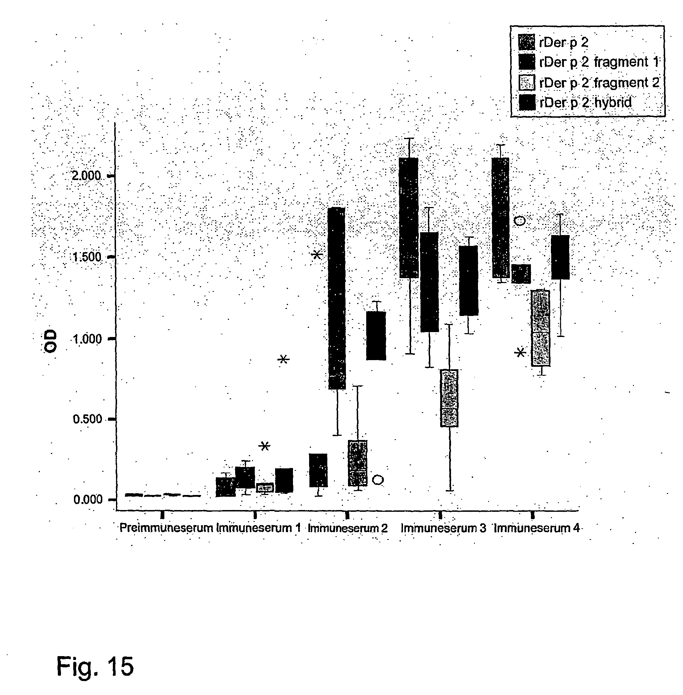

**Fig. 15** shows the evolution of Der p 2-specific IgG₁ induced by immunisation of mice with rDer p 2 and rDer p 2 derivatives. Groups of five mice each were immunized with purified rDer p 2 or rDer p 2 derivatives and induced IgG₁ antibodies were determined by ELISA. The optical density values (OD 405nm) displayed on the y-axis correspond to the level of IgG₁ antibodies in the mouse sera. The results are shown as box plots where 50% of the values are within the boxes and non-outliers between the bars. Lines within the boxes indicate the median values. Open circles and stars indicate outliers and extremes of each mouse group.

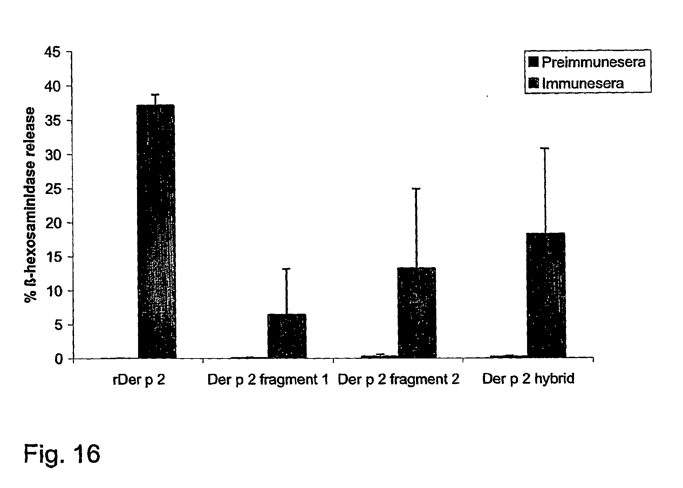

**Fig. 16** shows the low in vivo allergenic activity of rDer p 2 derivatives visualized by β-hexosaminidase release from RBL cells. Rat basophil leukemia (RBL) cells were loaded with mouse sera obtained before (Preimmunesera) and after (Immunesera) immunization with rDer p 2 wild-type allergen and rDer p 2 derivatives. Release of β-hexosaminidase was induced with rDer p 2 and is displayed as percentage of total β-hexosaminidase release (mean values ±SD for the five sera from each mouse group) (y-axis).

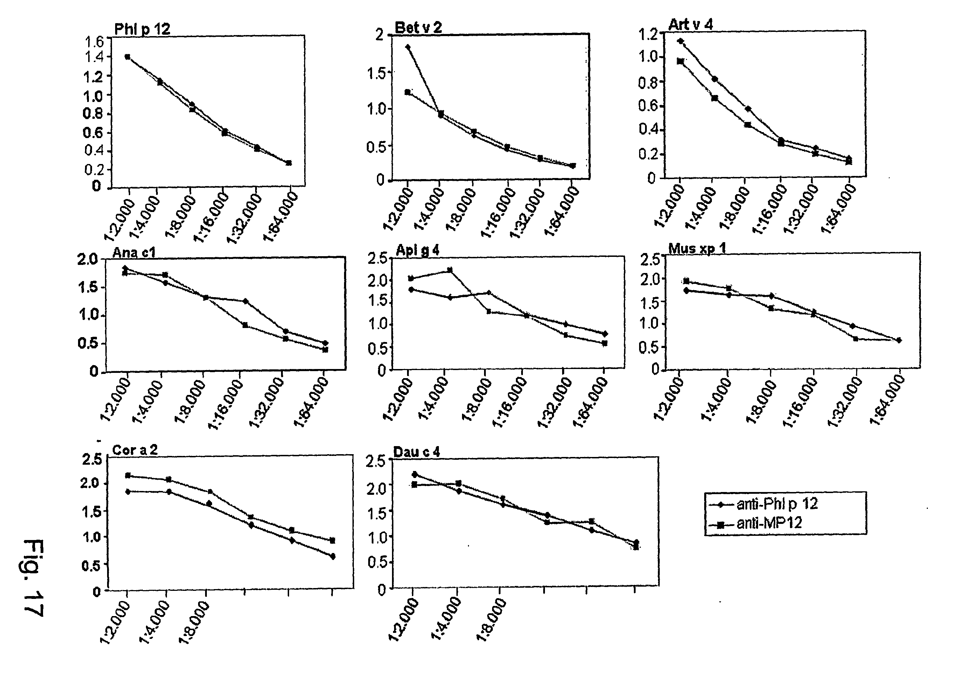

**Fig. 17** shows reactivity of rabbit antisera with profilins from timothy grass pollen (Phl p 12), birch pollen (Bet v 2), mugwort pollen (Art v 4), cashew nut (Ana c ), celery (Api g 4), banana (Mus xp 1), hazelnut (Cor a 2), and carrot (Dau c 4). Rabbit antisera raised against Phl p 12 (diamonds) and MP12 (squares) were tested for reactivity to said profilins by ELISA. Dilutions of sera are shown on the x-axis, the corresponding OD values on the y-axis. The corresponding preimmune sera did not display any reactivity.

### EXAMPLES:

In examples 1 to 5 the principles of the present invention are exemplified by a profilin allergen, timothy grass pollen profilin Phl p 12. Examples 6 to 11 relate to the main mite (Dermatophagoides pteronyssinus) allergen, Der p 2. Examples 12 and 13 show the cross reactivity of Phl p 12 with profilins of other sources than timothy grass pollen, demonstrating consequently the suitability for using Phl p 12 derivatives as vaccines for allergic diseases caused by other profilins.

### Example 1: Characterisation of a hypoallergenic derivative from timothy grass pollen profilin

### a) Generation, expression and purification of a hypoallergenic variant from timothy grass pollen profilin, Phl p 12

Overlapping PCR technique was used for engineering a reshuffled Phl p 12-derivative. PCR template was the cDNA coding for timothy grass pollen profilin, Phl p 12, subcloned in pet17b expression vector. The following primers were used to generate two PCR fragments containing overlapping sequences as well as NdeI and EcoRI restriction sites and a sequence coding for a C-terminal 6x Histidin residue for protein purification. For fragment 1 primer MDE-1: 5'CATATGAGGCCCGGCGCGGTCATC3' and primer MDE-2: 5'GTACGTCTGCCACGCCATCATGCCTTGTTCAAC3' were used, for fragment 2, primer MABC-1: 5'GTTGAACAAGGCATGATGTCGTG-GCAGACG3' and primer MABC-2: 5'GAATTCTTAATGGTGATGGTGATGGTGACCCT-GGATGACCATGTA3' were used. In the next step, both PCR products obtained as described were used as templates for the overlapping PCR reaction using primer MDE-1 and MABC-2 to generate the DNA coding for the Phl p 12 derivative (i.e., MP12) (schematically represented in Fig. 1). The MP-12 encoding DNA was cloned into pBluescript vector system (Stratagene) and DNA sequence was confirmed by double-strand sequencing (MWG Biotech, Germany).

For protein purification, MP12-encoding cDNA had to be subcloned into an pet17b expression vector system using NdeI and EcoRI restricition enzymes and the DNA sequence was again confirmed by double-strand sequencing (MWG Biotech).

For protein purification MP-12 was expressed in Escherichia coli BL21 (DE3) (Stratagene, East Kew, Australia) in liquid culture. E.coli were grown to an OD₆₀₀ of 0.4 in LB-medium containing 100 mg/l ampicillin. The expression of recombinant proteins was induced by adding isopropyl-b-thiogalactopyranoside to a final concentration of 1 mM and further culturing for additional 4 hours at 37°C. E.coli cells from a 500 ml culture were harvested by centrifugation, resuspended in buffer A (100mM NaH₂PO₄, 10mM Tris, 8M Urea, pH 7.5). After centrifugation at 20.000rpm, 30 min, the supernatant was transferred to a Ni-NTA agarose column (Quiagen, Hilden, Germany) and elution of the 6x His-tagged MP12 protein was performed using buffer A with decreasing pH values. The protein eluted at a pH of 4.9 and was subsequently refolded by stepwise dialysis against buffer A, pH 7.5, containing 6 - 0 M Urea. The final dialysis step was done against phosphate buffered saline (PBS), where MP12 was soluble as shown by centrifugation experiments.

Protein purity was confirmed by SDS PAGE and quantification was performed using a Micro BCA kit (Pierce, USA).

### b) Secondary structure analysis

Circular dichroism (CD) measurements were carried out on a Jasco J-715 spectropolarimeter using a 0.1 cm pathlength cell equilibrated at 20°C. Spectra were recorded with 0.5 nm resolution at a scan speed of 100nm/min and resulted from averaging 3 scans. The final spectra were baseline-corrected by substracting the corresponding MilliQ spectra obtained under identical conditions. Results were fitted with the secondary structure estimation program J-700.

The results indicate a considerable amount of secondary structure of the derivative. The spectrum of Phl p 12 is characterized with a minimum at 218nm and a strong maximum below 200nm, whereas the minimum of the derivative is shifted to a smaller wavelength and the zero-crossing of the curve is below 200nm (Fig. 2). These findings are indicative for an increasing portion of random-coil secondary structure within the derivative.

### c) Hypoallergenic Phl p 12 derivative lacks affinity for polyproline

Affinity to polyproline is a feature common to profilins from various organisms. It was demonstrated that the hypoallergenic Phl p 12 derivative, MP12, does not bind polyproline and thus exhibits altered biochemical properties.

Approximately 5 µg of purified recombinant MP12 in PBS was subjected to a polyproline-loaded CnBr-activated agarose column (Amersham Bioscience, Uppsala, Sweden) equilibrated with PBS. After collecting the flow-through, the column was washed with 3 volumes (PBS) and elution was performed with 5x 1ml PBS containg 2M or 6M Urea, respectively. Ten µl aliquots of the flow-through, the wash fractions and elution fractions were subjected to a 14% sodium dodecyl sulfate-polyacrylamide gel electrophoresis (SDS PAGE) and proteins were visualized by Commassie staining (Fig. 3). The results indicate a loss of the polyproline binding site due to reorganisation of the primary structure of Phl p 12.

### Example 2: Reduction of IgE binding capacity of MP12

### a) MP12 shows strongly reduced IgE binding capacity

The IgE binding capacity of recombinant MP12 was compared to that of recombinant Phl p 12 wild-type by dot blot analysis using sera from 24 profilin sensitised patients (Fig. 4). Phl p 12 and MP12 as well as human serum albumin (HSA) for control purposes, were dotted onto nitrocellulose and probed with sera from 24 profilin-sensitised patients. Bound IgE antibodies were detected using ¹²⁵I-labeled anti-human-IgE antibodies. All patients showed IgE reactivity with Phl p 12 wild-type, whereas none of the 24 patients reacted with MP12 or with the control protein HSA (Fig. 4).

To quantify the reduction of IgE binding capacity of MP12, fluid phase inhibitions were performed. For this purpose serum from six profilin-sensitised patients were preincubated with either 10µg of Phl p 12 and of MP12 and subconsequently incubated with ELISA plate-bound Phl p 12 (5µg/ml). Bound IgE antibodies were detected with an alkaline phosphatase-labeled anti-human IgE antibody (Pharmingen). Inhibition of IgE binding was calculated with the following formula: Inhibition%= 100x[(A-B)/A]; A representing OD values obtained after incubation of serum with BSA, B representing OD values after incubation of serum with Phl p 12 or MP12, respectively.

The ability of MP12 to inhibit binding of IgE to Phl p 12 is shown as percentage inhibition in Table 2, ranging from 20-40% with mean inhibition of 31.2% for MP12, whereas inhibition achieved with Phl p 12 ranged from 76-91% (mean 86%).

**Table 2: Inhibition of antibody binding to immobilised Phl 12 using Phl p 12 and MP12. IgE antibody binding was inhibited by preincubation of sera from 6 profilin-sensitised patients with Phl p 12 wild-type or MP12. Mean inhibition of antibody binding was calculated and is displayed.**

| **Protein name** | **Amino acid sequence** | **number of amino acids** | **calculated Pi** | **MW (kDa)** | **structural integrity** |
|---|---|---|---|---|---|
| Phl p 12 | MSWQTYVDEHLMCEIEGHHLASAAILGHDGTVWAQS ADFPQFKPEEITGIMKDFDEPGHLAPTGMFVAGAKYM VIQGEPGAVIRGKKGAGGITIKKTGQALVVGIYDEPM TPGQCNMVVERLGDYLVEQGM | 131 | 4.92 | 14.1 | + |
| MP 12 | MEPGAVIRGKKGAGGITIKKTGQALVGIYDEPMTPGQ CNMVVERLGDYLVEQGMMSWQTYVDEHLMCEIEGH HLASAAILGHDGTVWAQSADFPQFKPEEITGIMKDFD EPGHLAPTGMFVAGAKYMVIQGHHHHHH | 137 | 5.68 | 15 | + |

### b) MP12 exhibits reduced allergenic activity

Next, the reshuffled Phl p 12 was compared with Phl p 12 wild-type for its capacity to induce histamine release from basophils from profilin allergic patients.

Granulocytes were isolated from heparinised blood samples of timothy grass pollen allergic patients by Dextran sedimentation. After isolation, cells were incubated with various concentrations of Phl p 12, MP12 or, for control purposes, with a monoclonal anti-human IgE antibody (Immunotech, Marseille, France). Histamine released into the supernatant was measured by radioimmunoassay (Immunotech). Total histamine was determined after freeze thawing of cells. Results are expressed as mean values of duplicate determinations, and represent the percentage of total histamine.

As exemplified in Fig. 5, Phl p 12 induced strong and dose-dependent histamine release in basophils from both patients, yielding maximal histamine release at concentrations between 10⁻⁵-10⁻⁴ µg/ml, whereas no histamine release was observed with MP12 at concentrations up to 10⁻² µg/ml indicating more than 1000-fold reduction of allergenic activity. Moreover, the maximum histamine release from basophils after adding MP12 was considerable lower than that achieved with Phl p 12 wild-type.

### Example 3: Immunization with MP12 induces IgG antibodies that recognize Phl p 12 wild-type as well as profilins from other pollens.

In order to test, whether immunisation with reshuffled Phl p 12 will induce IgG antibodies that react with Phl p 12 wild-type and profilins from other pollens, rabbits were immunized three times with Phl p 12 or MP12 using Freund's complete and incomplete adjuvants (200µg/injection) (Charles River, Kisslegg, Germany). Serum samples were obtained in four weeks intervals. Sera were stored at -20°C until analysis.

Reactivity of MP12 and Phl p 12-induced IgG antibodies was studied by ELISA (Fig. 6). Phl p 12 as well as profilins from birch (Bet v 2) and mugwort were coated onto ELISA plates (5 µg/ml) and incubated with serial dilutions of rabbit antisera (1:2000-1:64000). Bound rabbit antibodies were detected with a 1:1000 diluted peroxidase-labeled donkey anti-rabbit antiserum (Amersham Pharmacia Biotech).

MP12 induced an IgG anti-Phl p 12 antibody response, that was comparable to that induced with Phl p 12 wild-type (Fig. 6A). Moreover, both, Phl p 12- and MP12-induced IgG antibodies, cross-reacted with profilins from birch and mugwort (Fig. 6B, C).

### Example 4: Anti-MP12 antibodies inhibit the binding of serum IgE from grass pollen allergic patients to complete Phl p 12

The ability of MP12-induced rabbit IgG to inhibit the binding of allergic patients' IgE to Phl p 12 was investigated by ELISA competition assay. ELISA plates (Nunc Maxisorp, Rosklide, Denmark) were coated with Phl p 12 (1µg/ml) and preincubated either with a 1:250 dilution of each of the anti-MP12 antiserum or the Phl p 12-antiserum and, for control purposes, with the corresponding preimmune sera. After washing, plates were incubated with 1:3 diluted sera from seven Phl p 12-sensitised grass pollen allergic patients and bound IgE antibodies were detected with a monoclonal rat anti-human IgE antibody (Pharmingen, San Diego, CA), diluted 1:1000, followed by a 1:2000 diluted HRP-coupled sheep anti-rat Ig antiserum (Amersham). The percentage inhibition of IgE binding achieved by preincubation with the anti-peptide or anti-mutant antisera was calculated as follows: % inhibition of IgE binding= 100-OD_{I}/OD_{P}X100. OD_{I} and OD_{P} represent the extinctions after preincubation with the rabbits' immune sera and the corresponding preimmune sera, respectively. As shown in Table 3, inhibition of patients' IgE binding to Phl p 12 achieved with anti-Phl p 12 antibodies was between 30.2-66.7% (49.8% mean inhibition). Likewise, considerable reduction of anti-Phl p 12 IgE reactivity was observed, ranging from 10.8-27.6% (20.8% mean inhibition) with antibodies raised against MP12 (Table 3).

**Table 3: Inhibition of allergic patients' IgE binding to rPhl p 12 by rabbit antibodies. The percentage inhibition of IgE binding to rPhl p 12 achieved by preincubation with rabbit antisera (rabbit anti-Phl p 12, anti-MP12) for seven Phl p 12-allergic patients and the calculated mean inhibition are displayed.**

| Patient | % inhibition | |
|---|---|---|
| | anti-Phl p 12 | anti-MP12 |
| 1 | 66.7 | 27.6 |
| 2 | 53.8 | 18.8 |
| 3 | 46.0 | 16.2 |
| 4 | 43.2 | 18.9 |
| 5 | 45.7 | 27.0 |
| 6 | 30.2 | 10.8 |
| 7 | 63.0 | 26.1 |
| mean | 49.8 | 20.8 |

### Example 5: Anti-MP12 antiserum inhibits basophil degranulation The biological relevance and possible protective activity of peptide-induced IgG antibodies was investigated in a defined cellular model system using rat basophil leukaemia (RBL) cells which were loaded with allergen-specific IgE.

RBL-2H3 cells were plated in 96 well tissue culture plates (4x10⁴ cells/well), incubated for 24 h at 37°C using 7% CO₂. Passive sensitisation was performed with mouse sera containing profilin-reactive IgE at a final dilution of 1:30 for 2 h. Unbound antibodies were removed by washing the cell layer 2 times in Tyrode 's buffer (137mM NaCl, 2.7mM KCl, 0.5mM MgCl₂, 1.8mM CaCl₂, 0.4mM NaH₂PO₄, 5.6mM D-glucose, 12mM NaHCO₃, 10mM HEPES and 0.1% w/v BSA, pH 7.2). RBL cells, preloaded with Phl p 12-specific mouse IgE were exposed to rPhl p 12 (0.005 µg/ml). Phl p 12 was preincubated in Tyrode's buffer with 0, 2, 5, 7.5 or 10% v/v of rabbit antiserum from a Phl p 12-immunized rabbit, a MP12-immunized rabbit or the corresponding preimmune sera for 2h at 37°C.

Preincubated Phl p 12 was added to the RBL cells for 30 min in a humidified atmosphere at 37°C and their supernatants were analyzed for β-hexosaminidase activity by incubation with 80 µM 4-methylumbelliferyl-N-acetyl-β-D-glucosamide (Sigma-Aldrich, Vienna, Austria) in citrate buffer (0.1M, pH 4.5) for 1 h at 37°C. The reaction was stopped by addition of 100 µl glycine buffer (0.2M glycine, 0.2M NaCl, pH 10.7) and the fluorescence was measured at λₑₓ: 360/λₑₘ: 465 nm using a fluorescence microplate reader (Spectrafluor, Tecan, Austria). Results are reported as fluorescence units and percentage of total β-hexosaminidase released after lysis of cells with 1% Triton X-100.

As exemplified in Fig. 7, both, preincubation of Phl p 12 with increasing concentrations (2-10% v/v) of rabbit anti-MP12 anti-bodies and with rabbit anti-Phl p 12 antibodies led to a dose-dependent inhibition of rPhl p 12-induced mediator release from RBLs that had been preloaded with Phl p 12-specific mouse IgE. No inhibition of basophil degranulation was observed when the allergen was preincubated with the same concentrations of preimmune Ig.

### Example 6: Expression, purification and characterization of a hypoallergenic derivative from Dermatophagoides pteronyssinus allergen Der p 2 (Der p 2 Hybrid)

House dust mite (HDM) allergy belongs to the most common allergies worldwide which affects more than 50% of all allergic patients. *Dermatophogoides pteronyssinus* was identified as the most important source of allergens in house dust in Europe.

Twenty groups of mite allergens have been characterized so far, and group 2 allergens were identified as the major mite allergens, against which more than 80% of mite allergic patients are sensitized and they are mainly localized in mite faeces. Group 2 allergens were first characterized as 14000-18000 Da allergens with a high IgE-binding activity. Isolation and analysis of cDNA clones coding for Der p 2, revealed then that Der p 2 comprises an allergen with 129 amino acid residues, a calculated molecular weight of 14000 Da and without N-glycosylation sites. Group 2 allergens contain three disulfide bonds and are composed of two anti-parallel β-sheets. T-cell epitopes of Der p 2 are located in all regions of the protein and IgE-epitopes were shown to be conformational.

Immunotherapy studies with crude mite extracts have demonstrated that dangerous systemic side effects may occur during immunotherapy with HDM-extracts (Akcakaya, N., et al. (2000) Ann Allergy Asthma Immunol 85:317) as well as the induction of new IgE reactivities to sea-foods (van Ree, R., et al. (1996) Allergy 51:108).

To overcome the disadvantages of extract-based immunotherapy, several strategies have been applied to develop hypoallergenic allergen derivatives. In case of Der p 2, variants were developed with reduced IgE reactivity by destroying disulfide bonds by site-directed mutagenesis, by destroying the disulfide bonds through N- and C-terminal deletion, or by introducing mutations. However, their biological activity is questionable.

In the following examples two recombinant fragments of the group 2 allergen of *Dermatophagoides pteronyssinusm* (Der p 2) comprising aa 1-53 and aa 54-129, to destroy conformational B-cell epitopes and to retain the major T-cell epitopes, were produced. Additionally, a recombinant Der p 2 hybrid molecule (aa 54-129 + 1-53), in which the two rDer p 2 fragments were recombined in inverse order by PCR-based gene-SOEing, was constructed.

Two recombinant fragments of Der p 2 comprising amino acids (aa) 1-53 and aa 54-129 were constructed by PCR-amplification as outlined in example 1 (see Fig. 8). A Der p 2 Hybrid molecule was generated in inverse order (aa 54-129 + 1-53) by PCR-based gene-SOEing (Linhart et al., FASEB J.16 (2002), 1301-1303).

### a) Expression in E. coli and purification of Der p 2, Der p 2 fragments and Der p 2 hybrid

cDNAs coding for His-tagged Der p 2, Der p 2 fragments (aa 1-53 and aa 54-129) and Der p 2 hybrid (aa 54-129+1-53) were generated by PCR amplification using primers (MWG, Ebersberg, Germany) as indicated in Table 4 and a Der p 2 cDNA was obtained by reverse transcription from Der p RNA.

**Table 4:**

| Primer | Sequence |
|---|---|
| 1 (F) | 5'-GGAATTCCATATGGATCAAGTCGATGTC-3' |
| 2 (R) | 5'-GGAATTCCTTA***GTGATGGTGATGGTGATG***TTCAATTTTAGCGGT-3' |
| 3 (F) | 5'-GGAATTCCATATGATCAAAGCCTCAAT-3' |
| 4 (R) | 5'-GGAATTCCTTA***GTGATGGTGATGGTGATG***ATCGCGGATTTTA-3' |
| 5 (overlapping) | 5'-CTTTGACATCGACTTGATCATCGCGGATTTTAGCAT-3' |
| 6 (overlapping) | 5'-CATGCTAAAATCCGCGATGATCAAGTCGATGTCAAA-3' |

Forward (F), reverse (R) and overlapping primers are indicated. The *EcoRI* sites and *NdeI* sites are underlined. Nucleotides coding for the His-tags are shown in bold/italic letters.

Primers 1 and 4 were used for the amplification of the rDer p 2 cDNA, primers 1 and 2 for the cDNA coding for rDer p 2 fragment 1 (aa 1-53) and primers 3 and 4 for the cDNA of the rDer p 2 fragment 2 (aa 54-129). rDer p 2 hybrid was generated by PCR-based gene-SOEing using primers 2 and 3 and the two overlapping primers 5 and 6. Upstream primers contained an NdeI and EcoRI site and downstream primers contained an EcoRI site as well as six His codons. PCR products were cut with NdeI/EcoRI, gel-purified and subcloned into the NdeI/EcoRI sites of plasmid pET17b. Calcium chloride method was used for the transformation of the plasmids into E.coli strain XL-1 Blue. Plasmid DNA was isolated by NuceloBond AX kit - maxi-prep (Macherey-Nagel, Germany) and the sequence of the cDNA inserts was confirmed by sequencing of both DNA strands on an automated sequencing system (MWG, Germany).

Recombinant proteins containing C-terminal Hexahistidine-tails were expressed in E.coli strain BL21 (DE3) in liquid culture by induction with 0.5mM isopropyl-β-thiogalactopyranoside (IPTG) at an OD600 of 1 for 5h at 37°C. Cells were harvested by centrifugation at 4,000 x g for 15 minutes at 4°C.

The bacterial pellets obtained from 11 liquid culture were resuspended in 10ml 25mM imidazol, pH 7.4, 0.1% v/v Triton X-100 and treated with 100µg lysozyme for 30 minutes at room temperature. Cells were lysed by 3 freeze/thawing cycles (-70°C/+50°C), DNA was degraded by incubation with 1 µg DNase I for 10 minutes at room temperature and cell debris were removed by centrifugation at 10,000 x g for 30 minutes at 4°C. rDer p 2 fragment 1 was found in the soluble fraction and purified under native conditions over Ni-NTA resin affinity columns (QIAGEN, Germany).

rDer p 2, rDer p 2 fragment 2 and rDer p 2 hybrid were found in the pellet in the inclusion body fraction, which was solubilized with 8M urea, 100mM NaH₂PO₄, 10mM Tris-Cl, pH 8 for 60 minutes at room temperature. Insoluble residues were removed by centrifugation (10,000 x g, 15min, 4°C) and rDer p 2, rDer p 2 fragment 2 and rDer p 2 hybrid were purified under denaturating conditions over Ni-NTA resin affinity columns (QIAGEN).

Fractions, containing recombinant proteins of more than 90% purity were dialysed against 50mM NaH₂PO₄ pH 7 and the final protein concentrations were determined by Micro BCA Protein Assay Kit (Pierce, USA).

The construction of a hybrid molecule as outlined above disrupted at least one of the two β-sheets of Der p 2 and the disulfide bond between C8 and C119 and thus the conformational IgE epitopes of Der p 2 destroyed and major T-cell epitopes preserved. The rDer p 2 derivatives were overexpressed as visible bands in E. coli yielded a distinct accumulation (Fig. 9, lanes 1). rDer p 2 fragment 1 was found in the soluble fraction, whereas the other proteins accumulated in the insoluble inclusion body fractions but could be solubilized in urea. rDer p 2 and rDer p 2 derivatives were purified by nickel affinity chromatography (Fig. 9, lanes 2) yielding 20 to 30mg protein /1 E. coli culture. After refolding by dialysis, rDer p 2, rDer p 2 fragment 1 and rDer p 2 hybrid remained soluble in physiological buffers at concentrations from 0.5mg/ml to 1mg/ml, whereas rDer p 2 fragment 2 only remained soluble at a concentration below 0.1mg/ml. SDS-PAGE analysis indicated a more than 90% purity of the proteins, which migrated as monomeric form and dimeric forms (Fig. 9, lanes 2).

### b) Matrix-assisted laser desorption and ionization-time of flight (MALDI-TOF) mass spectrometry of rder p 2 and rDer p 2 derivatives

Laser desorption mass spectra were acquired in a linear mode with a time of-flight Compact MALDI II instrument (Kratos, U.K.; piCHEM, Austria). Samples were dissolved in 10% acetonitrile, 0.1% trifluoroacetic acid and Alfa-cyano-4 hydroxy-cinnamic acid (dissolved in 60% acetonitrile, 0.1% trifluoroacetic acid) was used as a matrix. For sample preparation, a 1:1 mixture of protein and matrix solution was deposited onto the target and air-dried.

Analysis of the four proteins by MALDI-TOF mass spectrometry revealed molecular masses of 15072.9 Da, 6806.7 Da, 9216.3 Da and 15001.8 Da for rDer p 2, rDer p 2 fragment 1, rDer p 2 fragment 2 and rDer p 2 hybrid, respectively, which are in agreement with the theoretical masses of the proteins calculated from their amino acid sequences (Fig. 10).

### c) Circular dichroism (CD) analysis

The CD spectra of the purified recombinant proteins were recorded on a JASCO J715 spectropolarimeter that had been wavelength calibrated with neodymium glass in accordance with the manufacturer's suggestions. CD measurements were performed with rDer p 2 and rDer p 2 derivatives (c = 0.1 to 0.5mg/ml) dissolved in double distilled water at room temperature. A circular quartz cuvette with a path length of 0.1cm was used and the spectra were recorded with 0.2nm resolution at a scan speed of 50nm/min. The spectra were signal-averaged by accumulating at least three scans and the results are expressed as the mean residue ellipticity at a given wavelength.

The far ultraviolet CD spectrum of the purified recombinant Der p 2 shows a negative band at 217nm, indicating a β-sheet conformation (Fig. 11). In contrast, the CD spectra of the rDer p 2 derivatives indicate that these proteins are mainly unfolded. rDer p 2 fragment 1 shows a typical random coil conformation, identified by a negative band at ∼200nm. Also rDer p 2 fragment 2 shows a predominant random coil conformation, although the intensity of the signal was very low. rDer p 2 hybrid spectrum adsorbed mainly random coil conformation with small amounts of β-sheet structures (Fig. 11). The destruction of the three-dimensional conformation could be confirmed by circular dicroism analysis, showing a loss or reduction of β-sheet structure in the rDer p 2 derivatives compared to rDer p 2 wild-type.

### Example 7: Recombinant Der p 2 Hybrid (rDer p 2 Hybrid) shows strongly reduced IgE binding capacity

Purified recombinant Der p 2, the two rDer p 2 fragments, fragment 1 (aa 1-53) and fragment 2 (aa 54-129), and rDer p 2 hybrid were tested for IgE reactivity by non-denaturating dot blot assays. Two microlitres of the purified proteins (0.1mg/ml) and, for control purposes, BSA were dotted onto nitrocellulose membrane strips (Schleicher & Schuell, Germany). Nitrocellulose strips containing the dot-blotted proteins were blocked in buffer A (40mM Na₂HPO₄, 0.6mM NaH₂PO₄, pH 7.5, 0.5% [v/v] Tween 20, 0.5% [w/v] BSA, 0.05% [w/v] NaN₃) and incubated with sera from mite-allergic patients, serum from a non-allergic person (dilutions 1:10) or buffer A without serum. Bound IgE antibodies were detected with 125I-labeled anti-human IgE antibodies and visualized by autoradiography.

The IgE-binding capacity of rDer p 2 wild-type allergen was compared with the two rDer p 2 fragments and rDer p 2 hybrid by non-denaturing dot blot assays. Sera from 17 mite allergic individuals (lanes 1-17) showed varying IgE reactivity to nitrocellulose dotted rDer p 2, whereas almost no IgE reactivity to rDer p 2 fragment 1 could be detected. Only 3 sera showed very weak binding to rDer p 2 fragment 2 and 2 sera reacted with rDer p 2 hybrid (Fig. 12). Serum from a non-allergic person as well as buffer without serum showed no IgE reactivity to rDer p 2 or to rDer p 2 derivatives (Fig. 12 lanes 18, 19). No IgE reactivity to the control protein, BSA, was found (Fig. 12). As a consequence of the loss of the conformation and thus the conformational IgE-epitopes (see example 7), it could be shown that the rDer p 2 derivatives have almost completely lost their IgE-binding capacity compared to rDer p 2 wild-type.

### Example 8: Reduced allergenic activity of rDer p 2 derivatives as determined by CD 203c expression

Heparinized blood samples were obtained from allergic patients. Blood samples (100µl) were incubated with various concentrations of rDer p 2, rDer p 2 fragments, rDer p 2 hybrid, a monoclonal anti-IgE antibody (Immunotech, Marseille, France), or PBS for 15 minutes (37°C). CD 203c expression was determined as described (Hauswirth, A. W., et al. (2002) J Allergy Clin Immunol 110:102.).

The upregulation of CD 203c has been described as a surrogate marker for allergen-induced basophil activation and degranulation (Hauswirth, A. W., et al. (2002)). Therefore the allergenic activity of recombinant Der p 2, rDer p 2 fragments and rDer p 2 hybrid by measuring CD 203c upregulation on basophils from house dust mite allergic patients was compared (Fig. 13, 14). Fig. 13 shows representative results from 3 patients. Incubation of basophils with 10µg/ml of rDer p 2 wild-type significantly up-regulated CD 203c expression in each of the tested patients, whereas no upregulation was obtained with the same concentration of the individual fragments or with an equimolar mixture of the two fragments (Fig. 13). Additionally, basophils from the same 10 patients were exposed to different concentrations (5µg/ml - 0.32ng/ml) of rDer p 2 and rDer p 2 hybrid in 1:5 dilution steps. Fig. 14 shows the results from 6 representative patients. Exposure of basophils with rDer p 2 hybrid resulted in an upregulation of CD 203c expression at concentrations between 40ng/ml and 5000ng/ml, whereas rDer p 2 wild-type induced upregulation of CD 203c already at concentrations between 8 - 200ng/ml. In 8 out of 10 patients, rDer p 2 hybrid had a more than 10-fold reduced capacity to activate basophils compared to rDer p 2.

Anti-human IgE antibodies induced upregulation of CD 203c expression on basophils from all patients, whereas no upregulation was obtained with buffer alone (Fig. 13 + 14).

Determination of CD 203c expression on basophils from mite-allergic patients indicates a reduced biological activity of rDer p 2 hybrid compared to rDer p 2 wild-type and no biological activity can be observed with the rDer p 2 fragments. Moreover, basophil activation assays using RBL cells indicate that IgE Abs induced with the derivatives were less anaphylactic. These results indicate that hypoallergenic rDer p 2 derivatives will induce less IgE-mediated side-effects than the Der p 2 wild-type allergen when used for immunotherapy.

### Example 9: rDer p 2 derivatives induce rDer p 2-specific IgG antibodies in mice similar as rDer p 2 wild-type allergen

Groups of five eight-week-old female BALB/c mice each were immunized with 5µg of purified proteins (rDer p 2, rDer p 2 fragment 1, rDer p 2 fragment 2 or rDer p 2 hybrid), adsorbed to 200µl of AluGel-S (SERVA Electrophoresis, Germany) subcutaneously in the neck in 4 weeks intervals over a period of 20 weeks. Blood samples were collected one day before each immunization and stored at -20°C.

ELISA plates (Greiner, Austria) were coated with rDer p 2 diluted in PBS (c = 5µg/ml) over night at 4°C. The plates were washed twice with PBST (PBS; 0.05% v/v Tween 20) and blocked with blocking buffer (PBST; 1% w/v BSA) for 3h at room temperature. Mouse sera were diluted 1:1000 for measurement of Der p 2-specific IgG1 in PBST; 0.5% w/v BSA and 100µl of this dilution was added per well overnight at 4°C.

Plates were washed 5 times with PBST and bound IgG1 antibodies were detected with a monoclonal rat anti-mouse IgG1 antibody (BD Pharmingen, USA), followed by the addition of horseradish peroxidase-labeled goat anti-rat IgG antibodies (Amersham Bioscience, Sweden) as described (Vrtala, S., et al. (1996) J Allergy Clin Immunol 98:913).

The Der p 2 specific IgG₁ levels were determined in serum samples obtained from mice after immunization with rDer p 2 and rDer p 2 derivatives (Fig. 15). rDer p 2 as well as the rDer p 2 derivatives were immunogenic and induced IgG₁ responses in the mice after the second immunization (week 8) (Fig. 15). After the second immunization the IgG₁ responses induced with rDer p 2 fragment 1 and rDer p 2 hybrid were even higher than that induced with rDer p 2 (Fig. 15). After the last immunization, IgG₁ responses induced with the rDer p 2 derivatives were comparable to those induced with the rDer p 2 wild-type molecule (Fig. 15).

### Example 10: IgG1 antibodies induced by immunization with rDer p 2 derivatives inhibit mite-allergic patients' IgE binding to rDer p 2 wild-type

ELISA plates (Greiner, Austria) were coated with 100µl purified rDer p 2, diluted with PBS to a concentration of 5µg/ml, over night at 4°C. After washing twice with PBST and blocking with blocking buffer (PBST; 1% w/v BSA) for 3h at room temperature, plates were incubated overnight at 4°C with anti-rDer p 2, anti-rDer p 2 fragment 1, anti-rDer p 2 fragment 2 or anti-rDer p 2 hybrid antisera or the corresponding preimmune sera. Mouse anti-sera were diluted 1:20 and rabbit antisera were diluted 1:100 in PBST; 0.5% w/v BSA. After washing, the plates were incubated with 1:10 diluted sera from mite allergic patients overnight at 4°C and bound human IgE antibodies were detected with HRP-coupled goat anti-human IgE antibodies (KPL, USA) diluted 1:2500 in PBST; 0.5% w/v BSA as described (44, 45). The percentage of inhibition of IgE binding was calculated as follows: 100 - (ODs/ODp) x 100, where ODs and ODp represent the extinction coefficients after preincubation with the immune serum and the preimmune serum, respectively.

Mouse IgG1 antibodies induced by immunization with rDer p 2 and the rDer p 2 derivatives were investigated for their ability to inhibit mite-allergic patients' IgE binding to rDer p 2 in ELISA competition experiments.

The percentage of inhibition of allergic patients' IgE binding to rDer p 2 wild-type by mouse IgG antibodies is shown in Tables 5 and 6.

The inhibition obtained with mouse anti-rDer p 2 antibodies was between 61 and 87% (mean 75%), whereas mouse anti-rDer p 2 hybrid antibodies, anti-Der p 2 fragment 1 antibodies and anti-Der p 2 fragment 2 antibodies inhibited serum IgE binding to rDer p 2 wild-type between 47 and 76% (mean 62%), between 48 and 66% (mean 54%) and between 24 and 52% (mean 41%), respectively (Table 5).

**Table 5:**

| % Inhibition of IgE binding | | | | | |
|---|---|---|---|---|---|
| **Antibodies** | **Patient 1** | **Patient 2** | **Patient 3** | **Patient 4** | **mean** |
| **rDer p 2 fragment 1** | 48 | 66 | 53 | 50 | 54 |
| **rDer p 2 fragment 2** | 39 | 50 | 52 | 24 | 41 |
| **rDer p 2 hybrid** | 59 | 76 | 64 | 47 | 62 |
| **rDer p 2** | 61 | 87 | 77 | 73 | 75 |

In additional experiments, rabbits were immunized with purified rDer p 2 and the three rDer p 2 derivatives. The ability of rabbit anti-sera to inhibit mite-allergic patients' IgE binding to rDer p 2 was also tested by ELISA inhibition assays with an outcome similar as obtained for the mouse sera (Table 6). Rabbit anti-rDer p 2 antibodies inhibited patients' IgE binding to rDer p 2 between 47 and 89% (mean 66%), whereas anti-rDer p 2 hybrid antibodies inhibited human IgE binding between 20 and 86% (mean 59%). The inhibition obtained with rabbit anti-rDer p 2 fragment 1 antibodies was between 26 and 70% (mean 52%) and the inhibition with rabbit anti-rDer p 2 fragment 2 antibodies was between 32 and 54% (mean 42%). Using a mixture of the anti-fragment 1 and anti-fragment 2 antibodies the inhibition of patients' IgE binding to rDer p 2 wild-type was only slightly increased to a mean of 55% (Table 6).

**Table 6:**

| %Inhibition of IgE binding | | | | | | |
|---|---|---|---|---|---|---|
| **Antibodies** | **Patient 1** | **Patient 2** | **Patient 3** | **Patient 4** | **Patient 5** | **mean** |
| **rDer p 2 fragment 1** | 59 | 70 | 49 | 26 | 57 | 52 |
| **rDer p 2 fragment 2** | 40 | 49 | 33 | 32 | 54 | 42 |
| **fragment 1 + fragment 2** | 60 | 69 | 44 | 38 | 66 | 55 |
| **rDer p 2 hybrid** | 61 | 86 | 61 | 20 | 67 | 59 |
| **rDer p 2** | 60 | 89 | 53 | 47 | 78 | 66 |

Immunization of mice showed the immunogenicity of all three rDer p 2 derivatives by their capacity to induce IgG antibody responses. IgE-binding from mite-allergic patients to Der p 2 was inhibited by IgG antibodies induced with each of the rDer p 2 derivatives but rDer p 2 hybrid-induced IgG antibodies indicated a better inhibitory capacity compared to IgG antibodies induced with the two individual fragments and even to a mixture of fragment 1 and 2 induced IgG antibodies. These results are of importance, since blocking antibodies were shown to play a main role in SIT with recombinant allergens.

Anti-rDer p 2 and anti-rDerp 2 derivative antibodies induced by immunisation of mice inhibit allergic patients' IgE binding to rDer p 2 as shown in an ELISA inhibition assay.

Der p 2 Hybrid induces blocking antibodies in the present mouse model; immunogenicity is significantly increased by reshuffling the fragments.

### Example 11: Vaccines based on rDer p 2 derivatives have a reduced allergenicity in vivo compared to a rDer p 2-wild-type-based vaccine

Rat basophil leukemia (RBL) cells (subline RBL-2H3) were plated on ELISA plates (Nunc, Denmark) (100µl: 4 x 104 cells) in cell culture medium (100ml RPMI 1649, 10% FCS, 4mM L-Glutamine, 2mM Sodium Pyruvate, 10mM HEPES, 100µM 2-Mercaptoethanol, 1% Pen/Strep) over night at 37°C, 5% CO₂.

Cells were loaded with 2µl of serum obtained from mice immunized with rDer p 2, rDer p 2 fragment 1, rDer p 2 fragment 2 and rDer p 2 hybrid for 2h at 37°C, washed twice with 200µl Tyrode/BSA buffer (137mM NaCl, 2.7mM KCl, 0.5mM MgCl₂, 1.8mM CaCl₂, 0.4mM NaH₂PO₄, 5.6mM D-glucose, 12mM NaHCO₃, 10mM N-2-hydroxyethylpiperazine-N'-2-ethanesulfonic acid (HEPES), 0.1% bovine serum albumin, pH 7.2) (Sigma-Aldrich, Austria) and stimulated with rDer p 2 (c = 0.3µg/ml). Total β-hexosaminidase release was induced by addition of 10µl 10% v/v Triton X-100 (Merck, Germany).

For measuring the release of β-hexosaminidase, 50µl assay solution (80µM 4-methylumbelliferyl-N-acetyl- p - D-glucosaminide in 0.1M citrate buffer, pH 4.5) was incubated with 50µl supernatant for 1h at 37°C, 5%CO₂.

The reaction was stopped by adding 100µl glycin buffer (0.2M glycine, 0.2M NaCl, pH 10.7) and fluorescence was measured at λex: 360nm λem: 465nm using a fluorescence microplate reader (Dynatech MR 7000, Dynatech Laboratories, USA). Results are shown as mean percentages of total β-hexosaminidase release.

To investigate whether vaccination with rDer p 2 derivates induces allergic immune responses to Der p 2 wild-type allergen, mice were immunized with rDer p 2, rDer p 2 fragment 1, rDer p 2 fragment 2 and rDer p 2 hybrid, respectively. Then serum samples from the mice were used to load RBL cells to quantify the allergenic immune response to rDer p 2 wild-type allergen by RBL degranulation experiments. The release obtained with rDer p 2 wild-type allergen in RBLs loaded with mouse anti-rDer p 2 fragment 1, anti-rDer p 2 fragment 2 and anti-rDer p 2 hybrid antibodies was between 0 and 16.6% (mean 6.4%), between 0.2 and 28.6% (mean 13.2%) and between 4.7 and 37.1% (mean 18.3%), whereas RBLs, loaded with anti-rDer p 2 wild-type antibodies released between 35 and 39% (mean 37%) after stimulation with rDer p 2 wild-type (Fig. 16).

### Example 12: MP 12 induced IgG antibodies that recognize Phl p 12 wild-type, profilins from other pollens and plant -food derived profilins.

In order to test whether antibodies induced after immunization with MP 12 recognize profilins from pollens as well as from plant derived food, ELISA experiments were performed.

Profilins from timothy grass pollen (Phl p 12), birch pollen (Bet v 2), mugwort pollen (Art v 4) and from different plant foods (cashew nut (Ana c ), celery (Api g 4), banana (Mus xp 1), hazelnut (Cor a 2), and carrot (Dau c 4) were coated onto ELISA plates (5µg/ml) and incubated with serial dilutions of rabbit antisera (1:2000- 1:64000). Bound rabbit antibodies were detected with a POX-labeled donkey-anti-rabbit antiserum.

MP 12 induced an IgG antibody response that was comparable with that induced with Phl p 12 wild-type (Fig. 17). Both, Phl p 12 and MP12-induced IgG antibodies cross-reacted with profilins from pollens (grass, trees, weeds) and plant-derived food profilins (Fig. 17).

### Example 13: Anti-MP 12 antibodies inhibit the binding of serum IgE from grass pollen allergic patients to complete Phl p 12 as well as to profilins from other pollens (trees and weeds) and to plant food-profilins.

The ability of MP12-induced rabbit IgG to inhibit the binding of allergic patients' IgE to Phl p 12, to profilins from distinct pollens and to plant food-derived profilins was investigated by ELISA competition experiments.

ELISA plates (Nunc Maxisorp, Denmark) were coated with profilins from timothy grass (rPhl p 12), birch pollen (rBet v 1), carrot (rDau c 4), hazelnut (rCor a 2), banana (rMus xp 1) and cashew nut (rAna c 1) and preincubated with a 1:50 dilution of the anti-Phl p 12 antiserum, the anti-MP 12-antiserum and, for control purposes, with the corresponding preimmune sera. After washing, plates were incubated with 1:3 diluted sera from eight profilin-sensitized patients and bound IgE antibodies were detected with a HRP-labeled anti-human IgE antiserum from goat (KPL, USA), diluted 1:2500. The percentage inhibition of IgE binding achieved by preincubation with the anti-Phl p 12 and anti-MP 12-antisera was calculated as follows: % inhibition of IgE binding= 100-ODI/ODPx100. ODI and ODP represent the extinctions after preincubation with the rabbits' immune sera and the corresponding preimmune sera, respectively (Table 7).

**Table 7:**

| Percentage Inhibition of IgE binding to | | | | | | | | | | | | |
|---|---|---|---|---|---|---|---|---|---|---|---|---|
| Patient | Phl p 12 | | Bet v 2 | | Cor a 2 | | Mus xp 1 | | Dau c 4 | | Ana c1 | |
| | α-Phl p 12 | α-MP 12 | α-Phl p 12 | α-MP 12 | α-Phl p 12 | α-MP 12 | α-Phl p 12 | α-MP 12 | α-Phl p 12 | α-MP 12 | α-Phl p 12 | α-MP 12 |
| 1 | 91 | 82,7 | 88,3 | 84,4 | 61,3 | 58,7 | 70,6 | 46,4 | 82,7 | 83,3 | 70 | 71,7 |
| 2 | 82,5 | 72,8 | 74,9 | 77,6 | 73,3 | 60,3 | 74,4 | 50,8 | 75,5 | 83,2 | 62,3 | 45,1 |
| 3 | 89,4 | 72,3 | 75,4 | 76,4 | 58,8 | 61,6 | 74,4 | 33,5 | 65,4 | 63,1 | 61,6 | 56 |
| 4 | 77,4 | 72 | 69 | 69,6 | 56,00 | 52 | 57,2 | 39,8 | 65 | 66,8 | 71,5 | 41,5 |
| 5 | 86,3 | 65,2 | 35 | 66,4 | 68,6 | 52 | 75,5 | 27,2 | 97,5 | 93,8 | 59,5 | 56,2 |
| 6 | 71,2 | 84,7 | 54,4 | 72 | 57 | 62,7 | 73,9 | 25,6 | 58,5 | 68,3 | 44,3 | 42,6 |
| 7 | 83,6 | 70,4 | 60,2 | 70,8 | 69,7 | 59,7 | 72,5 | 35,6 | 72,4 | 71,3 | 28,1 | 32,2 |
| 8 | 89,1 | 57,4 | 61 | 79 | 57,8 | 57,8 | 73 | 29,6 | 70 | 67 | | |
| mean | 83,8 | 72,3 | 64,8 | 74,5 | 62,3 | 58,1 | 71,4 | 36,1 | 73,3 | 74,6 | 56,8 | 53,6 |

The mean inhibition of IgE binding to timothy grass pollen profilin achieved with Phl p 12-induced antibodies and MP 12-induced antibodies was comparable with 83.8% and 72.3%, respectively (Table 7). IgE binding to birch pollen profilin, Bet v 2, was even stronger inhibited with MP 12-specific antibodies (mean inhibition 74.5%) than with Phl p 12-induced antibodies (mean inhibition 64.8%). IgE binding to plant food profilins were inhibited with both antisera to a very similar degree (Cor a 2: 62.3% average inhibition with anti-Phl p 12-IgG, 58.1% with anti-MP 12-IgG; Dau c 4: 73,3% average inhibition with anti-Phl p 12-IgG, 74.6% with anti-MP 12-IgG; Ana c 1: 56.8% average inhibition with anti-Phl p 12-IgG, 53.6% with anti-MP 12-IgG). Only IgE binding to banana profilin, Mus xp 1, was less inhibited with anti-Mp 12- IgG (36.1%) than with anti-Phl p 12-induced IgG (71.4%) (Table 7).

Profilin represents an allergen that is expressed in all eukaryotic cells and thus represents a pan-allergen that might induce inhalative allergies (e.g., rhinoconjunctivits, asthma) as well as oral allergy syndromes after oral ingestion (itching and swelling of lips and the tounge) in sensitized patients.

The reshuffled Phl p 12-derivative, MP12, induces IgG antibodies after immunization that recognize profilins from both pollens as well as from plant-derived food. MP 12-induced antibodies inhibit patients' serum IgE binding to profilins from pollens and also to plant food-derived profilins. Thus, the MP12 is suitable for the treatment of pollen- food cross-sensitization attributable to profilin allergy.

## Claims

1. Method for producing derivatives of wild-type protein allergens with reduced allergenic activity, **characterized by** the following steps:
- providing a wild-type protein allergen with an allergenic activity,
- splicing said wild-type protein allergen into two parts, said two parts having a reduced allergenic activity or lacking allergenic activity and
- rejoining said two fragments in inverse orientation.

2. Method according to claim 1, **characterized in that** said derivative is produced in a host as a recombinant protein, especially with a host with high expression capacity.

3. Method according to claim 1 or 2, **characterized in that** said wild-type allergen is selected from the group of profilins, especially Phl p 12, birch allergens, especially Bet v 4, dust mite allergens, especially Der p2, storage mite allergens, especially Lep d 2, timothy grass allergens, especially Phl p 7.

4. Method according to any one of claims 1 to 3, **characterized in that** reduction in allergenic activity is measured by a reduction of inhibition of IgE binding capacity of at least 10 %, preferably at least 20 %, especially at least 30 %, compared to the wild-type allergen.

5. Method according to any one of claims 1 to 4, **characterized in that** reduction in allergenic activity is measured by lack of binding of IgE antibodies of allergen sensitised patient's sera to a dot blot of said derivative.

6. : Method according to any one of claims 1 to 5, **characterized in that** said derivatives are combined with a pharmaceutically acceptable excipient and finished to a pharmaceutical preparation.

7. Method according to any one of claims 1 to 6, **characterized in that** said derivatives are combined with a suitable vaccine adjuvant and finished to a pharmaceutically acceptable vaccine preparation.

8. Method according to claim 7, **characterized in that** said derivatives are combined with further allergens to a combination vaccine.

9. Method according to claim 8, **characterized in that** said allergen is a wild-type allergen, especially a mixture of wild-type allergens, recombinant wild-type allergens, derivatives of wild-type protein allergens or mixtures thereof.

10. Method according to any one of claims 6 to 9, **characterized in that** said preparation further contains an allergen extract.

11. Allergen derivative of a wild-type protein allergen, said wild-type protein allergen having an amino acid sequence of 1 to Z, **characterized in that** said derivative adjacently contains - in N-terminus to C-terminus orientation - the two wild-type allergen fragments X to Z and 1 to X, said two wild-type allergen fragments having reduced allergenic activity or lacking allergenic activity.

12. Allergen derivative according to claim 11, **characterized in that** X to Z and 1 to X are at least 30 amino acid residues long, preferably at least 50 amino acid residues, especially at least 60 amino acid residues.

13. Allergen derivative according to claim 11 or 12, **characterized in that** X to Z and 1 to X differ in length by 50 % or less, preferably by 30 % or less, especially by 20 % or less.

14. Allergen derivative according to any one of claims 11 to 13, **characterized in that** said wild-type allergen is selected from a type I allergen, preferably from table A, more preferred an allergen of timothy grass (Phelum pratense) pollen, especially Phl p 12, birch (Betula verrucosa) pollen, especially Bet v 4, yellow jacket (Vespula vulgaris) venom, paper wasp (Polistes annularis) venom, Parietaria judaica pollen, ryegrass pollen, dust mite allergens, especially Der p 2, or mixtures thereof.

15. Allergen composition comprising an allergen derivative according to any one of claims 11 to 14 and further allergens, preferably wild-type allergens, especially a mixture of wild-type allergens, recombinant wild-type allergens, derivatives of wild-type protein allergens or mixtures thereof.

16. Allergen composition according to claim 15, **characterized in that** said composition further contains an allergen extract.

17. Allergen composition according to claims 15 to 16, **characterized in that** it contains a pharmaceutically acceptable excipient.

18. Use of an allergen derivative or an allergen composition according to any one of claims 11 to 17 for the preparation of an allergen specific immunotherapy medicament.

19. Use of an allergen derivative or an allergen composition according to any one of claims 11 to 17 for the preparation of a medicament for the passive immunisation.

20. Use of an allergen derivative or an allergen composition according to any one of claims 11 to 17 for the preparation of a medicament for the prophylactic immunization.

21. Use according to any one of claims 18 to 20, **characterized in that** said medicament further contains adjuvants, diluents, preservatives or mixtures thereof.

22. Use according to any one of claims 18 to 21, **characterized in that** it comprises 10 ng to 1 g, preferably 100 ng to 10 mg, especially 0,5 µg to 200 µg of said recombinant allergen derivative.

23. Method for producing an allergen derivative according to any one of claims 11 to 17, c**haracterized** by the following steps:
- providing a DNA molecule encoding an allergen derivative according to any one of claims 11 to 17,
- transforming a host cell with said DNA molecule and
- expressing said derivative in said host cell and isolating said derivative.

24. Method according to claim 23, **characterized in that** said host is a host with high expression capacity.

25. Method for producing an allergen derivative according to any one of claims 11 to 17, **characterized in that** it is produced by chemical synthesis.

26. Use of a profilin derivative obtainable from a first wild-type profilin molecule by a method according to any one of claims 1 to 10 or 23 to 25 or an allergen derivative of a first wild-type profilin molecule according to claims 11 to 14 for the manufacture of a medicament for the prevention or the treatment of allergic diseases caused by a second wild-type profilin molecule.

27. Use according to claim 26, **characterized in that** said first and said second profilin molecules are selected from the group consisting of Phl p 12, Bet v 2, Art v 4, Ana c, Api g 4, Mus xp 1, Cor a 2, and Dau c 4.

28. Use according to claims 26, **characterized in that** said first profilin molecule is Phl p 12 and said second profilin molecule is selected from the group consisting of Bet v 2, Art v 4, Ana c, Api g 4, Mus xp 1, Cor a 2, and Dau c 4.

29. Use according to claim 27 or 28 for the manufacture of a medicament for the treatment and/or prevention of pollen-food cross sensitization attributable to profilin allergy.

## Patentansprüche

1. Verfahren zur Herstellung von Derivaten von Wildtyp-Protein-Allergenen mit verringerter allergener Aktivität, **gekennzeichnet durch** die folgenden Schritte:
- Vorsehen eines Wildtyp-Protein-Allergens mit einer allergenen Aktivität,
- Spleißen des Wildtyp-Protein-Allergens in zwei Teile, wobei die beiden Teile eine verringerte allergene Aktivität aufweisen oder ihnen die allergene Aktivität fehlt, und
- Wiederverbinden der beiden Fragmente in umgekehrter Orientierung.

2. Verfahren nach Anspruch 1, **dadurch gekennzeichnet, dass** das Derivat als rekombinantes Protein in einem Wirt, insbesondere in einem Wirt mit großer Expressionskapazität, erzeugt wird.

3. Verfahren nach Anspruch 1 oder 2, **dadurch gekennzeichnet, dass** das Wildtyp-Allergen ausgewählt ist aus der Gruppe der Profiline, insbesondere Phl p 12, Birken-Allergene, insbesondere Bet v 4, Staubmilben-Allergene, insbesondere Der p2, Vorratsmilben-Allergene, insbesondere Lep d 2, Wiesenlieschgras-Allergene, insbesondere Phl p 7.

4. Verfahren nach einem der Ansprüche 1 bis 3, **dadurch gekennzeichnet, dass** die Verringerung der allergenen Aktivität durch eine Verringerung der Inhibition der IgE-Bindungskapazität von mindestens 10%, vorzugsweise mindestens 20%, insbesondere mindestens 30%, im Vergleich zum Wildtyp-Allergen, gemessen wird.

5. Verfahren nach einem der Ansprüche 1 bis 4, **dadurch gekennzeichnet, dass** die Verringerung der allergenen Aktivität durch das Fehlen der Bindung von IgE-Antikörpern aus mit Allergen sensibilisierten Patienten-Seren an einen Dot-Blot des Derivats gemessen wird.

6. Verfahren nach einem der Ansprüche 1 bis 5, **dadurch gekennzeichnet, dass** die Derivate mit einem pharmazeutisch akzeptablen Exzipienten kombiniert und zu einem pharmazeutischen Präparat fertig gestellt werden.

7. Verfahren nach einem der Ansprüche 1 bis 6, **dadurch gekennzeichnet, dass** die Derivate mit einem geeigneten Vakzin-Adjuvans kombiniert und zu einem pharmazeutisch akzeptablen Vakzin-Präparat fertig gestellt werden.

8. Verfahren nach Anspruch 7, **dadurch gekennzeichnet, dass** die Derivate mit weiteren Allergenen zu einem Kombinations-Vakzin kombiniert werden.

9. Verfahren nach Anspruch 8, **dadurch gekennzeichnet, dass** das Allergen ein Wildtyp-Allergen ist, insbesondere eine Mischung von Wildtyp-Allergenen, rekombinanten Wildtyp-Allergenen, Derivaten von Wildtyp-Protein-Allergenen oder Mischungen davon.

10. Verfahren nach einem der Ansprüche 6 bis 9, **dadurch gekennzeichnet, dass** das Präparat weiters einen Allergen-Extrakt enthält.

11. Allergen-Derivat eines Wildtyp-Protein-Allergens, wobei das Wildtyp-Protein-Allergen eine Aminosäure-Sequenz von 1 bis Z aufweist, **dadurch gekennzeichnet, dass** das Derivat nebeneinander
- in N-Terminus zu C-Terminus-Orientierung - die beiden Wildtyp-Allergen-Fragmente X bis Z und 1 bis X enthält, wobei die beiden Wildtyp-Allergen-Fragmente eine reduzierte allergene Aktivität aufweisen oder ihnen die allergene Aktivität fehlt.

12. Allergen-Derivat nach Anspruch 11, **dadurch gekennzeichnet, dass** X bis Z und 1 bis X mindestens 30 Aminosäure-Reste lang, vorzugsweise mindestens 50 Aminosäure-Reste lang, insbesondere mindestens 60 Aminosäure-Reste lang sind.

13. Allergen-Derivat nach Anspruch 11 oder 12, **dadurch gekennzeichnet, dass** X bis Z und 1 bis X sich längenmäßig um 50% oder weniger, vorzugsweise um 30% oder weniger, insbesondere um 20% oder weniger, voneinander unterscheiden.

14. Allergen-Derivat nach einem der Ansprüche 11 bis 13, **dadurch gekennzeichnet, dass** das Wildtyp-Allergen ausgewählt ist aus Typ I-Allergen, vorzugsweise aus Tabelle A, mehr bevorzugt einem Allergen von Wiesenlieschgras(Phleum pratense)-Pollen, insbesondere Phl p 12, Birken(Betula verrucosa)-Pollen, insbesondere Bet v 4, Gift der Gemeinen Wespe (Vespula vulgaris), Gift der Papierwespe ("paper wasp", Polistes annularis), Parietaria judaica-Pollen, Weidelgras-Pollen, Staubmilben-Allergenen, insbesondere Der p 2, oder Mischungen davon.

15. Allergen-Zusammensetzung, umfassend ein Allergen-Derivat nach einem der Ansprüche 11 bis 14 und weitere Allergene, vorzugsweise Wildtyp-Allergene, insbesondere eine Mischung von Wildtyp-Allergenen, rekombinante Wildtyp-Allergenen, Derivate von Wildtyp-Protein-Allergenen oder Mischungen davon.

16. Allergen-Zusammensetzung nach Anspruch 15, **dadurch gekennzeichnet, dass** die Zusammensetzung weiters einen Allergen-Extrakt enthält.

17. Allergen-Zusammensetzung nach den Ansprüchen 15 bis 16, **dadurch gekennzeichnet, dass** sie einen pharmazeutisch akzeptablen Exzipienten enthält.

18. Verwendung eines Allergen-Derivats oder einer Allergen-Zusammensetzung nach einem der Ansprüche 11 bis 17 zur Herstellung eines Allergen-spezifischen Immuntherapie-Medikaments.

19. Verwendung eines Allergen-Derivats oder einer Allergen-Zusammensetzung nach einem der Ansprüche 11 bis 17 zur Herstellung eines Medikaments zur passiven Immunisierung.

20. Verwendung eines Allergen-Derivats oder einer Allergen-Zusammensetzung nach einem der Ansprüche 11 bis 17 zur Herstellung eines Medikaments zur prophylaktischen Immunisierung.

21. Verwendung nach einem der Ansprüche 18 bis 20, **dadurch gekennzeichnet, dass** das Medikament weiters Adjuvantien, Verdünnungsmittel, Konservierungsmittel oder Mischungen davon enthält.

22. Verwendung nach einem der Ansprüche 18 bis 21, **dadurch gekennzeichnet, dass** es 10 ng bis 1 g, vorzugsweise 100 ng bis 10 mg, insbesondere 0,5 µg bis 200 µg des rekombinanten Allergen-Derivats aufweist.

23. Verfahren zur Herstellung eines Allergen-Derivats nach einem der Ansprüche 11 bis 17, **gekennzeichnet durch** die folgenden Schritte:
- Vorsehen eines DNA-Moleküls, das für ein Allergen-Derivat nach einem der Ansprüche 11 bis 17 codiert,
- Transformieren einer Wirtszelle mit dem DNA-Molekül, und
- Exprimieren des Derivats in der Wirtszelle und Isolieren des Derivats.

24. Verfahren nach Anspruch 23, **dadurch gekennzeichnet, dass** der Wirt ein Wirt mit einer großen Expressionskapazität ist.

25. Verfahren zur Herstellung eines Allergen-Derivats nach einem der Ansprüche 11 bis 17, **dadurch gekennzeichnet, dass** es durch chemische Synthese erzeugt wird.

26. Verwendung eines Profilin-Derivats, erhältlich aus einem ersten Wildtyp-Profilin-Molekül mit einem Verfahren nach einem der Ansprüche 1 bis 10 oder 23 bis 25, oder eines Allergen-Derivats eines ersten Wildtyp-Profilin-Moleküls nach den Ansprüchen 11 bis 14 zur Herstellung eines Medikaments zur Prävention oder Behandlung allergischer Erkrankungen, die durch ein zweites Wildtyp-Profilin-Molekül verursacht sind.

27. Verwendung nach Anspruch 26, **dadurch gekennzeichnet, dass** das erste und das zweite Profilin-Molekül ausgewählt sind aus der Gruppe bestehend aus Phl p 12, Bet v 2, Art v 4, Ana c, Api g 4, Mus xp 1, Cor a 2 und Dau c 4.

28. Verwendung nach Anspruch 26, **dadurch gekennzeichnet, dass** das erste Profilin-Molekül Phl p 12 ist und das zweite Profilin-Molekül ausgewählt ist aus der Gruppe bestehend aus Bet v 2, Art v 4, Ana c, Api g 4, Mus xp 1, Cor a 2 und Dau c 4.

29. Verwendung nach Anspruch 27 oder 28 zur Herstellung eines Medikaments zur Behandlung und/oder Prävention von Pollen-Nahrungsmittel-Kreuzsensibilisierungen, die einer Profilin-Allergie zuschreibbar sind.

## Revendications

1. Procédé de production de dérivés d'allergènes protéiques de type sauvage présentant une activité allergène réduite, **caractérisé par** les étapes suivantes :
- mise à disposition d'un allergène protéique de type sauvage présentant une activité allergène,
- épissage dudit allergène protéique de type sauvage en deux parties, lesdites deux parties ayant une activité allergène réduite ou étant dépourvues d'activité allergène, et
- réunion desdits deux fragments dans un sens inverse.

2. Procédé selon la revendication 1, **caractérisé en ce que** ledit dérivé est produit dans un hôte en tant que protéine recombinante, en particulier avec un hôte présentant une capacité d'expression élevée.

3. Procédé selon la revendication 1 ou 2, **caractérisé en ce que** ledit allergène de type sauvage est choisi dans le groupe des profilines, en particulier Phl p 12, des allergènes de bouleau, en particulier Bet v 4, des allergènes des acariens de la poussière, en particulier Der p2, des allergènes des acariens de stockage, en particulier Lep d 2, des allergènes de fléole des prés, en particulier, Phl p 7.

4. Procédé selon l'une quelconque des revendications 1 à 3, **caractérisé en ce que** la réduction de l'activité allergène est mesurée par une réduction de l'inhibition de la capacité de liaison de l'IgE d'au moins 10 %, de préférence, d'au moins 20 %, en particulier, d'au moins 30 % par rapport à l'allergène de type sauvage.

5. Procédé selon l'une quelconque des revendications 1 à 4, **caractérisé en ce que** la réduction de l'activité allergène est mesurée par l'absence de liaison des anticorps d'IgE des sérums d'un patient sensibilisé à l'allergène à un buvardage en point dudit dérivé.

6. Procédé selon l'une quelconque des revendications 1 à 5, **caractérisé en ce que** lesdits dérivés sont combinés à un excipient pharmaceutiquement acceptable et finis en une préparation pharmaceutique.

7. Procédé selon l'une quelconque des revendications 1 à 6, **caractérisé en ce que** lesdits dérivés sont combinés à un adjuvant de vaccin approprié et finis en une préparation de vaccin pharmaceutiquement acceptable.

8. Procédé selon la revendication 7, **caractérisé en ce que** lesdits dérivés sont combinés à d'autres allergènes en un vaccin combiné.

9. Procédé selon la revendication 8, **caractérisée en ce que** ledit allergène est un allergène de type sauvage, en particulier, un mélange d'allergènes de type sauvage, d'allergènes de type sauvage recombinant, de dérivés d'allergènes protéiques de type sauvage ou leurs mélanges.

10. Procédé selon l'une quelconque des revendications 6 à 9, **caractérisé en ce que** ladite préparation contient en outre un extrait d'allergène.

11. Dérivé d'allergène d'un allergène protéique de type sauvage, ledit allergène protéique de type sauvage ayant une séquence d'acides aminés de 1 à Z, **caractérisé en ce que** ledit dérivé contient au niveau adjacent, dans le sens du N-terminal vers le C-terminal, les deux fragments d'allergène de type sauvage X à Z et 1 à X, les deux fragments d'allergène de type sauvage ayant une activité allergène réduite ou étant dépourvus d'activité allergène.

12. Dérivé d'allergène selon la revendication 11, **caractérisé en ce que** X à Z et 1 à X ont une longueur d'au moins 30 résidus d'acides aminés, de préférence, d'au moins 50 résidus d'acides aminés, en particulier d'au moins 60 résidus d'acides aminés.

13. Dérivé d'allergène selon la revendication 11 ou 12, **caractérisé en ce que** X à Z et 1 à X diffèrent en longueur de 50 % ou moins, de préférence, de 30 % ou moins, en particulier, de 20 % ou moins.

14. Dérivé d'allergène selon l'une quelconque des revendications 11 à 13, **caractérisé en ce que** ledit allergène de type sauvage est choisi parmi un allergène de type I, de préférence du tableau A, de manière davantage préférée, un allergène de pollen de fléole des prés (*Phelum pratense*), en particulier, Phl p 12, de pollen de bouleau (*Betula verrucosa*), en particulier Bet v 4, de venin de guêpe (*Vespula vulgaris*), de venin de guêpe cartonnière (*Polistes annularis*), de pollen de *Parietaria judaica*, de pollen d'ivraie, des allergènes d'acariens de la poussière, en particulier Der p 2 ou leurs mélanges.

15. Composition d'allergène comprenant un dérivé d'allergène selon l'une quelconque des revendications 11 à 14, et d'autres allergènes, de préférence, des allergènes de type sauvage, en particulier, un mélange d'allergènes de type sauvage, d'allergènes de type recombinant, de dérivés d'allergènes protéiques de type sauvage ou leurs mélanges.

16. Composition d'allergène selon la revendication 15**, caractérisé en ce que** ladite composition contient en outre un extrait d'allergène.

17. Composition d'allergène selon les revendications 15 à 16, **caractérisée en ce qu'**elle contient un excipient pharmaceutiquement acceptable.

18. Utilisation d'un dérivé d'allergène ou d'une composition d'allergène selon l'une des revendications 11 à 17, pour la préparation d'un médicament immunothérapeutique spécifique à l'allergène.

19. Utilisation d'un dérivé d'allergène ou d'une composition d'allergène selon l'une des revendications 11 à 17 pour la préparation d'un médicament pour l'immunisation passive.

20. Utilisation d'un dérivé d'allergène ou d'une composition d'allergène selon l'une des revendications 11 à 17, pour la préparation d'un médicament pour l'immunisation prophylactique.

21. Utilisation selon l'une quelconque des revendications 18 à 20, **caractérisée en ce que** ledit médicament contient en outre des adjuvants, des diluants, des conservateurs ou leurs mélanges.

22. Utilisation selon l'une quelconque des revendications 18 à 21, **caractérisée en ce qu'**elle comprend 10 ng à 1 g, de préférence, 100 ng à 10 mg, en particulier, 0,5 µg à 200 µg dudit dérivé d'allergène recombinant.

23. Procédé de production d'un dérivé d'allergène selon l'une quelconque des revendications 11 à 17, **caractérisé par** les étapes suivantes :
- fourniture d'une molécule d'ADN codant pour un dérivé d'allergène selon l'une quelconque des revendications 11 à 17,
- transformation d'une cellule hôte avec ladite molécule d'ADN et
- expression dudit dérivé dans ladite cellule hôte et isolement dudit dérivé.

24. Procédé selon la revendication 23, **caractérisé en ce que** ledit hôte est un hôte présentant une capacité d'expression élevée.

25. Procédé de production d'un dérivé d'allergène selon l'une quelconque des revendications 11 à 17, **caractérisé en ce qu'**il est produit par synthèse chimique.

26. Utilisation d'un dérivé de profiline pouvant être obtenu à partir d'une première molécule de profiline de type sauvage par un procédé selon l'une quelconque des revendications 1 à 10 ou 23 à 25 ou un dérivé d'allergène d'une première molécule de profiline de type sauvage selon les revendications 11 à 14, pour la production d'un médicament pour la prévention ou le traitement de maladies allergiques provoquées par une deuxième molécule de profiline de type sauvage.

27. Utilisation selon la revendication 26, **caractérisée en ce que** lesdites première et deuxième molécules de profiline sont choisies dans le groupe consistant en Phl p 12, Bet v 2, Art v 4, Ana c, Api g 4, Mus xp 1, Cor a 2 et Dau c 4.

28. Utilisation selon la revendication 26, **caractérisée en ce que** ladite première molécule de profiline est Phl p 12 et ladite deuxième molécule de profiline est choisie dans le groupe consistant en Bet v 2, Art v 4, Ana c, Api g 4, Mus xp 1, Cor a 2 et Dau c 4.

29. Utilisation selon la revendication 27 ou 28, pour la production d'un médicament pour le traitement et/ou la prévention d'une sensibilité croisée au pollen attribuable à une allergie à la profiline.
